# EUROPEAN PATENT APPLICATION

(11) **EP 2 177 535 A1**
(43) Date of publication of application: **21.04.2010**
(21) Application number: 08791668.0
(22) Date of filing: 25.07.2008
(51) Int. Cl.: C07K 16/10, A61K 39/395, A61P 31/14, C12N 15/09, G01N 33/53, G01N 33/576, C12P 21/08

(54) **ANTIBODY HAVING INHIBITORY ACTIVITY ON INFECTION WITH HEPATITIS C VIRUS (HCV), AND USE THEREOF**

(30) Priority: 25.07.2007 JP 2007193413
(71) Applicant: JAPAN as represented by DIRECTOR GENERAL OF NATIONAL INSTITUTE OF INFECTIOUS DISEASES, Tokyo 162-8640 (JP); TORAY INDUSTRIES, INC., Tokyo 103-8666 (JP)
(72) Inventor: WAKITA, Takaji, Tokyo 107-0062 (JP); SUZUKI, Tetsuro, Nishitokyo-shi Tokyo 202-0022 (JP); MORIKAWA, Kenichi, Tokyo 140-0011 (JP); OMI, Noriaki, Kamakura-shi Kanagawa 248-8555 (JP); NAKAMURA, Noriko, Kamakura-shi Kanagawa 248-8555 (JP); AKAZAWA, Daisuke, Kamakura-shi Kanagawa 248-8555 (JP)
(74) Representative: Kador & Partner
(86) International application number: PCT/JP2008/063424
(87) International publication number: WO 2009/014216

(57) **Abstract**

The objection of the invention is to provide an antibody that inhibits infection with hepatitis C virus (HCV). To this end, this invention provides an antibody that recognizes the hepatitis C virus (HCV) particle obtained from the hepatitis C virus (HCV) genome comprising the following (i) and (ii) ligated to each other as an antigen and has an inhibitory activity on infection with hepatitis C virus (HCV): (i) (a) the 5'-untranslated region, the core protein-encoding sequence, the E1 protein-encoding sequence, the E2 protein-encoding sequence, and the p7 protein-encoding sequence of the JFH-1 strain of the hepatitis C virus (HCV) or (b) the 5'-untranslated region, the core protein-encoding sequence, the E1 protein-encoding sequence, the E2 protein-encoding sequence, and the p7 protein-encoding sequence of the J6CF strain the hepatitis C virus (HCV); and (ii) the NS2 protein-encoding sequence, the NS3 protein-encoding sequence, the NS4A protein-encoding sequence, the NS4B protein-encoding sequence, the NS5A protein-encoding sequence, the NS5B protein-encoding sequence, and the 3'-untranslated region of the JFH-1 strain.

SEQUENCE LISTING

<110> Japan as represented by Director-General of National Institute of Infectious Diseases
Toray Industries Inc.

<120> Antibody having activity of inhibiting Hepatitis C virus infection and use thereof

<130> PH-3644-PCT

<140> PCT/JP2008/063424
<141> 2008-07-25

<150> JP 2007-193413
<151> 2007-07-25

<160> 68

<170> PatentIn version 3.4

<210> 1
<211> 9678
<212> DNA
<213> Artificial

<220>
<223> Cloned HCV genomic cDNA in pJFH-1

<220>
<223> Inventor: Wakita, Takaji
Inventor: Suzuki, Tetsuro
Inventor: Morikawa, Kenichi
Inventor: Omi, Noriaki
Inventor: Nakamura, Noriko
Inventor: Akazawa, Daisuke

<400> 1
acctgcccct aataggggcg acactccgcc atgaatcact cccctgtgag gaactactgt 60

cttcacgcag aaagcgccta gccatggcgt tagtatgagt gtcgtacagc ctccaggccc 120

ccccctcccg ggagagccat agtggtctgc ggaaccggtg agtacaccgg aattgccggg 180

aagactgggt cctttcttgg ataaacccac tctatgcccg gccatttggg cgtgcccccg 240

caagactgct agccgagtag cgttgggttg cgaaaggcct tgtggtactg cctgataggg 300

cgcttgcgag tgccccggga ggtctcgtag accgtgcacc atgagcacaa atcctaaacc 360

tcaaagaaaa accaaaagaa acaccaaccg tcgcccagaa gacgttaagt tcccgggcgg 420

cggccagatc gttggcggag tatacttgtt gccgcgcagg ggccccaggt tgggtgtgcg 480
cacgacaagg aaaacttcgg agcggtccca gccacgtggg agacgccagc ccatccccaa 540
agatcggcgc tccactggca aggcctgggg aaaaccaggt cgcccctggc ccctatatgg 600
gaatgaggga ctcggctggg caggatggct cctgtccccc cgaggctctc gcccctcctg 660
gggccccact gacccccggc ataggtcgcg caacgtgggt aaagtcatcg acaccctaac 720
gtgtggcttt gccgacctca tggggtacat ccccgtcgta ggcgccccgc ttagtggcgc 780
cgccagagct gtcgcgcacg gcgtgagagt cctggaggac ggggttaatt atgcaacagg 840
gaacctaccc ggtttcccct ttctatcttt cttgctggcc ctgttgtcct gcatcaccgt 900
tccggtctct gctgcccagg tgaagaatac cagtagcagc tacatggtga ccaatgactg 960
ctccaatgac agcatcactt ggcagctcga ggctgcggtt ctccacgtcc ccgggtgcgt 1020
cccgtgcgag agagtgggga atacgtcacg gtgttgggtg ccagtctcgc caaacatggc 1080
tgtgcggcag cccggtgccc tcacgcaggg tctgcggacg cacatcgata tggttgtgat 1140
gtccgccacc ttctgctctg ctctctacgt gggggacctc tgtggcgggg tgatgctcgc 1200
ggcccaggtg ttcatcgtct cgccgcagta ccactggttt gtgcaagaat gcaattgctc 1260
catctaccct ggcaccatca ctggacaccg catggcatgg gacatgatga tgaactggtc 1320
gcccacggcc accatgatcc tggcgtacgt gatgcgcgtc cccgaggtca tcatagacat 1380
cgttagcggg gctcactggg gcgtcatgtt cggcttggcc tacttctcta tgcagggagc 1440
gtgggcgaag gtcattgtca tccttctgct ggccgctggg gtggacgcgg gcaccaccac 1500
cgttggaggc gctgttgcac gttccaccaa cgtgattgcc ggcgtgttca gccatggccc 1560
tcagcagaac attcagctca ttaacaccaa cggcagttgg cacatcaacc gtactgcctt 1620
gaattgcaat gactccttga acaccggctt tctcgcggcc ttgttctaca ccaaccgctt 1680
taactcgtca gggtgtccag ggcgcctgtc cgcctgccgc aacatcgagg ctttccggat 1740
agggtggggc accctacagt acgaggataa tgtcaccaat ccagaggata tgaggccgta 1800
ctgctggcac taccccccaa agccgtgtgg cgtagtcccc gcgaggtctg tgtgtggccc 1860
agtgtactgt ttcaccccca gcccggtagt agtgggcacg accgacagac gtggagtgcc 1920

cacctacaca tggggagaga atgagacaga tgtcttccta ctgaacagca cccgaccgcc 1980

gcagggctca tggttcggct gcacgtggat gaactccact ggtttcacca agacttgtgg 2040

cgcgccacct tgccgcacca gagctgactt caacgccagc acggacttgt tgtgccctac 2100

ggattgtttt aggaagcatc ctgatgccac ttatattaag tgtggttctg ggccctggct 2160

cacaccaaag tgcctggtcc actaccctta cagactctgg cattacccct gcacagtcaa 2220

ttttaccatc ttcaagataa gaatgtatgt agggggggtt gagcacaggc tcacggccgc 2280

atgcaacttc actcgtgggg atcgctgcga cttggaggac agggacagga gtcagctgtc 2340

tcctctgttg cactctacca cggaatgggc catcctgccc tgcacctact cagacttacc 2400

cgctttgtca actggtcttc tccaccttca ccagaacatc gtggacgtac aatacatgta 2460

tggcctctca cctgctatca caaaatacgt cgttcgatgg gagtgggtgg tactcttatt 2520

cctgctctta gcggacgcca gagtctgcgc ctgcttgtgg atgctcatct tgttgggcca 2580

ggccgaagca gcattggaga agttggtcgt cttgcacgct gcgagtgcgg ctaactgcca 2640

tggcctccta tattttgcca tcttcttcgt ggcagcttgg cacatcaggg gtcgggtggt 2700

ccccttgacc acctattgcc tcactggcct atggcccttc tgcctactgc tcatggcact 2760

gccccggcag gcttatgcct atgacgcacc tgtgcacgga cagataggcg tgggtttgtt 2820

gatattgatc accctcttca cactcacccc ggggtataag accctcctcg gccagtgtct 2880

gtggtggttg tgctatctcc tgaccctggg ggaagccatg attcaggagt gggtaccacc 2940

catgcaggtg cgcggcggcc gcgatggcat cgcgtgggcc gtcactatat tctgcccggg 3000

tgtggtgttt gacattacca aatggctttt ggcgttgctt gggcctgctt acctcttaag 3060

ggccgctttg acacatgtgc cgtacttcgt cagagctcac gctctgataa gggtatgcgc 3120

tttggtgaag cagctcgcgg ggggtaggta tgttcaggtg gcgctattgg cccttggcag 3180

gtggactggc acctacatct atgaccacct cacacctatg tcggactggg ccgctagcgg 3240

cctgcgcgac ttagcggtcg ccgtggaacc catcatcttc agtccgatgg agaagaaggt 3300

catcgtctgg ggagcggaga cggctgcatg tggggacatt ctacatggac ttcccgtgtc 3360

cgcccgactc ggccaggaga tcctcctcgg cccagctgat ggctacacct ccaaggggtg 3420

gaagctcctt gctcccatca ctgcttatgc ccagcaaaca cgaggcctcc tgggcgccat 3480

agtggtgagt atgacggggc gtgacaggac agaacaggcc ggggaagtcc aaatcctgtc 3540

cacagtctct cagtccttcc tcggaacaac catctcgggg gttttgtgga ctgtttacca 3600

cggagctggc aacaagactc tagccggctt acggggtccg gtcacgcaga tgtactcgag 3660

tgctgagggg gacttggtag gctggcccag cccccctggg accaagtctt tggagccgtg 3720

caagtgtgga gccgtcgacc tatatctggt cacgcggaac gctgatgtca tcccggctcg 3780

gagacgcggg gacaagcggg gagcattgct ctccccgaga cccatttcga ccttgaaggg 3840

gtcctcgggg gggccggtgc tctgccctag gggccacgtc gttgggctct tccgagcagc 3900

tgtgtgctct cggggcgtgg ccaaatccat cgatttcatc cccgttgaga cactcgacgt 3960

tgttacaagg tctcccactt tcagtgacaa cagcacgcca ccggctgtgc cccagaccta 4020

tcaggtcggg tacttgcatg ctccaactgg cagtggaaag agcaccaagg tccctgtcgc 4080

gtatgccgcc caggggtaca aagtactagt gcttaacccc tcggtagctg ccaccctggg 4140

gtttggggcg tacctatcca aggcacatgg catcaatccc aacattagga ctggagtcag 4200

gaccgtgatg accggggagg ccatcacgta ctccacatat ggcaaatttc tcgccgatgg 4260

gggctgcgct agcggcgcct atgacatcat catatgcgat gaatgccacg ctgtggatgc 4320

tacctccatt ctcggcatcg gaacggtcct tgatcaagca gagacagccg gggtcagact 4380

aactgtgctg gctacggcca caccccccgg gtcagtgaca accccccatc ccgatataga 4440

agaggtaggc ctcgggcggg agggtgagat ccccttctat gggagggcga ttcccctatc 4500

ctgcatcaag ggagggagac acctgatttt ctgccactca aagaaaaagt gtgacgagct 4560

cgcggcggcc cttcggggca tgggcttgaa tgccgtggca tactatagag ggttggacgt 4620

ctccataata ccagctcagg gagatgtggt ggtcgtcgcc accgacgccc tcatgacggg 4680

gtacactgga gactttgact ccgtgatcga ctgcaatgta gcggtcaccc aagctgtcga 4740

cttcagcctg gaccccacct tcactataac cacacagact gtcccacaag acgctgtctc 4800

acgcagtcag cgccgcgggc gcacaggtag aggaagacag ggcacttata ggtatgtttc 4860

cactggtgaa cgagcctcag gaatgtttga cagtgtagtg ctttgtgagt gctacgacgc 4920

aggggctgcg tggtacgatc tcacaccagc ggagaccacc gtcaggctta gagcgtattt 4980

caacacgccc ggcctacccg tgtgtcaaga ccatcttgaa ttttgggagg cagttttcac 5040

cggcctcaca cacatagacg cccacttcct ctcccaaaca aagcaagcgg gggagaactt 5100

cgcgtaccta gtagcctacc aagctacggt gtgcgccaga gccaaggccc ctcccccgtc 5160

ctgggacgcc atgtggaagt gcctggcccg actcaagcct acgcttgcgg gccccacacc 5220

tctcctgtac cgtttgggcc ctattaccaa tgaggtcacc ctcacacacc ctgggacgaa 5280

gtacatcgcc acatgcatgc aagctgacct tgaggtcatg accagcacgt gggtcctagc 5340

tggaggagtc ctggcagccg tcgccgcata ttgcctggcg actggatgcg tttccatcat 5400

cggccgcttg cacgtcaacc agcgagtcgt cgttgcgccg gataaggagg tcctgtatga 5460

ggcttttgat gagatggagg aatgcgcctc tagggcggct ctcatcgaag aggggcagcg 5520

gatagccgag atgttgaagt ccaagatcca aggcttgctg cagcaggcct ctaagcaggc 5580

ccaggacata caacccgcta tgcaggcttc atggcccaaa gtggaacaat tttgggccag 5640

acacatgtgg aacttcatta gcggcatcca atacctcgca ggattgtcaa cactgccagg 5700

gaaccccgcg gtggcttcca tgatggcatt cagtgccgcc ctcaccagtc cgttgtcgac 5760

cagtaccacc atccttctca acatcatggg aggctggtta gcgtcccaga tcgcaccacc 5820

cgcgggggcc accggctttg tcgtcagtgg cctggtgggg gctgccgtgg gcagcatagg 5880

cctgggtaag gtgctggtgg acatcctggc aggatatggt gcgggcattt cgggggccct 5940

cgtcgcattc aagatcatgt ctggcgagaa gccctctatg gaagatgtca tcaatctact 6000

gcctgggatc ctgtctccgg gagccctggt ggtgggggtc atctgcgcgg ccattctgcg 6060

ccgccacgtg ggaccggggg agggcgcggt ccaatggatg aacaggctta ttgcctttgc 6120

ttccagagga aaccacgtcg cccctactca ctacgtgacg gagtcggatg cgtcgcagcg 6180

tgtgacccaa ctacttggct ctcttactat aaccagccta ctcagaagac tccacaattg 6240

gataactgag gactgcccca tcccatgctc cggatcctgg ctccgcgacg tgtgggactg 6300

ggtttgcacc atcttgacag acttcaaaaa ttggctgacc tctaaattgt tccccaagct 6360

gcccggcctc cccttcatct cttgtcaaaa ggggtacaag ggtgtgtggg ccggcactgg 6420

catcatgacc acgcgctgcc cttgcggcgc caacatctct ggcaatgtcc gcctgggctc 6480

tatgaggatc acagggccta aaacctgcat gaacacctgg caggggacct ttcctatcaa 6540

ttgctacacg gagggccagt gcgcgccgaa accccccacg aactacaaga ccgccatctg 6600

gagggtggcg gcctcggagt acgcggaggt gacgcagcat gggtcgtact cctatgtaac 6660

aggactgacc actgacaatc tgaaaattcc ttgccaacta ccttctccag agtttttctc 6720

ctgggtggac ggtgtgcaga tccataggtt tgcacccaca ccaaagccgt ttttccggga 6780

tgaggtctcg ttctgcgttg ggcttaattc ctatgctgtc gggtcccagc ttccctgtga 6840

acctgagccc gacgcagacg tattgaggtc catgctaaca gatccgcccc acatcacggc 6900

ggagactgcg gcgcggcgct tggcacgggg atcacctcca tctgaggcga gctcctcagt 6960

gagccagcta tcagcaccgt cgctgcgggc cacctgcacc acccacagca acacctatga 7020

cgtggacatg gtcgatgcca acctgctcat ggagggcggt gtggctcaga cagagcctga 7080

gtccagggtg cccgttctgg actttctcga gccaatggcc gaggaagaga gcgaccttga 7140

gccctcaata ccatcggagt gcatgctccc caggagcggg tttccacggg ccttaccggc 7200

ttgggcacgg cctgactaca acccgccgct cgtggaatcg tggaggaggc cagattacca 7260

accgcccacc gttgctggtt gtgctctccc cccccccaag aaggccccga cgcctccccc 7320

aaggagacgc cggacagtgg gtctgagcga gagcaccata tcagaagccc tccagcaact 7380

ggccatcaag acctttggcc agcccccctc gagcggtgat gcaggctcgt ccacgggggc 7440

gggcgccgcc gaatccggcg gtccgacgtc ccctggtgag ccggccccct cagagacagg 7500

ttccgcctcc tctatgcccc ccctcgaggg ggagcctgga gatccggacc tggagtctga 7560

tcaggtagag cttcaacctc ccccccaggg ggggggggta gctcccggtt cgggctcggg 7620

gtcttggtct acttgctccg aggaggacga taccaccgtg tgctgctcca tgtcatactc 7680

ctggaccggg gctctaataa ctccctgtag ccccgaagag gaaaagttgc caatcaaccc 7740

tttgagtaac tcgctgttgc gataccataa caaggtgtac tgtacaacat caaagagcgc 7800

ctcacagagg gctaaaaagg taacttttga caggacgcaa gtgctcgacg cccattatga 7860

ctcagtctta aaggacatca agctagcggc ttccaaggtc agcgcaaggc tcctcacctt 7920

ggaggaggcg tgccagttga ctccacccca ttctgcaaga tccaagtatg gattcggggc 7980

caaggaggtc cgcagcttgt ccgggagggc cgttaaccac atcaagtccg tgtggaagga 8040

cctcctggaa gacccacaaa caccaattcc cacaaccatc atggccaaaa atgaggtgtt 8100

ctgcgtggac cccgccaagg ggggtaagaa accagctcgc ctcatcgttt accctgacct 8160

cggcgtccgg gtctgcgaga aaatggccct ctatgacatt acacaaaagc ttcctcaggc 8220

ggtaatggga gcttcctatg gcttccagta ctcccctgcc caacgggtgg agtatctctt 8280

gaaagcatgg gcggaaaaga aggaccccat gggtttttcg tatgataccc gatgcttcga 8340

ctcaaccgtc actgagagag acatcaggac cgaggagtcc atataccagg cctgctccct 8400

gcccgaggag gcccgcactg ccatacactc gctgactgag agactttacg taggagggcc 8460

catgttcaac agcaagggtc aaacctgcgg ttacagacgt tgccgcgcca gcggggtgct 8520

aaccactagc atgggtaaca ccatcacatg ctatgtgaaa gccctagcgg cctgcaaggc 8580

tgcggggata gttgcgccca caatgctggt atgcggcgat gacctagtag tcatctcaga 8640

aagccagggg actgaggagg acgagcggaa cctgagagcc ttcacggagg ccatgaccag 8700

gtactctgcc cctcctggtg atccccccag accggaatat gacctggagc taataacatc 8760

ctgttcctca aatgtgtctg tggcgttggg cccgcggggc cgccgcagat actacctgac 8820

cagagaccca accactccac tcgcccgggc tgcctgggaa acagttagac actcccctat 8880

caattcatgg ctgggaaaca tcatccagta tgctccaacc atatgggttc gcatggtcct 8940

aatgacacac ttcttctcca ttctcatggt ccaagacacc ctggaccaga acctcaactt 9000

tgagatgtat ggatcagtat actccgtgaa tcctttggac cttccagcca taattgagag 9060

gttacacggg cttgacgcct tttctatgca cacatactct caccacgaac tgacgcgggt 9120

ggcttcagcc ctcagaaaac ttggggcgcc acccctcagg gtgtggaaga gtcgggctcg 9180

cgcagtcagg gcgtccctca tctcccgtgg agggaaagcg gccgtttgcg gccgatatct 9240

cttcaattgg gcggtgaaga ccaagctcaa actcactcca ttgccggagg cgcgcctact 9300

ggacttatcc agttggttca ccgtcggcgc cggcgggggc gacatttttc acagcgtgtc 9360

gcgcgcccga ccccgctcat tactcttcgg cctactccta cttttcgtag gggtaggcct 9420

cttcctactc cccgctcggt agagcggcac acactaggta cactccatag ctaactgttc 9480

cttttttttt tttttttttt tttttttttt tttttttttt ttttcttttt tttttttttc 9540

cctctttctt cccttctcat cttattctac tttctttctt ggtggctcca tcttagccct 9600

agtcacggct agctgtgaaa ggtccgtgag ccgcatgact gcagagagtg ccgtaactgg 9660

tctctctgca gatcatgt 9678

<210> 2
<211> 9683
<212> DNA
<213> Artificial

<220>
<223> Cloned HCV genomic cDNA in pJ6/JFH-1

<400> 2
acccgcccct aataggggcg acactccgcc atgaatcact cccctgtgag gaactactgt 60

cttcacgcag aaagcgtcta gccatggcgt tagtatgagt gtcgtacagc ctccaggccc 120

ccccctcccg ggagagccat agtggtctgc ggaaccggtg agtacaccgg aattgccggg 180

aagactgggt cctttcttgg ataaacccac tctatgcccg gccatttggg cgtgcccccg 240

caagactgct agccgagtag cgttgggttg cgaaaggcct tgtggtactg cctgataggg 300

tgcttgcgag tgccccggga ggtctcgtag accgtgcacc atgagcacaa atcctaaacc 360

tcaaagaaaa accaaaagaa acaccaaccg tcgcccacaa gacgttaagt ttccgggcgg 420

cggccagatc gttggcggag tatacttgtt gccgcgcagg ggccccaggt tgggtgtgcg 480

cgcgacaagg aagacttcgg agcggtccca gccacgtgga aggcgccagc ccatccctaa 540

agatcggcgc tccactggca aatcctgggg aaaaccagga tacccctggc ccctatacgg 600

gaatgaggga ctcggctggg caggatggct cctgtccccc cgaggttccc gtccctcttg 660

gggccccaat gacccccggc ataggtcgcg caacgtgggt aaggtcatcg ataccctaac 720

gtgcggcttt gccgacctca tggggtacat ccctgtcgtg ggcgccccgc tcggcggcgt 780

cgccagagct ctcgcgcatg gcgtgagagt cctggaggac ggggttaatt ttgcaacagg 840

gaacttaccc ggttgctcct tttctatctt cttgctggcc ctgctgtcct gcatcaccac 900

cccggtctcc gctgccgaag tgaagaacat cagtaccggc tacatggtga ctaacgactg 960

caccaatgac agcattacct ggcagctcca ggctgctgtc ctccacgtcc ccgggtgcgt 1020

cccgtgcgag aaagtgggga atgcatctca gtgctggata ccggtctcac cgaatgtggc 1080

cgtgcagcgg cccggcgccc tcacgcaggg cttgcggacg cacatcgaca tggttgtgat 1140

gtccgccacg ctctgctctg ccctctacgt gggggacctc tgcggtgggg tgatgctcgc 1200

agcccaaatg ttcattgtct cgccgcagca ccactggttt gtccaagact gcaattgctc 1260

catctaccct ggtaccatca ctggacaccg catggcatgg gacatgatga tgaactggtc 1320

gcccacggct accatgatct tggcgtacgc gatgcgtgtc cccgaggtca ttatagacat 1380

cattagcggg gctcattggg gcgtcatgtt cggcttggcc tacttctcta tgcagggagc 1440

gtgggcgaaa gtcgttgtca tccttctgtt ggccgccggg gtggacgcgc gcacccatac 1500

tgttgggggt tctgccgcgc agaccaccgg gcgcctcacc agcttatttg acatgggccc 1560

caggcagaaa atccagctcg ttaacaccaa tggcagctgg cacatcaacc gcaccgccct 1620

gaactgcaat gactccttgc acaccggctt tatcgcgtct ctgttctaca cccacagctt 1680

caactcgtca ggatgtcccg aacgcatgtc cgcctgccgc agtatcgagg ccttccgggt 1740

gggatggggc gccttgcaat atgaggataa tgtcaccaat ccagaggata tgagacccta 1800

ttgctggcac tacccaccaa ggcagtgtgg cgtggtctcc gcgaagactg tgtgtggccc 1860

agtgtactgt ttcaccccca gcccagtggt agtgggcacg accgacaggc ttggagcgcc 1920

cacttacacg tggggggaga atgagacaga tgtcttccta ttgaacagca ctcgaccacc 1980

gctggggtca tggttcggct gcacgtggat gaactcttct ggctacacca agacttgcgg 2040

cgcaccaccc tgccgtacta gagctgactt caacgccagc acggacctgt tgtgccccac 2100

ggactgtttt aggaagcatc ctgataccac ttacctcaaa tgcggctctg ggccctggct 2160

cacgccaagg tgcctgatcg actaccccta caggctctgg cattacccct gcacagttaa 2220

ctataccatc ttcaaaataa ggatgtatgt gggaggggtt gagcacaggc tcacggctgc 2280

atgcaatttc actcgtgggg atcgttgcaa cttggaggac agagacagaa gtcaactgtc 2340

tcctttgttg cactccacca cggaatgggc cattttacct tgctcttact cggacctgcc 2400

cgccttgtcg actggtcttc tccacctcca ccaaaacatc gtggacgtac aattcatgta 2460

tggcctatca cctgccctca caaaatacat cgtccgatgg gagtgggtaa tactcttatt 2520

cctgctctta gcggacgcca gggtttgcgc ctgcttatgg atgctcatct tgttgggcca 2580

ggccgaagca gcactagaga agctggtcat cttgcacgct gcgagcgcag ctagctgcaa 2640

tggcttccta tattttgtca tctttttcgt ggctgcttgg tacatcaagg gtcgggtagt 2700

ccccttagct acctattccc tcactggcct gtggtccttt agcctactgc tcctagcatt 2760

gccccaacag gcttatgctt atgacgcatc tgtgcatggc cagataggag cggctctgct 2820

ggtaatgatc accctcttca cactcacccc ggggtataag accctcctcg gccagtgtct 2880

gtggtggttg tgctatctcc tgaccctggg ggaagccatg attcaggagt gggtaccacc 2940

catgcaggtg cgcggcggcc gcgatggcat cgcgtgggcc gtcactatat tctgcccggg 3000

tgtggtgttt gacattacca aatggctttt ggcgttgctt gggcctgctt acctcttaag 3060

ggccgctttg acacatgtgc cgtacttcgt cagagctcac gctctgataa gggtatgcgc 3120

tttggtgaag cagctcgcgg ggggtaggta tgttcaggtg gcgctattgg cccttggcag 3180

gtggactggc acctacatct atgaccacct cacacctatg tcggactggg ccgctagcgg 3240

cctgcgcgac ttagcggtcg ccgtggaacc catcatcttc agtccgatgg agaagaaggt 3300

catcgtctgg ggagcggaga cggctgcatg tggggacatt ctacatggac ttcccgtgtc 3360

cgcccgactc ggccaggaga tcctcctcgg cccagctgat ggctacacct ccaaggggtg 3420

gaagctcctt gctcccatca ctgcttatgc ccagcaaaca cgaggcctcc tgggcgccat 3480

agtggtgagt atgacggggc gtgacaggac agaacaggcc ggggaagtcc aaatcctgtc 3540

cacagtctct cagtccttcc tcggaacaac catctcgggg gttttgtgga ctgtttacca 3600

cggagctggc aacaagactc tagccggctt acggggtccg gtcacgcaga tgtactcgag 3660

tgctgagggg gacttggtag gctggcccag cccccctggg accaagtctt tggagccgtg 3720

caagtgtgga gccgtcgacc tatatctggt cacgcggaac gctgatgtca tcccggctcg 3780

gagacgcggg gacaagcggg gagcattgct ctccccgaga cccatttcga ccttgaaggg 3840

gtcctcgggg gggccggtgc tctgccctag gggccacgtc gttgggctct tccgagcagc 3900

tgtgtgctct cggggcgtgg ccaaatccat cgatttcatc cccgttgaga cactcgacgt 3960

tgttacaagg tctcccactt tcagtgacaa cagcacgcca ccggctgtgc cccagaccta 4020

tcaggtcggg tacttgcatg ctccaactgg cagtggaaag agcaccaagg tccctgtcgc 4080

gtatgccgcc caggggtaca aagtactagt gcttaacccc tcggtagctg ccaccctggg 4140

gtttggggcg tacctatcca aggcacatgg catcaatccc aacattagga ctggagtcag 4200

gaccgtgatg accggggagg ccatcacgta ctccacatat ggcaaatttc tcgccgatgg 4260

gggctgcgct agcggcgcct atgacatcat catatgcgat gaatgccacg ctgtggatgc 4320

tacctccatt ctcggcatcg gaacggtcct tgatcaagca gagacagccg gggtcagact 4380

aactgtgctg gctacggcca caccccccgg gtcagtgaca accccccatc ccgatataga 4440

agaggtaggc ctcgggcggg agggtgagat ccccttctat gggagggcga ttcccctatc 4500

ctgcatcaag ggagggagac acctgatttt ctgccactca aagaaaaagt gtgacgagct 4560

cgcggcggcc cttcggggca tgggcttgaa tgccgtggca tactatagag ggttggacgt 4620

ctccataata ccagctcagg gagatgtggt ggtcgtcgcc accgacgccc tcatgacggg 4680

gtacactgga gactttgact ccgtgatcga ctgcaatgta gcggtcaccc aagctgtcga 4740

cttcagcctg gaccccacct tcactataac cacacagact gtcccacaag acgctgtctc 4800

acgcagtcag cgccgcgggc gcacaggtag aggaagacag ggcacttata ggtatgtttc 4860

cactggtgaa cgagcctcag gaatgtttga cagtgtagtg ctttgtgagt gctacgacgc 4920

aggggctgcg tggtacgatc tcacaccagc ggagaccacc gtcaggctta gagcgtattt 4980

caacacgccc ggcctacccg tgtgtcaaga ccatcttgaa ttttgggagg cagttttcac 5040

cggcctcaca cacatagacg cccacttcct ctcccaaaca aagcaagcgg gggagaactt 5100

cgcgtaccta gtagcctacc aagctacggt gtgcgccaga gccaaggccc ctcccccgtc 5160

ctgggacgcc atgtggaagt gcctggcccg actcaagcct acgcttgcgg gccccacacc 5220

tctcctgtac cgtttgggcc ctattaccaa tgaggtcacc ctcacacacc ctgggacgaa 5280

gtacatcgcc acatgcatgc aagctgacct tgaggtcatg accagcacgt gggtcctagc 5340

tggaggagtc ctggcagccg tcgccgcata ttgcctggcg actggatgcg tttccatcat 5400

cggccgcttg cacgtcaacc agcgagtcgt cgttgcgccg gataaggagg tcctgtatga 5460

ggcttttgat gagatggagg aatgcgcctc tagggcggct ctcatcgaag aggggcagcg 5520

gatagccgag atgttgaagt ccaagatcca aggcttgctg cagcaggcct ctaagcaggc 5580

ccaggacata caacccgcta tgcaggcttc atggcccaaa gtggaacaat tttgggccag 5640

acacatgtgg aacttcatta gcggcatcca atacctcgca ggattgtcaa cactgccagg 5700

gaaccccgcg gtggcttcca tgatggcatt cagtgccgcc ctcaccagtc cgttgtcgac 5760

cagtaccacc atccttctca acatcatggg aggctggtta gcgtcccaga tcgcaccacc 5820

cgcgggggcc accggctttg tcgtcagtgg cctggtgggg gctgccgtgg gcagcatagg 5880

cctgggtaag gtgctggtgg acatcctggc aggatatggt gcgggcattt cgggggccct 5940

cgtcgcattc aagatcatgt ctggcgagaa gccctctatg gaagatgtca tcaatctact 6000

gcctgggatc ctgtctccgg gagccctggt ggtgggggtc atctgcgcgg ccattctgcg 6060

ccgccacgtg ggaccggggg agggcgcggt ccaatggatg aacaggctta ttgcctttgc 6120

ttccagagga aaccacgtcg cccctactca ctacgtgacg gagtcggatg cgtcgcagcg 6180

tgtgacccaa ctacttggct ctcttactat aaccagccta ctcagaagac tccacaattg 6240

gataactgag gactgcccca tcccatgctc cggatcctgg ctccgcgacg tgtgggactg 6300

ggtttgcacc atcttgacag acttcaaaaa ttggctgacc tctaaattgt tccccaagct 6360

gcccggcctc cccttcatct cttgtcaaaa ggggtacaag ggtgtgtggg ccggcactgg 6420

catcatgacc acgcgctgcc cttgcggcgc caacatctct ggcaatgtcc gcctgggctc 6480

tatgaggatc acagggccta aaacctgcat gaacacctgg caggggacct ttcctatcaa 6540

ttgctacacg gagggccagt gcgcgccgaa accccccacg aactacaaga ccgccatctg 6600

gagggtggcg gcctcggagt acgcggaggt gacgcagcat gggtcgtact cctatgtaac 6660

aggactgacc actgacaatc tgaaaattcc ttgccaacta ccttctccag agtttttctc 6720

ctgggtggac ggtgtgcaga tccataggtt tgcacccaca ccaaagccgt ttttccggga 6780

tgaggtctcg ttctgcgttg ggcttaattc ctatgctgtc gggtcccagc ttccctgtga 6840

acctgagccc gacgcagacg tattgaggtc catgctaaca gatccgcccc acatcacggc 6900

ggagactgcg gcgcggcgct tggcacgggg atcacctcca tctgaggcga gctcctcagt 6960

gagccagcta tcagcaccgt cgctgcgggc cacctgcacc acccacagca acacctatga 7020

cgtggacatg gtcgatgcca acctgctcat ggagggcggt gtggctcaga cagagcctga 7080

gtccagggtg cccgttctgg actttctcga gccaatggcc gaggaagaga gcgaccttga 7140

gccctcaata ccatcggagt gcatgctccc caggagcggg tttccacggg ccttaccggc 7200

ttgggcacgg cctgactaca acccgccgct cgtggaatcg tggaggaggc cagattacca 7260

accgcccacc gttgctggtt gtgctctccc cccccccaag aaggccccga cgcctccccc 7320

aaggagacgc cggacagtgg gtctgagcga gagcaccata tcagaagccc tccagcaact 7380

ggccatcaag acctttggcc agcccccctc gagcggtgat gcaggctcgt ccacgggggc 7440

gggcgccgcc gaatccggcg gtccgacgtc ccctggtgag ccggccccct cagagacagg 7500

ttccgcctcc tctatgcccc ccctcgaggg ggagcctgga gatccggacc tggagtctga 7560

tcaggtagag cttcaacctc ccccccaggg ggggggggta gctcccggtt cgggctcggg 7620

gtcttggtct acttgctccg aggaggacga taccaccgtg tgctgctcca tgtcatactc 7680

ctggaccggg gctctaataa ctccctgtag ccccgaagag gaaaagttgc caatcaaccc 7740

tttgagtaac tcgctgttgc gataccataa caaggtgtac tgtacaacat caaagagcgc 7800

ctcacagagg gctaaaaagg taacttttga caggacgcaa gtgctcgacg cccattatga 7860

ctcagtctta aaggacatca agctagcggc ttccaaggtc agcgcaaggc tcctcacctt 7920

ggaggaggcg tgccagttga ctccacccca ttctgcaaga tccaagtatg gattcggggc 7980

caaggaggtc cgcagcttgt ccgggagggc cgttaaccac atcaagtccg tgtggaagga 8040

cctcctggaa gacccacaaa caccaattcc cacaaccatc atggccaaaa atgaggtgtt 8100

ctgcgtggac cccgccaagg ggggtaagaa accagctcgc ctcatcgttt accctgacct 8160

cggcgtccgg gtctgcgaga aaatggccct ctatgacatt acacaaaagc ttcctcaggc 8220

ggtaatggga gcttcctatg gcttccagta ctcccctgcc caacgggtgg agtatctctt 8280

gaaagcatgg gcggaaaaga aggaccccat gggtttttcg tatgataccc gatgcttcga 8340

ctcaaccgtc actgagagag acatcaggac cgaggagtcc atataccagg cctgctccct 8400

gcccgaggag gcccgcactg ccatacactc gctgactgag agactttacg taggagggcc 8460

catgttcaac agcaagggtc aaacctgcgg ttacagacgt tgccgcgcca gcggggtgct 8520

aaccactagc atgggtaaca ccatcacatg ctatgtgaaa gccctagcgg cctgcaaggc 8580

tgcggggata gttgcgccca caatgctggt atgcggcgat gacctagtag tcatctcaga 8640

aagccagggg actgaggagg acgagcggaa cctgagagcc ttcacggagg ccatgaccag 8700

gtactctgcc cctcctggtg atccccccag accggaatat gacctggagc taataacatc 8760

ctgttcctca aatgtgtctg tggcgttggg cccgcggggc cgccgcagat actacctgac 8820

cagagaccca accactccac tcgcccgggc tgcctgggaa acagttagac actcccctat 8880

caattcatgg ctgggaaaca tcatccagta tgctccaacc atatgggttc gcatggtcct 8940

aatgacacac ttcttctcca ttctcatggt ccaagacacc ctggaccaga acctcaactt 9000

tgagatgtat ggatcagtat actccgtgaa tcctttggac cttccagcca taattgagag 9060

gttacacggg cttgacgcct tttctatgca cacatactct caccacgaac tgacgcgggt 9120

ggcttcagcc ctcagaaaac ttggggcgcc acccctcagg gtgtggaaga gtcgggctcg 9180

cgcagtcagg gcgtccctca tctcccgtgg agggaaagcg gccgtttgcg gccgatatct 9240

cttcaattgg gcggtgaaga ccaagctcaa actcactcca ttgccggagg cgcgcctact 9300

ggacttatcc agttggttca ccgtcggcgc cggcgggggc gacatttttc acagcgtgtc 9360

gcgcgcccga ccccgctcat tactcttcgg cctactccta cttttcgtag gggtaggcct 9420

cttcctactc cccgctcggt agagcggcac acactaggta cactccatag ctaactgttc 9480

cttttttttt tttttttttt tttttttttt tttttttttt ttttcttttt tttttttttc 9540

cctctttctt cccttctcat cttattctac tttctttctt ggtggctcca tcttagccct 9600

agtcacggct agctgtgaaa ggtccgtgag ccgcatgact gcagagagtg ccgtaactgg 9660

tctctctgca gatcatgtct aga 9683

<210> 3
<211> 9669
<212> DNA
<213> Artificial

<220>
<223> Cloned HCV genomic cDNA in pTH/JFH-1

<400> 3
acctgcccct aataggggcg acactccgcc atgaatcact cccctgtgag gaactactgt 60

cttcacgcag aaagcgccta gccatggcgt tagtatgagt gtcgtacagc ctccaggccc 120

ccccctcccg ggagagccat agtggtctgc ggaaccggtg agtacaccgg aattgccggg 180

aagactgggt cctttcttgg ataaacccac tctatgcccg gccatttggg cgtgcccccg 240

caagactgct agccgagtag cgttgggttg cgaaaggcct tgtggtactg cctgataggg 300

cgcttgcgag tgccccggga ggtctcgtag accgtgcacc atgagcacga atcctaaacc 360

tcaaagaaaa accaaacgta acaccaaccg ccgcccacag gacgtcaagt tcccgggcgg 420

tggccagatc gttggtggag tttacctgtt gccgcgcagg ggccccaggt tgggtgtgcg 480

cgcgactagg aagacttccg agcggtcgca acctcgtgga aggcgacaac ctatccccaa 540

ggatcgccga cccgagggca gggcctgggc tcagcctggg tacccttggc ccctctatgg 600

caacgagggc atggggtggg caggatggct cctgtcaccc cgtggctccc ggcctagttg 660

gggccccaat gacccccggc gcaggtcgcg taatttgggt aaagtcatcg ataccctcac 720

atgcggcttc gccgacctca tggggtacat tccgctcgtc ggcgctccct tggggggcgc 780

tgccagggcc ttggcgcatg gcgtccgggt tctggaggac ggcgtgaact atgcaacagg 840

gaatctgccc ggttgctctt tctctatctt cctcttggct ctgctgtcct gtctaaccat 900

cccagcttcc gcttatgaag tgcgcaacgt gtccggggtg taccatgtca cgaacgactg 960

ctccaactcg agcattgtgt acgagacagg ggacatgatt atgcacaccc ctgggtgcgt 1020

gccctgtgtt cgggagaaca actcctcccg ctgctgggca gcgctcactc ccacgctcgc 1080

ggccaggaac gccagcgtcc ccaccacgac aatacggcgc cacgtcgatt tgctcgttgg 1140

ggcggctgct ttctgctccg ctatgtacgt gggggatctc tgcggatctg ttttcctcgt 1200

ctcccagttg ttcaccttct cgcctcgccg gcatgagaca gtgcaggact gcaattgttc 1260

aatctatccc ggccacgtat caggtcaccg catggcttgg gatatgatga tgaactggtc 1320

acctacaaca gccctactgg tatcgcagtt actccggatc ccacaagccg tcgtggacat 1380

ggtggcgggg gcccactggg gagtcctggc gggccttgcc tactattcca tggcggggaa 1440
ctgggctaag gttttgattg tgctgctact ctttgccggc gttgatgggg cgacctacgt 1500
gacggggggg tcggaagcca gaggggcctc tggcttagca aacctctttt catttggggc 1560
gtctcagaag atccagctca taaataccaa cggcagttgg cacatcaata gaactgccct 1620
gaactgcaat gactccctcc acactgggtt tcttgccgcg ctattctaca cacacaaatt 1680
caacgcgtcc ggatgtccag agcgcatggc cagctgccgc cccattgaag agttcgctca 1740
ggggtatggt cccatcactt atgctgagcc ctccccctcg gaccagaggc cctattgctg 1800
gcactacgcg cctcgaccgt gtggtatcat acccgcgtcg caggtgtgtg gtccagtgta 1860
ctgcttcacc ccaagccctg ttgtggtggg gacgaccgat cgctccggtg cccccacgta 1920
taattggggg gcgaatgaga cggacgtgct gtatctcaac aacacgcggc cgccgcaagg 1980
caactggttc ggctgcacat ggatgaatgg caccgggttc accaagacgt gcgggggccc 2040
cccgtgcaac atcggggggg gcggcaacaa caacaccttg acctgcccca cggactgttt 2100
ccggaaacac cccgaggcca cctacaccaa atgtggttcg ggaccttggt tgacacctag 2160
gtgcatggtc gactacccat acaggctctg gcactacccc tgcaccgtta actttaccat 2220
ctttaaggtt aggatgtacg tgggaggtgt ggagcacagg ctcaacgccg catgcaattg 2280
gacccgagga gagcgttgta acttagagga cagggataga tcagagctta gcccgctgct 2340
gctgtcaaca acagagtggc aggtgctacc ttgttccttc accaccctac cggctctgtc 2400
cactggtttg atccatctcc accagaacat cgtggacgtg caatacctgt acggtatagg 2460
gtcggcggtt gtctcctatg caatcaaatg ggaatatgtc ttgttgctct tcctcctcct 2520
ggcagacgcg cgcgtctgcg cctgcttgtg gatgatgctg ctgatagctc aagctgaggc 2580
cgccttagag aacctggtgg tcctcaatgc ggcgtccctg gctggagcgc atggccttct 2640
ctctttcctt gtgttcttct gtgccgcttg gtacatcaag ggcaggttga tccccggggc 2700
ggcgtatgct ttttacggcg tatggccgct gctcctactc ctgctggcgt taccaccacg 2760
agcatacgcc atggaccggg agatggctgc atcgtgcgga ggcgcggttt ttgtaggtct 2820
ggcattcctg accttgtcac cacactataa ggcattcctc gccaagctcc tgtggtggtt 2880

gtgctatctc ctgaccctgg gggaagccat gattcaggag tgggtaccac ccatgcaggt 2940

gcgcggcggc cgcgatggca tcgcgtgggc cgtcactata ttctgcccgg gtgtggtgtt 3000

tgacattacc aaatggcttt tggcgttgct tgggcctgct tacctcttaa gggccgcttt 3060

gacacatgtg ccgtacttcg tcagagctca cgctctgata agggtatgcg ctttggtgaa 3120

gcagctcgcg gggggtaggt atgttcaggt ggcgctattg gcccttggca ggtggactgg 3180

cacctacatc tatgaccacc tcacacctat gtcggactgg gccgctagcg gcctgcgcga 3240

cttagcggtc gccgtggaac ccatcatctt cagtccgatg gagaagaagg tcatcgtctg 3300

gggagcggag acggctgcat gtggggacat tctacatgga cttcccgtgt ccgcccgact 3360

cggccaggag atcctcctcg gcccagctga tggctacacc tccaaggggt ggaagctcct 3420

tgctcccatc actgcttatg cccagcaaac acgaggcctc ctgggcgcca tagtggtgag 3480

tatgacgggg cgtgacagga cagaacaggc cggggaagtc caaatcctgt ccacagtctc 3540

tcagtccttc ctcggaacaa ccatctcggg ggttttgtgg actgtttacc acggagctgg 3600

caacaagact ctagccggct tacggggtcc ggtcacgcag atgtactcga gtgctgaggg 3660

ggacttggta ggctggccca gcccccctgg gaccaagtct ttggagccgt gcaagtgtgg 3720

agccgtcgac ctatatctgg tcacgcggaa cgctgatgtc atcccggctc ggagacgcgg 3780

ggacaagcgg ggagcattgc tctccccgag acccatttcg accttgaagg ggtcctcggg 3840

ggggccggtg ctctgcccta ggggccacgt cgttgggctc ttccgagcag ctgtgtgctc 3900

tcggggcgtg gccaaatcca tcgatttcat ccccgttgag acactcgacg ttgttacaag 3960

gtctcccact ttcagtgaca acagcacgcc accggctgtg ccccagacct atcaggtcgg 4020

gtacttgcat gctccaactg gcagtggaaa gagcaccaag gtccctgtcg cgtatgccgc 4080

ccaggggtac aaagtactag tgcttaaccc ctcggtagct gccaccctgg ggtttggggc 4140

gtacctatcc aaggcacatg gcatcaatcc caacattagg actggagtca ggaccgtgat 4200

gaccggggag gccatcacgt actccacata tggcaaattt ctcgccgatg ggggctgcgc 4260

tagcggcgcc tatgacatca tcatatgcga tgaatgccac gctgtggatg ctacctccat 4320

tctcggcatc ggaacggtcc ttgatcaagc agagacagcc ggggtcagac taactgtgct 4380

ggctacggcc acaccccccg ggtcagtgac aaccccccat cccgatatag aagaggtagg 4440

cctcgggcgg gagggtgaga tccccttcta tgggagggcg attcccctat cctgcatcaa 4500

gggagggaga cacctgattt tctgccactc aaagaaaaag tgtgacgagc tcgcggcggc 4560

ccttcggggc atgggcttga atgccgtggc atactataga gggttggacg tctccataat 4620

accagctcag ggagatgtgg tggtcgtcgc caccgacgcc ctcatgacgg ggtacactgg 4680

agactttgac tccgtgatcg actgcaatgt agcggtcacc caagctgtcg acttcagcct 4740

ggaccccacc ttcactataa ccacacagac tgtcccacaa gacgctgtct cacgcagtca 4800

gcgccgcggg cgcacaggta gaggaagaca gggcacttat aggtatgttt ccactggtga 4860

acgagcctca ggaatgtttg acagtgtagt gctttgtgag tgctacgacg caggggctgc 4920

gtggtacgat ctcacaccag cggagaccac cgtcaggctt agagcgtatt tcaacacgcc 4980

cggcctaccc gtgtgtcaag accatcttga attttgggag gcagttttca ccggcctcac 5040

acacatagac gcccacttcc tctcccaaac aaagcaagcg ggggagaact tcgcgtacct 5100

agtagcctac caagctacgg tgtgcgccag agccaaggcc cctcccccgt cctgggacgc 5160

catgtggaag tgcctggccc gactcaagcc tacgcttgcg ggccccacac ctctcctgta 5220

ccgtttgggc cctattacca atgaggtcac cctcacacac cctgggacga agtacatcgc 5280

cacatgcatg caagctgacc ttgaggtcat gaccagcacg tgggtcctag ctggaggagt 5340

cctggcagcc gtcgccgcat attgcctggc gactggatgc gtttccatca tcggccgctt 5400

gcacgtcaac cagcgagtcg tcgttgcgcc ggataaggag gtcctgtatg aggcttttga 5460

tgagatggag gaatgcgcct ctagggcggc tctcatcgaa gaggggcagc ggatagccga 5520

gatgttgaag tccaagatcc aaggcttgct gcagcaggcc tctaagcagg cccaggacat 5580

acaacccgct atgcaggctt catggcccaa agtggaacaa ttttgggcca gacacatgtg 5640

gaacttcatt agcggcatcc aatacctcgc aggattgtca acactgccag ggaaccccgc 5700

ggtggcttcc atgatggcat tcagtgccgc cctcaccagt ccgttgtcga ccagtaccac 5760

catccttctc aacatcatgg gaggctggtt agcgtcccag atcgcaccac ccgcgggggc 5820

caccggcttt gtcgtcagtg gcctggtggg ggctgccgtg ggcagcatag gcctgggtaa 5880

ggtgctggtg gacatcctgg caggatatgg tgcgggcatt tcgggggccc tcgtcgcatt 5940

caagatcatg tctggcgaga agccctctat ggaagatgtc atcaatctac tgcctgggat 6000

cctgtctccg ggagccctgg tggtgggggt catctgcgcg gccattctgc gccgccacgt 6060

gggaccgggg gagggcgcgg tccaatggat gaacaggctt attgcctttg cttccagagg 6120

aaaccacgtc gcccctactc actacgtgac ggagtcggat gcgtcgcagc gtgtgaccca 6180

actacttggc tctcttacta taaccagcct actcagaaga ctccacaatt ggataactga 6240

ggactgcccc atcccatgct ccggatcctg gctccgcgac gtgtgggact gggtttgcac 6300

catcttgaca gacttcaaaa attggctgac ctctaaattg ttccccaagc tgcccggcct 6360

ccccttcatc tcttgtcaaa aggggtacaa gggtgtgtgg gccggcactg gcatcatgac 6420

cacgcgctgc ccttgcggcg ccaacatctc tggcaatgtc cgcctgggct ctatgaggat 6480

cacagggcct aaaacctgca tgaacacctg gcaggggacc tttcctatca attgctacac 6540

ggagggccag tgcgcgccga aaccccccac gaactacaag accgccatct ggagggtggc 6600

ggcctcggag tacgcggagg tgacgcagca tgggtcgtac tcctatgtaa caggactgac 6660

cactgacaat ctgaaaattc cttgccaact accttctcca gagtttttct cctgggtgga 6720

cggtgtgcag atccataggt ttgcacccac accaaagccg tttttccggg atgaggtctc 6780

gttctgcgtt gggcttaatt cctatgctgt cgggtcccag cttccctgtg aacctgagcc 6840

cgacgcagac gtattgaggt ccatgctaac agatccgccc cacatcacgg cggagactgc 6900

ggcgcggcgc ttggcacggg gatcacctcc atctgaggcg agctcctcag tgagccagct 6960

atcagcaccg tcgctgcggg ccacctgcac cacccacagc aacacctatg acgtggacat 7020

ggtcgatgcc aacctgctca tggagggcgg tgtggctcag acagagcctg agtccagggt 7080

gcccgttctg gactttctcg agccaatggc cgaggaagag agcgaccttg agccctcaat 7140

accatcggag tgcatgctcc ccaggagcgg gtttccacgg gccttaccgg cttgggcacg 7200

gcctgactac aacccgccgc tcgtggaatc gtggaggagg ccagattacc aaccgcccac 7260

cgttgctggt tgtgctctcc ccccccccaa gaaggccccg acgcctcccc caaggagacg 7320

ccggacagtg ggtctgagcg agagcaccat atcagaagcc ctccagcaac tggccatcaa 7380

gacctttggc cagcccccct cgagcggtga tgcaggctcg tccacggggg cgggcgccgc 7440

cgaatccggc ggtccgacgt cccctggtga gccggccccc tcagagacag gttccgcctc 7500

ctctatgccc cccctcgagg gggagcctgg agatccggac ctggagtctg atcaggtaga 7560

gcttcaacct cccccccagg gggggggggt agctcccggt tcgggctcgg ggtcttggtc 7620

tacttgctcc gaggaggacg ataccaccgt gtgctgctcc atgtcatact cctggaccgg 7680

ggctctaata actccctgta gccccgaaga ggaaaagttg ccaatcaacc ctttgagtaa 7740

ctcgctgttg cgataccata acaaggtgta ctgtacaaca tcaaagagcg cctcacagag 7800

ggctaaaaag gtaacttttg acaggacgca agtgctcgac gcccattatg actcagtctt 7860

aaaggacatc aagctagcgg cttccaaggt cagcgcaagg ctcctcacct tggaggaggc 7920

gtgccagttg actccacccc attctgcaag atccaagtat ggattcgggg ccaaggaggt 7980

ccgcagcttg tccgggaggg ccgttaacca catcaagtcc gtgtggaagg acctcctgga 8040

agacccacaa acaccaattc ccacaaccat catggccaaa aatgaggtgt tctgcgtgga 8100

ccccgccaag gggggtaaga aaccagctcg cctcatcgtt taccctgacc tcggcgtccg 8160

ggtctgcgag aaaatggccc tctatgacat tacacaaaag cttcctcagg cggtaatggg 8220

agcttcctat ggcttccagt actcccctgc ccaacgggtg gagtatctct tgaaagcatg 8280

ggcggaaaag aaggacccca tgggtttttc gtatgatacc cgatgcttcg actcaaccgt 8340

cactgagaga gacatcagga ccgaggagtc catataccag gcctgctccc tgcccgagga 8400

ggcccgcact gccatacact cgctgactga gagactttac gtaggagggc ccatgttcaa 8460

cagcaagggt caaacctgcg gttacagacg ttgccgcgcc agcggggtgc taaccactag 8520

catgggtaac accatcacat gctatgtgaa agccctagcg gcctgcaagg ctgcggggat 8580

agttgcgccc acaatgctgg tatgcggcga tgacctagta gtcatctcag aaagccaggg 8640

gactgaggag gacgagcgga acctgagagc cttcacggag gccatgacca ggtactctgc 8700

ccctcctggt gatcccccca gaccggaata tgacctggag ctaataacat cctgttcctc 8760

aaatgtgtct gtggcgttgg gcccgcgggg ccgccgcaga tactacctga ccagagaccc 8820

aaccactcca ctcgcccggg ctgcctggga aacagttaga cactccccta tcaattcatg 8880

gctgggaaac atcatccagt atgctccaac catatgggtt cgcatggtcc taatgacaca 8940

cttcttctcc attctcatgg tccaagacac cctggaccag aacctcaact ttgagatgta 9000

tggatcagta tactccgtga atcctttgga ccttccagcc ataattgaga ggttacacgg 9060

gcttgacgcc ttttctatgc acacatactc tcaccacgaa ctgacgcggg tggcttcagc 9120

cctcagaaaa cttggggcgc cacccctcag ggtgtggaag agtcgggctc gcgcagtcag 9180

ggcgtccctc atctcccgtg gagggaaagc ggccgtttgc ggccgatatc tcttcaattg 9240

ggcggtgaag accaagctca aactcactcc attgccggag gcgcgcctac tggacttatc 9300

cagttggttc accgtcggcg ccggcggggg cgacattttt cacagcgtgt cgcgcgcccg 9360

accccgctca ttactcttcg gcctactcct acttttcgta ggggtaggcc tcttcctact 9420

ccccgctcgg tagagcggca cacactaggt acactccata gctaactgtt cctttttttt 9480

tttttttttt tttttttttt tttttttttt tttttctttt tttttttttt ccctctttct 9540

tcccttctca tcttattcta ctttctttct tggtggctcc atcttagccc tagtcacggc 9600

tagctgtgaa aggtccgtga gccgcatgac tgcagagagt gccgtaactg gtctctctgc 9660

agatcatgt 9669

<210> 4
<211> 18
<212> DNA
<213> Artificial

<220>
<223> primer

<400> 4
tgtaaaacga cggccagt 18

<210> 5
<211> 40
<212> DNA
<213> Artificial

<220>
<223> primer

<400> 5

ggtttaggat tcgtgctcat ggtgcacggt ctacgagacc 40

<210> 6
<211> 40
<212> DNA
<213> Artificial

<220>
<223> primer

<400> 6
ggtctcgtag accgtgcacc atgagcacga atcctaaacc 40

<210> 7
<211> 40
<212> DNA
<213> Artificial

<220>
<223> primer

<400> 7
agatagcaca accaccacag gagcttggcg aggaatgcct 40

<210> 8
<211> 40
<212> DNA
<213> Artificial

<220>
<223> primer

<400> 8
aggcattcct cgccaagctc ctgtggtggt tgtgctatct 40

<210> 9
<211> 20
<212> DNA
<213> Artificial

<220>
<223> primer

<400> 9
cagctaccga ggggttaagc 20

<210> 10
<211> 30
<212> DNA
<213> Artificial

<220>
<223> primer

<400> 10
cacaagcttg ccgaagtgaa gaacatcagt 30

<210> 11
<211> 32
<212> DNA
<213> Artificial

<220>
<223> primer

<400> 11
gctctagatt aatgagcccc gctaatgatg tc 32

<210> 12
<211> 28
<212> DNA
<213> Artificial

<220>
<223> primer

<400> 12
cacaagcttc gcacccatac tgttgggg 28

<210> 13
<211> 32
<212> DNA
<213> Artificial

<220>
<223> primer

<400> 13
gctctagatt accatcggac gatgtatttt gt 32

<210> 14

<211>  30
<212>  DNA
<213>  Artificial

<220>
<223>  primer

<400>  14
cacaagcttg cccaggtgaa gaataccagt                                        30


<210>  15
<211>  32
<212>  DNA
<213>  Artificial

<220>
<223>  primer

<400>  15
gctctagatt agtgagcccc gctaacgatg tc                                     32


<210>  16
<211>  28
<212>  DNA
<213>  Artificial

<220>
<223>  primer

<400>  16
cacaagcttg gcaccaccac cgttggag                                          28


<210>  17
<211>  32
<212>  DNA
<213>  Artificial

<220>
<223>  primer

<400>  17
gctctagatt atgtgatagc aggtgagagg cc                                     32


<210>  18
<211>  10
<212>  PRT

<213>  Artificial

<220>
<223>  synthetic peptide

<400>  18

Leu Val His Tyr Pro Tyr Arg Leu Trp His
1               5                   10


<210>  19
<211>  10
<212>  PRT
<213>  Artificial

<220>
<223>  synthetic peptide

<400>  19

Trp Leu Thr Pro Lys Cys Leu Val His Tyr
1               5                   10


<210>  20
<211>  10
<212>  PRT
<213>  Artificial

<220>
<223>  synthetic peptide

<400>  20

Pro Lys Cys Leu Val His Tyr Pro Tyr Arg
1               5                   10


<210>  21
<211>  10
<212>  PRT
<213>  Artificial

<220>
<223>  synthetic peptide

<400>  21

Tyr Pro Tyr Arg Leu Trp His Tyr Pro Cys

1                5                   10


<210>  22
<211>  10
<212>  PRT
<213>  Artificial

<220>
<223>  synthetic peptide

<400>  22

Asn Phe Thr Ile Phe Lys Ile Arg Met Tyr
1               5                   10



<210>  23
<211>  10
<212>  PRT
<213>  Artificial

<220>
<223>  synthetic peptide

<400>  23

Ile Phe Lys Ile Arg Met Tyr Val Cys Gly
1               5                   10



<210>  24
<211>  19
<212>  PRT
<213>  Artificial

<220>
<223>  synthetic peptide

<400>  24

Trp Leu Thr Pro Lys Cys Leu Val His Tyr Pro Tyr Arg Leu Trp His
1               5                   10                  15


Tyr Pro Cys




<210>  25

<211>  162
<212>  PRT
<213>  Hepatitis C virus strain JFH-1

<400>  25

Ala Gln Val Lys Asn Thr Ser Ser Ser Tyr Met Val Thr Asn Asp Cys
1               5                   10                  15

Ser Asn Asp Ser Ile Thr Trp Gln Leu Glu Ala Ala Val Leu His Val
            20                  25                  30

Pro Gly Cys Val Pro Cys Glu Arg Val Gly Asn Thr Ser Arg Cys Trp
        35                  40                  45

Val Pro Val Ser Pro Asn Met Ala Val Arg Gln Pro Gly Ala Leu Thr
    50                  55                  60

Gln Gly Leu Arg Thr His Ile Asp Met Val Val Met Ser Ala Thr Phe
65                  70                  75                  80

Cys Ser Ala Leu Tyr Val Gly Asp Leu Cys Gly Gly Val Met Leu Ala
                85                  90                  95

Ala Gln Val Phe Ile Val Ser Pro Gln Tyr His Trp Phe Val Gln Glu
            100                 105                 110

Cys Asn Cys Ser Ile Tyr Pro Gly Thr Ile Thr Gly His Arg Met Ala
        115                 120                 125

Trp Asp Met Met Met Asn Trp Ser Pro Thr Ala Thr Met Ile Leu Ala
    130                 135                 140

Tyr Val Met Arg Val Pro Glu Val Ile Ile Asp Ile Val Ser Gly Ala
145                 150                 155                 160

His Trp

<210> 26
<211> 337
<212> PRT
<213> Hepatitis C virus strain JFH-1

<400> 26

Gly Thr Thr Thr Val Gly Gly Ala Val Ala Arg Ser Thr Asn Val Ile
1               5                   10                  15

Ala Gly Val Phe Ser His Gly Pro Gln Gln Asn Ile Gln Leu Ile Asn
            20                  25                  30

Thr Asn Gly Ser Trp His Ile Asn Arg Thr Ala Leu Asn Cys Asn Asp
        35                  40                  45

Ser Leu Asn Thr Gly Phe Leu Ala Ala Leu Phe Tyr Thr Asn Arg Phe
    50                  55                  60

Asn Ser Ser Gly Cys Pro Gly Arg Leu Ser Ala Cys Arg Asn Ile Glu
65                  70                  75                  80

Ala Phe Arg Ile Gly Trp Gly Thr Leu Gln Tyr Glu Asp Asn Val Thr
                85                  90                  95

Asn Pro Glu Asp Met Arg Pro Tyr Cys Trp His Tyr Pro Pro Lys Pro
            100                 105                 110

Cys Gly Val Val Pro Ala Arg Ser Val Cys Gly Pro Val Tyr Cys Phe
        115                 120                 125

Thr Pro Ser Pro Val Val Val Gly Thr Thr Asp Arg Arg Gly Val Pro
    130                 135                 140

Thr Tyr Thr Trp Gly Glu Asn Glu Thr Asp Val Phe Leu Leu Asn Ser
145                 150                 155                 160

Thr Arg Pro Pro Gln Gly Ser Trp Phe Gly Cys Thr Trp Met Asn Ser
                165                 170                 175

Thr Gly Phe Thr Lys Thr Cys Gly Ala Pro Pro Cys Arg Thr Arg Ala
            180                 185                 190

Asp Phe Asn Ala Ser Thr Asp Leu Leu Cys Pro Thr Asp Cys Phe Arg
        195                 200                 205

Lys His Pro Asp Ala Thr Tyr Ile Lys Cys Gly Ser Gly Pro Trp Leu
    210                 215                 220

Thr Pro Lys Cys Leu Val His Tyr Pro Tyr Arg Leu Trp His Tyr Pro
225                 230                 235                 240

Cys Thr Val Asn Phe Thr Ile Phe Lys Ile Arg Met Tyr Val Gly Gly
                245                 250                 255

Val Glu His Arg Leu Thr Ala Ala Cys Asn Phe Thr Arg Gly Asp Arg
            260                 265                 270

Cys Asp Leu Glu Asp Arg Asp Arg Ser Gln Leu Ser Pro Leu Leu His
        275                 280                 285

Ser Thr Thr Glu Trp Ala Ile Leu Pro Cys Thr Tyr Ser Asp Leu Pro
    290                 295                 300

Ala Leu Ser Thr Gly Leu Leu His Leu His Gln Asn Ile Val Asp Val
305                 310                 315                 320

Gln Tyr Met Tyr Gly Leu Ser Pro Ala Ile Thr Lys Tyr Val Val Arg
                325                 330                 335

Trp

<210>  27
<211>  162
<212>  PRT
<213>  Hepatitis C virus strain J6CF

<220>

<223>  E1(1-162)

<400>  27

Ala Glu Val Lys Asn Ile Ser Thr Gly Tyr Met Val Thr Asn Asp Cys
1               5                   10                  15

Thr Asn Asp Ser Ile Thr Trp Gln Leu Gln Ala Ala Val Leu His Val
            20                  25                  30

Pro Gly Cys Val Pro Cys Glu Lys Val Gly Asn Ala Ser Gln Cys Trp
        35                  40                  45

Ile Pro Val Ser Pro Asn Val Ala Val Gln Arg Pro Gly Ala Leu Thr
    50                  55                  60

Gln Gly Leu Arg Thr His Ile Asp Met Val Val Met Ser Ala Thr Leu
65                  70                  75                  80

Cys Ser Ala Leu Tyr Val Gly Asp Leu Cys Gly Gly Val Met Leu Ala
                85                  90                  95

Ala Gln Met Phe Ile Val Ser Pro Gln His His Trp Phe Val Gln Asp
            100                 105                 110

Cys Asn Cys Ser Ile Tyr Pro Gly Thr Ile Thr Gly His Arg Met Ala
        115                 120                 125

Trp Asp Met Met Met Asn Trp Ser Pro Thr Ala Thr Met Ile Leu Ala
    130                 135                 140

Tyr Ala Met Arg Val Pro Glu Val Ile Ile Asp Ile Ile Ser Gly Ala
145                 150                 155                 160

His Trp

<210>  28
<211>  337

<212>  PRT
<213>  Hepatitis C virus strain J6CF

<220>
<223>  E2(1-337)

<400>  28

Arg Thr His Thr Val Gly Gly Ser Ala Ala Gln Thr Thr Gly Arg Leu
1               5                   10                  15

Thr Ser Leu Phe Asp Met Gly Pro Arg Gln Lys Ile Gln Leu Val Asn
            20                  25                  30

Thr Asn Gly Ser Trp His Ile Asn Arg Thr Ala Leu Asn Cys Asn Asp
        35                  40                  45

Ser Leu His Thr Gly Phe Ile Ala Ser Leu Phe Tyr Thr His Ser Phe
    50                  55                  60

Asn Ser Ser Gly Cys Pro Glu Arg Met Ser Ala Cys Arg Ser Ile Glu
65                  70                  75                  80

Ala Phe Arg Val Gly Trp Gly Ala Leu Gln Tyr Glu Asp Asn Val Thr
                85                  90                  95

Asn Pro Glu Asp Met Arg Pro Tyr Cys Trp His Tyr Pro Pro Arg Gln
            100                 105                 110

Cys Gly Val Val Ser Ala Lys Thr Val Cys Gly Pro Val Tyr Cys Phe
        115                 120                 125

Thr Pro Ser Pro Val Val Val Gly Thr Thr Asp Arg Leu Gly Ala Pro
    130                 135                 140

Thr Tyr Thr Trp Gly Glu Asn Glu Thr Asp Val Phe Leu Leu Asn Ser
145                 150                 155                 160

Thr Arg Pro Pro Leu Gly Ser Trp Phe Gly Cys Thr Trp Met Asn Ser
                165                 170                 175

Ser Gly Tyr Thr Lys Thr Cys Gly Ala Pro Pro Cys Arg Thr Arg Ala
            180                 185                 190

Asp Phe Asn Ala Ser Thr Asp Leu Leu Cys Pro Thr Asp Cys Phe Arg
        195                 200                 205

Lys His Pro Asp Thr Thr Tyr Leu Lys Cys Gly Ser Gly Pro Trp Leu
    210                 215                 220

Thr Pro Arg Cys Leu Ile Asp Tyr Pro Tyr Arg Leu Trp His Tyr Pro
225                 230                 235                 240

Cys Thr Val Asn Tyr Thr Ile Phe Lys Ile Arg Met Tyr Val Gly Gly
                245                 250                 255

Val Glu His Arg Leu Thr Ala Ala Cys Asn Phe Thr Arg Gly Asp Arg
            260                 265                 270

Cys Asn Leu Glu Asp Arg Asp Arg Ser Gln Leu Ser Pro Leu Leu His
        275                 280                 285

Ser Thr Thr Glu Trp Ala Ile Leu Pro Cys Ser Tyr Ser Asp Leu Pro
    290                 295                 300

Ala Leu Ser Thr Gly Leu Leu His Leu His Gln Asn Ile Val Asp Val
305                 310                 315                 320

Gln Phe Met Tyr Gly Leu Ser Pro Ala Leu Thr Lys Tyr Ile Val Arg
                325                 330                 335

Trp

<210> 29
<211> 10
<212> PRT
<213> Artificial

<220>
<223>  synthetic peptide

<400>  29

Asn Val Thr Asn Pro Glu Asp Met Arg Pro
1               5                   10


<210>  30
<211>  10
<212>  PRT
<213>  Artificial

<220>
<223>  synthetic peptide

<400>  30

Asn Pro Glu Asp Met Arg Pro Tyr Cys Trp
1               5                   10


<210>  31
<211>  10
<212>  PRT
<213>  Artificial

<220>
<223>  synthetic peptide

<400>  31

Asn Tyr Thr Ile Phe Lys Ile Arg Met Tyr
1               5                   10


<210>  32
<211>  10
<212>  PRT
<213>  Artificial

<220>
<223>  synthetic peptide

<400>  32

Ile Phe Lys Ile Arg Met Tyr Val Gly Gly
1               5                   10

<210>  33
<211>  10
<212>  PRT
<213>  Artificial

<220>
<223>  synthetic peptide

<400>  33

Phe Ile Val Ser Pro Gln His His Trp Phe
1               5                   10


<210>  34
<211>  10
<212>  PRT
<213>  Artificial

<220>
<223>  synthetic peptide

<400>  34

Ser Pro Gln His His Trp Phe Val Gln Asp
1               5                   10


<210>  35
<211>  10
<212>  PRT
<213>  Artificial

<220>
<223>  synthetic peptide

<400>  35

His His Trp Phe Val Gln Asp Cys Asn Cys
1               5                   10


<210>  36
<211>  10
<212>  PRT
<213>  Artificial

<220>

<223>  synthetic peptide

<400>  36

Met Ala Trp Asp Met Met Met Asn Trp Ser
1               5                   10

<210>  37
<211>  10
<212>  PRT
<213>  Artificial

<220>
<223>  synthetic peptide

<400>  37

Leu Ile Asp Tyr Pro Tyr Arg Leu Trp His
1               5                   10

<210>  38
<211>  10
<212>  PRT
<213>  Artificial

<220>
<223>  synthetic peptide

<400>  38

Tyr Pro Tyr Arg Leu Trp His Tyr Pro Cys
1               5                   10

<210>  39
<211>  10
<212>  PRT
<213>  Artificial

<220>
<223>  synthetic peptide

<400>  39

Asp Met Arg Pro Tyr Cys Trp His Tyr Pro
1               5                   10

<210> 40
<211> 10
<212> PRT
<213> Artificial

<220>
<223> synthetic peptide

<400> 40

Asn Pro Glu Asp Met Arg Pro Tyr Cys Trp
1               5                   10


<210> 41
<211> 10
<212> PRT
<213> Artificial

<220>
<223> synthetic peptide

<400> 41

Pro Tyr Cys Trp His Tyr Pro Pro Arg Gln
1               5                   10


<210> 42
<211> 10
<212> PRT
<213> Artificial

<220>
<223> synthetic peptide

<400> 42

Ser Thr Arg Pro Pro Leu Gly Ser Trp Phe
1               5                   10


<210> 43
<211> 10
<212> PRT
<213> Artificial

<220>
<223> synthetic peptide

<400> 43

Gly Ser Trp Phe Gly Cys Thr Trp Met Asn
1               5                   10

<210> 44
<211> 14
<212> PRT
<213> Artificial

<220>
<223> synthetic peptide

<400> 44

Asn Val Thr Asn Pro Glu Asp Met Pro Arg Pro Tyr Cys Trp
1               5                   10

<210> 45
<211> 13
<212> PRT
<213> Artificial

<220>
<223> synthetic peptide

<400> 45

Asn Tyr Thr Ile Phe Lys Ile Arg Met Tyr Val Gly Gly
1               5                   10

<210> 46
<211> 16
<212> PRT
<213> Artificial

<220>
<223> synthetic peptide

<400> 46

Phe Ile Val Ser Pro Gln His His Trp Phe Val Gln Asp Cys Asn Cys
1               5                   10                  15

<210> 47
<211> 13

<212>  PRT
<213>  Artificial

<220>
<223>  synthetic peptide

<400>  47

Leu Ile Asp Tyr Pro Tyr Arg Leu Trp His Tyr Pro Cys
1               5                   10


<210>  48
<211>  16
<212>  PRT
<213>  Artificial

<220>
<223>  synthetic peptide

<400>  48

Asn Pro Glu Asp Met Arg Pro Tyr Cys Trp His Tyr Pro Pro Arg Gln
1               5                   10                  15


<210>  49
<211>  36
<212>  DNA
<213>  Artificial

<220>
<223>  primer

<400>  49
aggcttacta gtagctggaa tgggcacatg cagatc                                36


<210>  50
<211>  35
<212>  DNA
<213>  Artificial

<220>
<223>  primer

<400>  50
catgtctaga acactcattc ctgttgaagc tcttg                                 35

<210> 51
<211> 360
<212> DNA
<213> Mus musculus

<400> 51
gaggttcagc tccagcagtc tgggactgtg ctggcaaggc ctggggcttc agtgaagatg 60

tcctgcaagg cttctggcta cacctttacc agctactgga tgcactgggt aaaacagagg 120

cctggacagg gtctggaatg gattggcgct atttatcctg gaaatagtga tactagctac 180

aaccagaagt tcaagggcaa ggccaaactg actgcagtca catccaccag cactgcctac 240

atggagctca gcagcctgac aaatgaggac tctgcggtct attactgtac aagagatggt 300

tactacaggg gaaactactt tgactactgg ggccaaggca ccactctcac agtctcctca 360

<210> 52
<211> 120
<212> PRT
<213> Mus musculus

<400> 52

Glu Val Gln Leu Gln Gln Ser Gly Thr Val Leu Ala Arg Pro Gly Ala
1               5                   10                  15

Ser Val Lys Met Ser Cys Lys Ala Ser Gly Tyr Thr Phe Thr Ser Tyr
            20                  25                  30

Trp Met His Trp Val Lys Gln Arg Pro Gly Gln Gly Leu Glu Trp Ile
        35                  40                  45

Gly Ala Ile Tyr Pro Gly Asn Ser Asp Thr Ser Tyr Asn Gln Lys Phe
    50                  55                  60

Lys Gly Lys Ala Lys Leu Thr Ala Val Thr Ser Thr Ser Thr Ala Tyr
65                  70                  75                  80

Met Glu Leu Ser Ser Leu Thr Asn Glu Asp Ser Ala Val Tyr Tyr Cys
                85                  90                  95

Thr Arg Asp Gly Tyr Tyr Arg Gly Asn Tyr Phe Asp Tyr Trp Gly Gln
            100                 105                 110

Gly Thr Thr Leu Thr Val Ser Ser
        115                 120

<210> 53
<211> 336
<212> DNA
<213> Mus musculus

<220>
<221> misc_feature
<222> (295)..(297)
<223> n is a, c, g, or t

<220>
<223>

<400> 53
gacattgtgc tgacacagtc tcctgcttcc ttagctgtat ctctggggca gagggccacc      60

atctcataca gggccagcaa aagtgtcagt acatctggct atagttatat gcactggaac     120

caacagaaac caggacagcc acccagactc ctcatctatc ttgtatccaa cctagaatct     180

ggggtccctg ccaggttcag tggcagtggg tctgggacag acttcaccct caacatccat     240

cctgtggagg aggaggatgc tgcaacctat tactgtcagc acattaggga gcttnnnacg     300

ttcggagggg ggaccaagct ggaaataaaa cgggct                               336

<210> 54
<211> 114
<212> PRT
<213> Mus musculus

<220>
<221> misc_feature
<222> (101)..(101)
<223> Xaa can be any naturally occurring amino acid

<220>
<223>

<400> 54

Asp Ile Val Leu Thr Gln Ser Pro Ala Ser Leu Ala Val Ser Leu Gly
1               5                   10                  15

Gln Arg Ala Thr Ile Ser Tyr Arg Ala Ser Lys Ser Val Ser Thr Ser
            20                  25                  30

Gly Tyr Ser Tyr Met His His Trp Asn Gln Gln Lys Pro Gly Gln Pro
        35                  40                  45

Pro Arg Leu Leu Ile Tyr Leu Val Ser Asn Leu Glu Ser Gly Val Pro
    50                  55                  60

Ala Arg Phe Ser Gly Ser Gly Ser Gly Thr Asp Phe Thr Leu Asn Ile
65                  70                  75                  80

His Pro Val Glu Glu Glu Asp Ala Ala Thr Tyr Tyr Cys Gln His His
                85                  90                  95

Ile Arg Glu Leu Xaa Thr Phe Gly Gly Gly Thr Lys Leu Glu Ile Lys
            100                 105                 110

Arg Ala

<210> 55
<211> 31
<212> PRT
<213> Mus musculus

<220>
<223> Framework 1 of H chain V region of monoclonal antibody JF/M1-4

<400> 55

Glu Val Gln Leu Gln Gln Ser Gly Thr Val Leu Ala Arg Pro Gly Ala
1               5                   10                  15

Ser Val Lys Met Ser Cys Lys Ala Ser Gly Tyr Thr Phe Thr Ser
            20                  25                  30

<210>  56
<211>  4
<212>  PRT
<213>  Mus musculus

<220>
<223>  CDR 1 of H chain V region of monoclonal antibody JF/M1-4

<400>  56

Tyr Trp Met His
1

<210>  57
<211>  14
<212>  PRT
<213>  Mus musculus

<220>
<223>  Framework 2 of H chain V region of monoclonal antibody JF/M1-4

<400>  57

Trp Val Lys Gln Arg Pro Gly Gln Gly Leu Glu Trp Ile Gly
1               5                   10

<210>  58
<211>  17
<212>  PRT
<213>  Mus musculus

<220>
<223>  CDR 2 of H chain V region of monoclonal antibody JF/M1-4

<400>  58

Ala Ile Tyr Pro Gly Asn Ser Asp Thr Ser Tyr Asn Gln Lys Phe Lys
1               5                   10                  15

Gly

<210>  59
<211>  32

<212>  PRT
<213>  Mus musculus

<220>
<223>  Framework 3 of H chain V region of monoclonal antibody JF/M1-4

<400>  59

Lys Ala Lys Leu Thr Ala Val Thr Ser Thr Ser Thr Ala Tyr Met Glu
1               5                   10                  15

Leu Ser Ser Leu Thr Asn Glu Asp Ser Ala Val Tyr Tyr Cys Thr Arg
            20                  25                  30

<210>  60
<211>  11
<212>  PRT
<213>  Mus musculus

<220>
<223>  CDR 3 of H chain V region of monoclonal antibody JF/M1-4

<400>  60

Asp Gly Tyr Tyr Arg Gly Asn Tyr Phe Asp Tyr
1               5                   10

<210>  61
<211>  11
<212>  PRT
<213>  Mus musculus

<220>
<223>  Framework 4 (J segment) of H chain V region of monoclonal antibody JF/M1-4

<400>  61

Trp Gly Gln Gly Thr Thr Leu Thr Val Ser Ser
1               5                   10

<210>  62
<211>  23
<212>  PRT
<213>  Mus musculus

<220>

<223>  Framework 1 of L chain V region of monoclonal antibody JF/M1-4

<400>  62

Asp Ile Val Leu Thr Gln Ser Pro Ala Ser Leu Ala Val Ser Leu Gly
1               5                   10                  15

Gln Arg Ala Thr Ile Ser Tyr
            20

<210>  63
<211>  15
<212>  PRT
<213>  Mus musculus

<220>
<223>  CDR 1 of L chain V region of monoclonal antibody JF/M1-4

<400>  63

Arg Ala Ser Lys Ser Val Ser Thr Ser Gly Tyr Ser Tyr Met His
1               5                   10                  15

<210>  64
<211>  16
<212>  PRT
<213>  Mus musculus

<220>
<223>  Framework 2 of L chain V region of monoclonal antibody JF/M1-4

<400>  64

His Trp Asn Gln Gln Lys Pro Gly Gln Pro Pro Arg Leu Leu Ile Tyr
1               5                   10                  15

<210>  65
<211>  7
<212>  PRT
<213>  Mus musculus

<220>
<223>  CDR 2 of L chain V region of monoclonal antibody JF/M1-4

<400>  65

Leu Val Ser Asn Leu Glu Ser
1               5

<210>  66
<211>  32
<212>  PRT
<213>  Mus musculus

<220>
<223>  Framework 3 of L chain V region of monoclonal antibody JF/M1-4

<400>  66

Gly Val Pro Ala Arg Phe Ser Gly Ser Gly Ser Gly Thr Asp Phe Thr
1               5                   10                  15

Leu Asn Ile His Pro Val Glu Glu Glu Asp Ala Ala Thr Tyr Tyr Cys
            20                  25                  30

<210>  67
<211>  9
<212>  PRT
<213>  Mus musculus

<220>
<221>  misc_feature
<222>  (8)..(8)
<223>  Xaa can be any naturally occurring amino acid

<220>
<223>  CDR 3 of L chain V region of monoclonal antibody JF/M1-4

<400>  67

Gln His His Ile Arg Glu Leu Xaa Thr
1               5

<210>  68
<211>  12
<212>  PRT
<213>  Mus musculus

<220>
<223>  Framework 4 (J chain) of L chain V region of monoclonal antibody JF/M1-4

<400> 68

Phe Gly Gly Gly Thr Lys Leu Glu Ile Lys Arg Ala
1               5                   10

## Description

### Technical Field

The present invention relates to an antibody having inhibitory activity on infection with hepatitis C virus (HCV) and use thereof.

### Background Art

Hepatitis C viruses (HCV) were found and identified as major causative viruses of non-A and non-B hepatitis (Non-patent Document 1). Further, highly sensitive methods for detecting HCV had been established recent years, and the number of new HCV patients because of blood transfusion dramatically decreased. However, the number of virus carriers in Japan is deduced to be over 2,000,000, and the world wide figure is over 170,000,000, including so-called virus carriers who have not yet developed hepatitis symptoms. This is mainly because the rate of chronicity of hepatitis due to HCV infection is as high as 70% to 80% and there are no effective antivirus agents other than interferons at present. Further, hepatitis C is a serious infection with a poor prognosis since approximately half of the patients with chronic hepatitis C would unexceptionally experience worsening of symptoms from hepatitis C to cirrhosis and hepatic cancer. Accordingly, development of antivirus agents or vaccines aimed at preventing virus carriers from developing the disease or eliminating viruses has been awaited.

When a virus having an envelope such as HCV infects a cell, a virus first needs to bind to a specific protein (i.e., a virus receptor) on the cell surface. Thereafter, the virus membrane is fused with a cell membrane and the virus gene is injected into the cell. Treatment and prevention of HCV require the development of an antibody that can inhibit cellular infection. In particular, an antibody that can prevent HCV from binding to a virus receptor on the cell surface and invading into the cell is important.

An HCV envelope protein is considered to play a key role in binding of HCV to a cell surface. Thus, research has been made regarding detection of an antibody reacting with an envelope protein in the blood serum of the patient. The percentage of patients exhibiting positive reactions with either the C100 antibody (the NS4-NS5 antibody) or the core antibody and exhibiting positive reaction with an envelope protein antibody was found to be approximately 10%. Since only 3 of 31 patients exhibiting positive reactions with the envelope protein antibody are naturally cured, the development of neutralizing antibodies in these 3 patients was considered (Non-patent Document 2). The percentage of patients who experienced the development of neutralizing antibodies was as low as 1% of all patients.

In the case of acute hepatitis B, however, development of antibodies against envelope proteins of the hepatitis B virus is 100%, and viruses are destroyed. In the case of HIV, antibodies against HIV envelope proteins are detected with high frequency. The fact that antibodies against HCV envelope proteins are detected in only 10% of the patients is deduced to result from the presence of a mechanism that may complicate development of antibodies against HCV envelope proteins (Non-patent Document 3).

Meanwhile, it was demonstrated that HCV E2 proteins expressed in mammalian animal cells would specifically bind to CD81, which is present on the human cell surface (Non-patent Document 4). With the use of such experimentation system, development of an antibody exhibiting activity of neutralizing binding (i.e., neutralization of binding, referred to as "NOB") of E2 proteins and CD81 from hepatitis C patients has been attempted.

A representative example is an antibody exhibiting NOB activity that is obtained by preparing an antibody gene library from marrow lymphocytes of patients of chronic hepatitis C of genotype 1a and obtaining an antibody of interest via phage display (Patent Document 1). Another research group prepared hybridomas from peripheral B cells of patients of hepatitis C of genotype 1b and obtained an antibody exhibiting NOB activity (Non-patent Document 5, Patent Document 2).

According to the abovementioned method of obtaining monoclonal antibodies from HCV patients, however, the number of patients is limited, and the source is limited to patients in which antibodies inhibiting infection with HCV are present. Thus, it is difficult to increase the repertoires of antibodies inhibiting infection and to discover antibodies useful as anti-HCV agents.

Further, a method in which recombinant envelope proteins are administered to a mouse to induce antibodies (Patent Document 3) and a method in which lymphocytes are fused to myeloma cells to prepare antibody-producing hybridomas to obtain antibodies against envelope proteins (Patent Document 4, Non-patent Document 6) have been attempted. However, antibodies that inhibit HCV infection have not yet been demonstrated.

As a reason why an antibody that neutralizes HCV infection via immunization of an animal with an envelope protein has not yet been prepared, it is suggested that a recombinant envelope protein used for immunization has a structure different from that of the envelope protein inherent to the virus. It has already been reported that a recombinant envelope protein is likely to undergo aggregation and is not capable of forming a native conformation (Non-patent Document 7).

It has also been demonstrated that an antibody exhibiting NOB activity would not always inhibit infection (Non-patent Document 8). Thus, development of a method for effectively inducing an antibody against an envelope protein that is able to inhibit virus infection and an antibody that inhibits virus infection is necessary, from the viewpoint of treatment and prevention of HCV with the use of an antibody.

Under the above circumstances, a technique for preparing infectious HCV particles in a cell culture system has been developed recent years (Patent Document 5, Patent Document 6 and Patent Document 7). Compared with the case in which an envelope protein is expressed via a gene recombination technique and the resultant is used as an antigen, the HCV particles exhibit infectivity, and the conformation of the HCV antigen is thus considered to be maintained. Thus, such HCV particles can be suitable as antigens for producing antibodies for inhibiting HCV infection.
[Patent Document 1] JP Patent Publication (kohyo) No. 2005-531286 A
[Patent Document 2] JP Patent Publication (kohyo) No. 2006-504645 A
[Patent Document 3] JP Patent Publication (kohyo) No. 2004-500366 A
[Patent Document 4] JP Patent Publication (kohyo) No. H-06-505389 A
[Patent Document 5] WO 05080575 A1
[Patent Document 6] WO 06022422 A1
[Patent Document 7] WO 06096459 A2
[Non-patent Document 1] Choo et al., Science, 1989, vol. 244, pp. 359-362
[Non-patent Document 2] Matsuura et al., J. Virol., 1992, vol. 66, pp. 1425-1431
[Non-patent Document 3] Saito et al., Jikken Igaku, 1991, vol. 9, pp. 2075-2080
[Non-patent Document 4] Pileri et al., Science, 1998, vol. 282, pp. 938-941
[Non-patent Document 5] Hadlock et al., J. Virol., 2000, vol. 74, pp. 10407-10416
[Non-patent Document 6] Suzuki et al., Saishin Igaku, 2003, vol. 58, pp. 2017-2022
[Non-patent Document 7] Op de Beeck et al., J. Gen. Virol., 2001, vol. 82, pp. 2589-2595
[Non-patent Document 8] Burioni et al., J. Virol., 2002, vol. 76, pp. 11775-11779

### Disclosure of the Invention

### Problem to Be Solved by the Invention

The object of the present invention is to provide an antibody that inhibits infection with hepatitis C virus (HCV).

### Means for Solving the Problem

The present inventors administered hepatitis C virus (HCV) particles of the JFH-1 strain or a chimera virus of the J6CF strain and the JFH-1 strain as an antigen to mice and measured inhibitory activity on infection with HCV in the blood serum of the mice to which HCV had been administered. As a result, they discovered that such HCV particles had inhibitory activity on infection with HCV.

Further, spleen cells were prepared from mice to which HCV particles had been administered, the spleen cells were fused to mouse myeloma cell lines to prepare antibody-producing hybridomas, inhibitory activity on infection with HCV of antibodies produced by the hybridomas was evaluated, and monoclonal antibodies inhibiting HCV infection were obtained. This has led to the completion of the present invention. Specifically, the present invention includes the following.
[1] An antibody that recognizes a hepatitis C virus (HCV) particle obtained from the genome of a hepatitis C virus (HCV) comprising the following (i) and (ii) ligated to each other as an antigen and has inhibitory activity on infection with hepatitis C virus (HCV):
   (i) (a) the 5' untranslated region, the core protein-encoding sequence, the E1 protein-encoding sequence, the E2 protein-encoding sequence, and the p7 protein-encoding sequence of the JFH-1 strain of the hepatitis C virus (HCV) or (b) the 5' untranslated region, the core protein-encoding sequence, the E1 protein-encoding sequence, the E2 protein-encoding sequence, and the p7 protein-encoding sequence of the J6CF strain of the hepatitis C virus (HCV); and
   (ii) the NS2 protein-encoding sequence, the NS3 protein-encoding sequence, the NS4A protein-encoding sequence, the NS4B protein-encoding sequence, the NS5A protein-encoding sequence, the NS5B protein-encoding sequence, and the 3' untranslated region of the JFH-1 strain.
[2] The antibody according to [1] above, wherein the hepatitis C virus (HCV) genome is a nucleic acid comprising the nucleotide sequence as shown in SEQ ID NO: 1 or 2 of the Sequence Listing (provided that nucleotide "T" in the nucleotide sequence is read as "U" when the nucleic acid is RNA).
[3] The antibody according to [1] or [2] above, wherein the antibody class is IgM.
[4] The antibody according to any of [1] to [3] above, which recognizes at least 10 continuous amino acids in the amino acid sequence as shown in SEQ ID NO: 24 of the Sequence Listing.
[5] The antibody according to [4] above, which has a heavy chain variable region containing the amino acid sequence as shown in SEQ ID NO: 52 of the Sequence Listing.
[6] The antibody according to [4] or [5] above, which has a light chain variable region containing the amino acid sequence as shown in SEQ ID NO: 54 of the Sequence Listing.
[7] The antibody according to any of [4] to [6] above, which is produced by the hybridoma cell line of Accession Number FERM BP-10982.
[8] The antibody according to any of [1] to [3] above, which recognizes at least 10 continuous amino acids in the amino acid sequence as shown in SEQ ID NO: 44 or 45 of the Sequence Listing.
[9] The antibody according to [8] above, which is produced by the hybridoma cell line of Accession Number FERM BP-10980.
[10] The antibody according to any of [1] to [3] above, which recognizes at least 10 continuous amino acids in the amino acid sequence as shown in SEQ ID NO: 36, 45, 46, 47, or 48 of the Sequence Listing.
[11] The antibody according to [10] above, which is produced by the hybridoma cell line of Accession Number FERM BP-10981.
[12] An inhibitory agent for infection with hepatitis C virus (HCV) comprising, as an active ingredient, the antibody according to any of [1] to [11] above.
[13] A medicament comprising, as an active ingredient, the antibody according to any of [1] to [11] above.
[14] A therapeutic or preventive agent for hepatitis C comprising, as an active ingredient, the antibody according to any of [1] to [11] above.
[15] A method for detecting hepatitis C virus (HCV) comprising detecting hepatitis C virus (HCV) particles using the antibody according to any of [1] to [11] above.

### Effects of the Invention

The antibody having inhibitory activity on infection with HCV of the present invention and the use thereof can be used for treatment or prevention of hepatitis C and research for elucidation of the mechanism of HCV infection.

### Brief Description of the Drawings

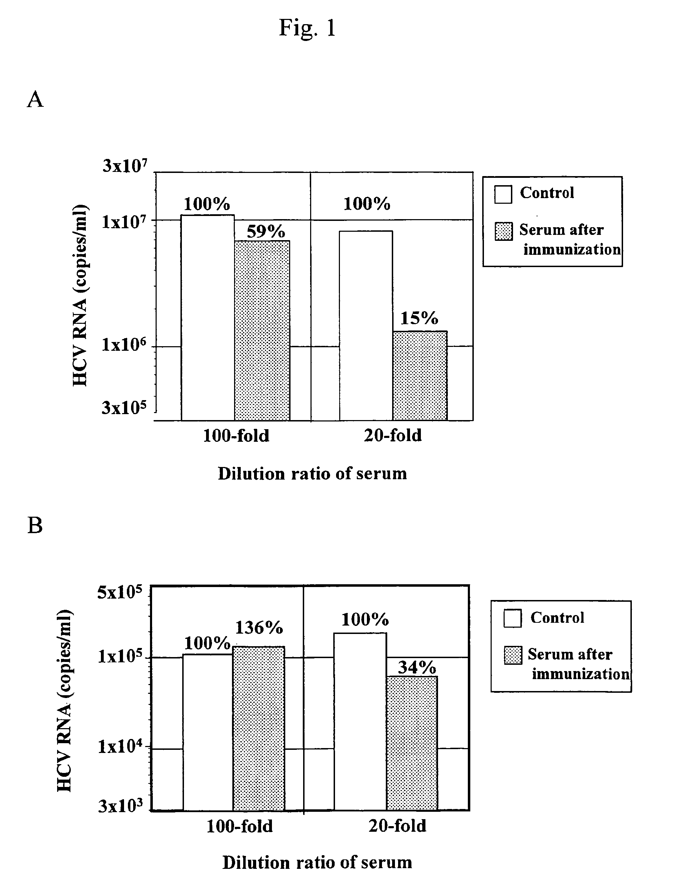

Fig. 1A shows activity of the blood serum of a mouse to which J6/JFH-1-HCV particles had been administered for inhibiting infection with HCV of genotype 2a. Fig. 1B shows activity of the blood serum of a mouse to which J6/JFH-1-HCV particles had been administered for inhibiting infection with HCV of genotype 1b.

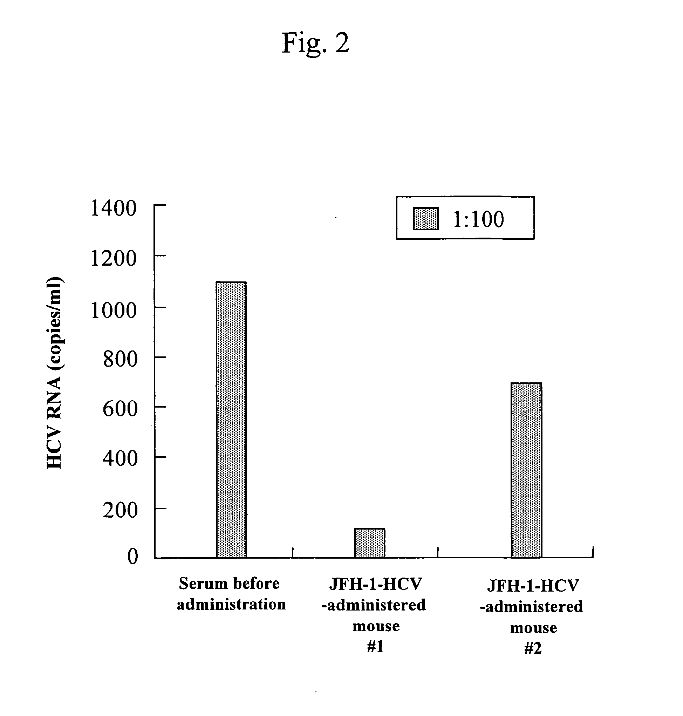

Fig. 2 shows activity of the blood serum of a mouse to which JFH-1-HCV particles had been administered for inhibiting infection with HCV of genotype 2a.

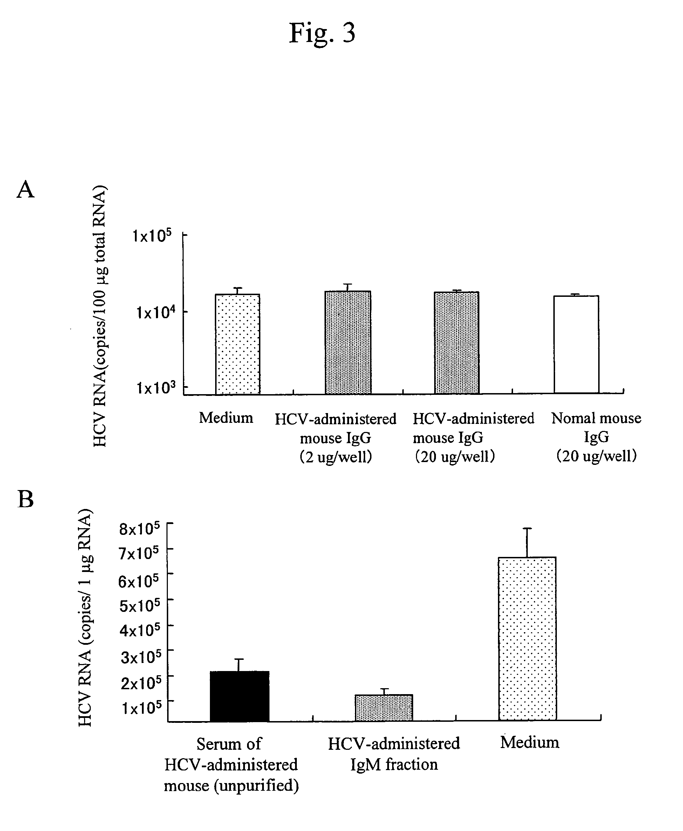

Fig. 3A shows activity of the blood serum IgG fraction of a mouse to which J6/JFH-1-HCV particles had been administered for inhibiting infection with HCV. Fig. 3B shows activity of the blood serum IgM fraction of a mouse to which J6/JFH-1-HCV particles had been administered for inhibiting infection with HCV.

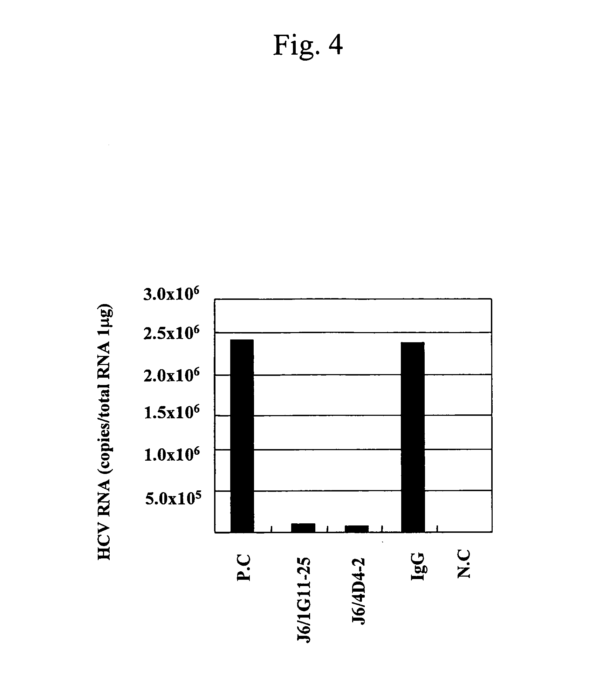

Fig. 4 shows inhibitory activity of a monoclonal antibody against J6/JFH-1-HCV upon infection with HCV. In the drawing, "P.C" represents the results of a positive control to which no antibody had been added; "N.C" represents the results of a negative control to which no infectious HCV particles had been added; and "IgG" represents the results of a control antibody.

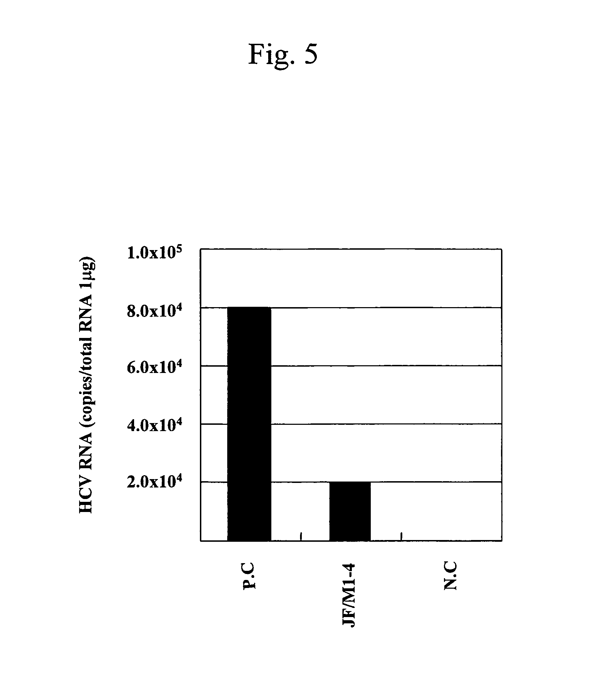

Fig. 5 shows inhibitory activity of a monoclonal antibody against JFH-1-HCV upon infection with HCV. In the drawings, "P.C" represents the results of a positive control to which no antibody had been added; and "N.C" represents the results of a negative control to which no infectious HCV particles had been added.

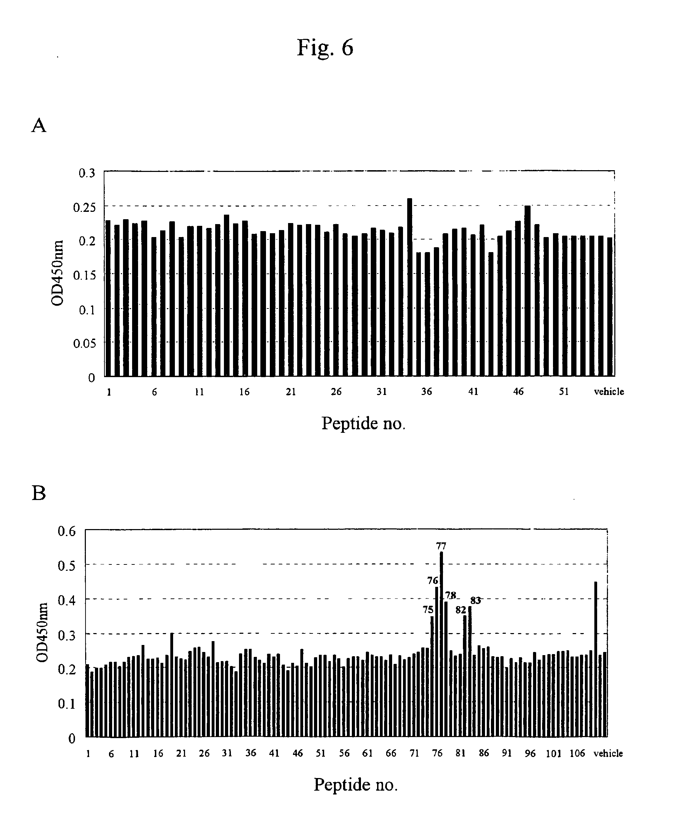

Fig. 6A shows binding intensity of the JF/M1-4 monoclonal antibody to a peptide having a sequence derived from JFH-1 E1 (envelope protein 1). Fig. 6B shows binding intensity of the JF/M1-4 monoclonal antibody to a peptide having a sequence derived from JFH-1 E2 (envelope protein 2). Bar charts (vehicles) representing peptide nos. 53 to 56 in Fig. 6A and peptide nos. 111 and 112 in Fig. 6B show control experiments that are carried out with the addition of PBS instead of peptides.

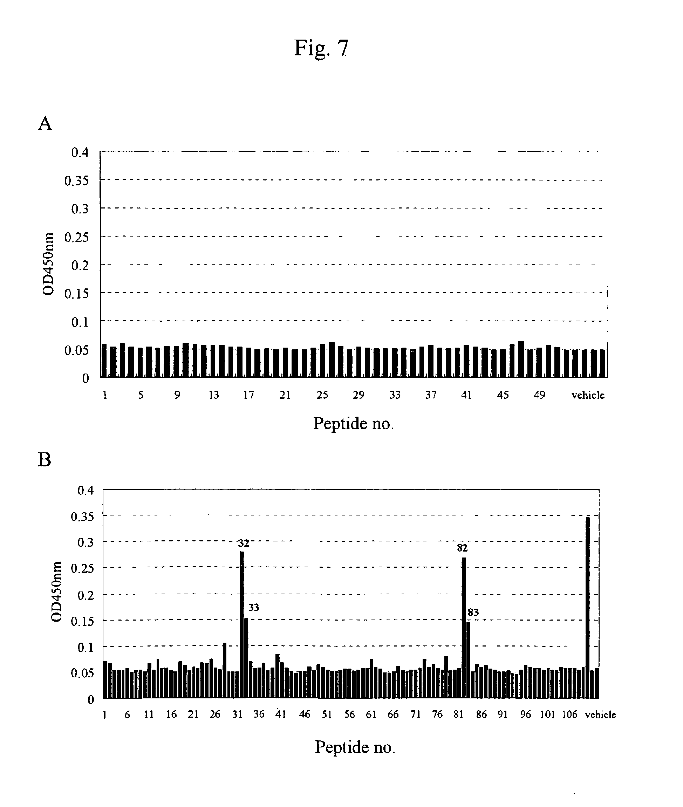

Fig. 7A shows binding intensity of the J6/1G11-25 monoclonal antibody when binding to a peptide having a sequence derived from J6 E1 (envelope protein 1). Fig. 7B shows binding intensity of the J6/1G11-25 monoclonal antibody when binding to a peptide having a sequence derived from J6 E2 (envelope protein 2). Bar charts (vehicles) representing peptide nos. 53 to 56 in Fig. 7A and peptide nos. 111 and 112 in Fig. 7B show control experiments that are carried out with the addition of PBS instead of peptides.

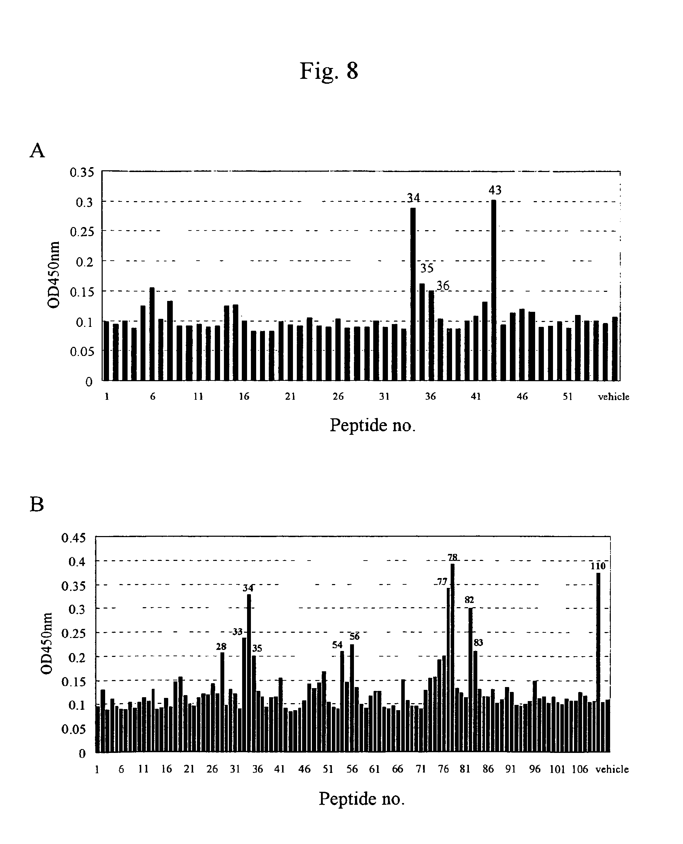

Fig. 8A shows binding intensity of the J6/4D4-2 monoclonal antibody when binding to a peptide having a sequence derived from J6 E1 (envelope protein 1). Fig. 8B shows binding intensity of the J6/4D4-2 monoclonal antibody when binding to a peptide having a sequence derived from E2 (J6 envelope protein 2). Bar charts (vehicles) representing peptide nos. 53 to 56 in Fig. 8A and peptide nos. 111 and 112 in Fig. 8B show control experiments that are carried out with the addition of PBS instead of peptides.

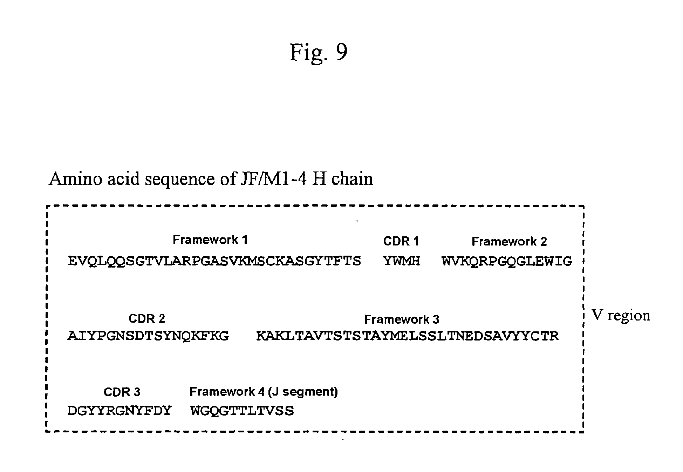

Fig. 9 shows the structures of the complementary determining regions (CDRs) and the framework regions in the H chain variable region of the JF/M1-4 monoclonal antibody and amino acid sequences thereof. This H chain V region comprises, sequentially from the N terminus to the C terminus, framework 1 (SEQ ID NO: 55), CDR1 (SEQ ID NO: 56), framework 2 (SEQ ID NO: 57), CDR2 (SEQ ID NO: 58), framework 3 (SEQ ID NO: 59), CDR3 (SEQ ID NO: 60), and framework 4 (also referred to as the J segment, J region, or J chain) (SEQ ID NO: 61) regions.

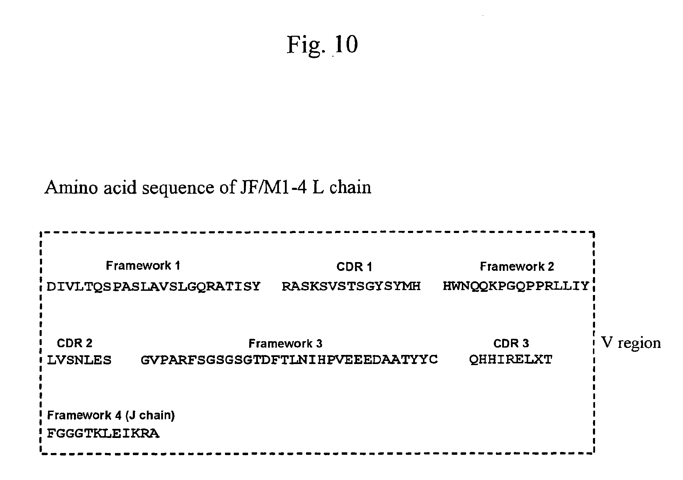

Fig. 10 shows the structures of the complementary determining regions (CDRs) and the framework regions in the L chain variable region of the JF/M1-4 monoclonal antibody and amino acid sequences thereof. This L chain V region comprises, sequentially from the N terminus to the C terminus, framework 1 (SEQ ID NO: 62), CDR1 (SEQ ID NO: 63), framework 2 (SEQ ID NO: 64), CDR2 (SEQ ID NO: 65), framework 3 (SEQ ID NO: 66), CDR3 (SEQ ID NO: 67), and framework 4 (also referred to as the J segment, J region, or J chain) (SEQ ID NO: 68) regions.

### Best Modes for Carrying out the Invention

The present invention relates to an antibody that can be induced with the use of an infectious HCV particle produced from the specific HCV genome as the antigen and that can inhibit HCV infection.

The present invention can be implemented via conventional molecular biological and immunological techniques within the technical scope in the art. Such techniques are thoroughly described in, for example, Sambrook et al., Molecular Cloning: A Laboratory Manual, Cold Spring Harbor Laboratory (vol. 3, 2001) or Ed Harlow et al., Antibodies: A Laboratory Manual, Cold Spring Harbor Laboratory, 1988.

All publications, patents, and patent applications cited herein are incorporated herein by reference in their entirety.

This description includes part or all of the contents as disclosed in Japanese Patent Application No. 2007-193413, to which the present application claims priority.

### (1) Preparation of infectious HCV particles

Infectious HCV particles that can be used as antigens in the present invention can be produced in a cell culture system. Fundamental techniques for doing so are described in WO 04104198 A1, WO 06022422 A1, WO 06096459 A2, Wakita, T. et al., Nat. Med. 11: 791-796, 2005, Lindenbach, B.D. et al., Science 309 :623-626, 2005, and Pietschmann, T. et al., Proc. Natl. Acad. Sci. U.S.A., 103: 7408-7413, 2006.

A specific example of the HCV genome that can be used for generating infectious HCV particles used for producing the antibody having inhibitory activity on infection with HCV of the present invention is virus genome RNA of the JFH-1 strain of genotype 2a, which comprises, sequentially from the 5' end to the 3' end, the 5' untranslated region, the core protein-encoding sequence, the E1 protein-encoding sequence, the E2 protein-encoding sequence, the p7 protein-encoding sequence, the NS2 protein-encoding sequence, the NS3 protein-encoding sequence, the NS4A protein-encoding sequence, the NS4B protein-encoding sequence, the NS5A protein-encoding sequence, the NS5B protein-encoding sequence, and the 3' untranslated region. Alternatively, such HCV genome can be composed of virus genome RNAs of two or more types of HCV strains, comprising, sequentially from the 5' end to the 3' end, the 5' untranslated region, the core protein-encoding sequence, the E1 protein-encoding sequence, the E2 protein-encoding sequence, the p7 protein-encoding sequence, and NS2 protein-encoding sequence of an HCV strain other than the JFH-1 strain and the NS3 protein-encoding sequence, the NS4A protein-encoding sequence, the NS4B protein-encoding sequence, the NS5A protein-encoding sequence, the NS5B protein-encoding sequence, and the 3' untranslated region of the JFH-1 strain. Further, the HCV genome can be composed of virus genome RNAs of two or more types of HCV strains, comprising, sequentially from the 5' end to the 3' end, the 5' untranslated region, the core protein-encoding sequence, the E1 protein-encoding sequence, the E2 protein-encoding sequence, and the p7 protein-encoding sequence of an HCV strain other than the JFH-1 strain and the NS2 protein-encoding sequence, the NS3 protein-encoding sequence, the NS4A protein-encoding sequence, the NS4B protein-encoding sequence, the NS5A protein-encoding sequence, the NS5B protein-encoding sequence, and the 3' untranslated region of the JFH-1 strain.

According to a preferable embodiment of the present invention, the HCV genome is a chimeric genome comprising, sequentially from the 5' end to the 3' end, the full-length genome derived from the JFH-1 strain of genotype 2a (e.g., the genome comprising the nucleotide sequence as shown in SEQ ID NO: 1) and the 5' untranslated region, the core protein-encoding region, the E1 protein-encoding region, the E2 protein-encoding region, the p7 protein-encoding region, a region up to amino acid 16 at the N-terminus of the NS2 protein-encoding region, a region from amino acid 17 at the N terminus to the C terminus of the NS2 protein-encoding region of the J6CF strain, the NS3 protein-encoding region, the NS4A protein-encoding region, the NS4B protein-encoding region, the NS5A protein-encoding region, the NS5B protein-encoding region, and the 3' untranslated region derived from the JFH-1 strain of genotype 2a. A particularly preferable example thereof is a nucleic acid cloned into J6/JFH-1 comprising the nucleotide sequence as shown in SEQ ID NO: 2.

According to a particularly preferable embodiment of the present invention, the HCV genome is a nucleic acid comprising the nucleotide sequence as shown in SEQ ID NO: 1 or 2, provided that nucleotide "T" in the nucleotide sequence is read as "U" when the nucleic acid is RNA. The infectious HCV particles of the present invention can be produced with the use of HCV genomic RNA or HCV genomic DNA.

Specifically, the present invention relates to an antibody having activity of inhibiting infection with HCV, which reacts with, as an antigen, the HCV particle obtained from (a) the HCV genome comprising, sequentially from the 5' end to the 3' end, the 5' untranslated region, the core protein-encoding sequence, the E1 protein-encoding sequence, the E2 protein-encoding sequence, and the p7 protein-encoding sequence of the JFH-1 strain, the NS2 protein-encoding sequence, the NS3 protein-encoding sequence, the NS4A protein-encoding sequence, the NS4B protein-encoding sequence; the NS5A protein-encoding sequence, the NS5B protein-encoding sequence, and the 3' untranslated region of the JFH-1 strain or (b) the HCV genome comprising, sequentially from the 5' end to the 3' end, the 5' untranslated region, the core protein-encoding sequence, the E1 protein-encoding sequence, the E2 protein-encoding sequence, and the p7 protein-encoding sequence of the J6CF strain of HCV and the NS2 protein-encoding sequence, the NS3 protein-encoding sequence, the NS4A protein-encoding sequence, the NS4B protein-encoding sequence, the NS5A protein-encoding sequence, the NS5B protein-encoding sequence, and the 3' untranslated region of the JFH-1 strain (preferably the HCV genome comprising, sequentially from the 5' end to the 3' end, the 5' untranslated region, the core protein-encoding sequence, the E1 protein-encoding sequence, the E2 protein-encoding sequence, the p7 protein-encoding sequence, and a sequence encoding a region up to amino acid 16 at the N terminus of the NS2 protein-encoding region of the J6CF strain and a sequence encoding a region from amino acid 17 at the N terminus to the C terminus of the NS2 protein-encoding region, the NS3 protein-encoding sequence, the NS4A protein-encoding sequence, the NS4B protein-encoding sequence, the NS5A protein-encoding sequence, the NS5B protein-encoding sequence, and the 3' untranslated region of the JFH-1 strain).

When activity of the antibody of the present invention for inhibiting infection is evaluated, in addition to the HCV particles (a) or (b) above, HCV particles obtained from the HCV genome (c) comprising the 5' untranslated region of the JFH-1 strain, the core protein-encoding sequence, the E1 protein-encoding sequence, the E2 protein-encoding sequence, and the p7 protein-encoding sequence of the TH strain of genotype 1b (Wakita, T. et al., J. Biol. Chem., 269, 14205-14210, 1994, JP Patent Publication (kokai) No. 2004-179 A), the NS2 protein-encoding sequence, the NS3 protein-encoding sequence, the NS4A protein-encoding sequence, the NS4B protein-encoding sequence, the NS5A protein-encoding sequence, the NS5B protein-encoding sequence, and the 3' untranslated region of the JFH-1 strain ligated in that order (preferably the HCV genome comprising the 5' untranslated region derived from the JFH-1 strain, the core protein-encoding sequence, the E1 protein-encoding sequence, the E2 protein-encoding sequence, the p7 protein-encoding sequence, and a sequence encoding a region up to amino acid 33 at the N terminus of the NS2 protein-encoding region of the TH strain, a sequence encoding a region from amino acid 34 at the N terminus to the C terminus of NS2 protein-encoding region, the NS3 protein-encoding sequence, the NS4A protein-encoding sequence, the NS4B protein-encoding sequence, the NS5A protein-encoding sequence, the NS5B protein-encoding sequence, and the 3' untranslated region of the JFH-1 strain ligated in that order) can be used.

The above infectious HCV particles can be prepared by synthesizing RNA from a vector comprising cDNA of full-length HCV genomic RNA according to any of (a) to (c) above cloned into a site downstream of the transcription promoter (e.g., a vector comprising HCV genomic RNA cloned under the control of the T7 promoter) and introducing the RNA into cells. RNA can be synthesized *in vitro* with the use of a nucleic acid comprising HCV cDNA cloned under the control of the T7 promoter as a template using a kit, such as the MEGAscript T7 kit (Ambion). RNA may be introduced into any cells, provided that such cells permit generation of HCV particles. Examples thereof include cultured cells of Huh7 cells, HepG2 cells, IMY-N9 cells, HeLa cells, and 293 cells. A more preferable example is liver-derived cultured cells such as Huh7 cells. Further preferable examples include Huh7 cells and cells derived from Huh7 cells (i.e., Huh7.5 cells and Huh7.5.1 cells). Further, cells obtained by expressing CD81 and/or Claudin1 genes in Huh7 cells, HepG2 cells, IMY-N9 cells, HeLa cells, or 293 cells can be used. Huh7 cells or derivative strain cells from Huh7 cells are particularly preferable. In the present invention, the term "derivative strain" refers to cell lines derived from the relevant cells.

RNA can be introduced into cells by any known methods. Examples of such methods include calcium phosphate coprecipitation, the DEAE-dextran method, lipofection, microinjection, and electroporation. Lipofection and electroporation are preferable and electroporation is more preferable.

The capacity of the cells for virus particle production can be evaluated with the use of an antibody reacting with a factor that constitutes HCV particles released in the culture solution, such as the core protein, the E1 protein, or the E2 protein. Also, HCV genomic RNA contained in the HCV particles in the culture solution may be amplified via RT-PCR using specific primers to indirectly detect the presence of HCV particles.

Whether or not the prepared viruses are infectious can be evaluated by culturing cells into which HCV RNA has been introduced in the aforementioned manner, applying (adding) the resulting supernatant to HCV permissive cells (e.g., Huh7 cells), and immunologically staining the cells with an anti-core antibody 48 hours later to count the number of infected cells. Alternatively, evaluation can be carried out by subjecting the cell extract to electrophoresis on SDS-polyacrylamide gel and detecting core proteins via Western blotting.

### (2) Purification of infectious HCV particles

The virus solution containing the infectious HCV particles obtained in (1) above is subjected to, for example, centrifugation and/or filtration through a filter to remove cells and cell residues. A solution from which residues have been removed can be concentrated approximately 10- to 100-fold using a ultrafiltration membrane with a molecular weight cut off of 100,000 to 500,000. A solution containing HCV from which residues have been removed can be purified via chromatography and density-gradient centrifugation in arbitrary combinations or alone. Hereafter, representative chromatography or density-gradient centrifugation techniques are described, although the techniques are not limited thereto.

Gel filtration chromatography can be carried out preferably using a chromatography support comprising a cross-linked polymer of allyl dextran and N,N'-methylenebisacrylamide as the gel matrix, and more preferably by using Sephacryl^{®} S-300, S-400, and S-500 chromatography to purify infectious HCV particles.

Ion-exchange chromatography can be carried out using, preferably, Q-Sepharose^{®} as an anion exchange resin and SP Sepharose^{®} as a cation exchange resin to purify HCV particles.

Affinity chromatography can be carried out preferably using, as a support, resin comprising a substrate selected from among heparin, sulfated cellulofine, lectin, and various dyes bound thereto as a ligand to purify HCV particles. Further preferably, HCV particles can be purified with the use of a support comprising HiTrap Heparin HP^{®}, HiTrap Blue HP^{®}, HiTrap Benzamidine FF^{®}, sulfated cellulofine, LCA, ConA, RCA-120, and WGA bound thereto. The most preferable method is purification of HCV particles with the use of sulfated cellulofine as a support. HCV particles can be purified 30-fold or more in terms of the ratio of the HCV RNA copy number to the total protein amount in the solution.

Purification via density-gradient centrifugation can be preferably carried out with the use of a sugar polymer, such as cesium chloride, sucrose, Nycodenz^{®}, Ficoll^{®}, or Percoll^{®}, as a solute that makes density gradient. Sucrose can be further preferably used. Preferably, water or a buffer, such as phosphate, Tris, acetate, or glycine buffer, can be used. The centrifugal force that is employed at the time of purification via density-gradient centrifugation is preferably 1 x 10⁴ to 1 x 10⁹ g, more preferably 5 x 10⁴ to 1 x 10⁷ g, and most preferably 5 x 10⁴ to 5 x 10⁵ g.

Purification is carried out preferably at 0°C to 40°C, more preferably 0°C to 25°C, and most preferably 0°C to 10°C.

When purification is carried out via density-gradient centrifugation in combination with column chromatography, such techniques may be carried out in any order. Preferably, HCV particles are first purified through a plurality of chromatography columns followed by density-gradient centrifugation. More preferably, a fraction containing HCV particles obtained via anion exchange column chromatography followed by affinity chromatography is subjected to purification via density-gradient centrifugation. Most preferably, a fraction containing HCV particles obtained with the use of a Q-Sepharose^{®} column is further purified with the use of a sulfated cellulofine-based column, and the resulting fraction containing HCV particles is then purified via density-gradient centrifugation. During the steps of column chromatography and density-gradient centrifugation, dialysis or ultrafiltration may be carried out to substitute a solute of a solution containing HCV particles and/or to concentrate HCV particles.

### (3) Inactivation of infectious HCV particles

The antibody of the present invention reacts with the HCV particle as an antigen. When producing the antibody of the present invention, infectivity of the HCV particles is not correlated with antigenicity, although use of inactivated HCV particles is preferable. Infectious HCV particles can be inactivated by adding and mixing an inactivator such as formalin, β-propiolactone, or glutardialdehyde in, for example, a virus suspension and allowing the inactivator to react with viruses (Appaiahgari et al., Vaccine, 22: 3669-3675, 2004). Further, infectious HCV particles may be irradiated with ultraviolet rays to eliminate infectivity of viruses, and viruses can be immediately inactivated. Irradiation with ultraviolet rays realizes virus inactivation with little influence on proteins that constitute viruses. A source of ultraviolet rays used for inactivation can be a commercially available germicidal lamp. In particular, a 15w germicidal lamp can be used, although the source is not limited thereto. In the present invention, HCV particles that are purified by the method described above are used, and methods of inactivation are not limited by a purified or unpurified state. Preferably, a solution containing infectious HCV particles may be irradiated with ultraviolet rays at 20 mW/cm² at room temperature for at least 5 minutes to inactivate infectious HCV particles.

### (4) Immunization of animals

The antibody of the present invention can be obtained by administering HCV particles to animals to induce the antibody via immune reaction. Any non-human animals (non-human mammalian animals), such as mice, rats, hamsters, or rabbits, which can produce spleen cells capable of producing hybridomas, may be used for immunization. In the present invention, mice are preferable, and examples involving the use of mice are described below.

In general, 4- to 10-week-old mice may be immunized with the HCV particle antigen obtained in the steps (1) to (3) above. According to circumstances, the step of purification may be altered or omitted, and virus inactivation may be omitted. In general, immunization is carried out by hypodermically or intraperitoneally administering an antigen with an adjuvant several times. Examples of adjuvants include, but are not limited to, the Freund's complete adjuvants, the Freund's incomplete adjuvant, aluminum hydroxide gel with pertussis vaccine, Titer Max Gold (Vaxel), and the GERBU adjuvant (GERBU Biotechnik). Final immunization is carried out without the use of an adjuvant, the antigen is intraveously or intraperitoneally administered, and polyclonal antibodies are obtained from the blood serum of the immunized mice 3 to 10 days, and preferably 4 days, after the final administration of HCV particles in accordance with a known method (Antibodies: A Laboratory Manual, Cold Spring Harbor Laboratory, 1988). Whether or not the immunized animal produces the antibody of the present invention can be inspected by sampling the blood from the venous plexus of the ocular fundus or caudal vein of the immunized animal before the final immunization and measuring the activity of inhibiting infection with HCV used as the antigen.

The antibody according to the present invention may be a monoclonal or polyclonal antibody, and preferably, a monoclonal antibody. Also, the antibody of the present invention may be derived from any organism, preferably a mammalian animal, and more preferably a human. The antibody of the present invention may be a chimeric antibody (e.g., a chimeric antibody of a human antibody and an antibody derived from another mammalian animal). An example of particularly preferable chimeric antibody is a humanized antibody obtained by transplanting a sequence at the hypervariable region of a mouse antibody into a human antibody. The antibody of the present invention may be a chimeric antibody having a framework region of a human antibody and a heavy chain variable region and/or a light chain variable region including a hypervariable region of an antibody derived from another mammalian animal.

### (5) Preparation of hybridoma cell that produces monoclonal antibody

When the antibody of the present invention, and in particular, when the monoclonal antibody, is prepared, the spleen is removed from the immunized animal, and the spleen cells are fused to the myeloma cells to prepare hybridoma cells. Any myeloma cells that can be multiplied *in vitro* can be used for cell fusion. Examples thereof include mouse-derived established cells, such as 8-azaguanine-resistant mouse (BALB/c-derived) myeloma cell lines P3-X63Ag8-U1 (P3-U1), SP2/0-Ag14 (SP2/0), P3-X63-Ag8653 (653), P3-X63-Ag 8(X63), and P3/NS1/1-Ag4-1 (NS1). These cell lines are available from RIKEN BioResource Center, ATCC (American Type Culture Collection), or ECACC (European Collection of Cell Cultures). Culture and subculture are carried out in accordance with a conventional technique (Antibodies: A Laboratory Manual, Cold Spring Harbor Laboratory, 1988, Selected Methods in Cellular Immunology W.H. Freeman and Company, 1980).

The spleen cells and the myeloma cells obtained above are washed, the myeloma cells are mixed with the spleen cells at a proportion of 1:1 to 10, and polyethylene glycol is added thereto for cell fusion. Polyethylene glycol is capable of fusing cells, polyethylene glycol with less cytotoxicity is preferable, and, for example, polyethylene glycol-1500 (PEG-1500) can be preferably used. After cell fusion, cells are suspended and washed in a medium. Cells are washed using a medium that is used for myeloma cell culture, such as Dulbecco's modified MEN medium or RPMI-1640. Culture medium for fused cells is selected so as to selectively obtain fused cells of interest, and an example of such medium is a medium prepared by adding hypoxanthine (10⁻⁴ M), thymidine (1.5 x 10⁻⁵ M), and aminopterin (4 x 10⁻⁷ M) to HAT medium (a medium prepared by adding 2-mercaptoethanol (5 x 10⁻⁵M), penicillin (100 units/ml), streptomycin (100 µg/ml), and fetal calf serum (FCS)(10%, Invitrogen) to a medium used for myeloma cell culture, such as Dulbecco's modified MEN medium).

After culture, part of the culture supernatant is separated, and cells producing antibodies that inhibit infection with HCV are selected via an HCV infection test. Alternatively, cells producing antibodies that react with HCV proteins can be selected via enzyme immunoassay. Subsequently, cloning is carried out via limiting dilution or colony formation in a methylcellulose medium, and cells that have been proven to produce antibodies reacting with HCV proteins and antibodies having inhibitory activity on infection with HCV are selected as a monoclonal antibody-producing hybridoma cell line.

### (6) Selection of anti-HCV monoclonal antibody having inhibitory activity on infection with HCV

Hybridomas that produce anti-HCV monoclonal antibodies having inhibitory activity on infection with HCV can be selected via a method for assaying inhibitory activity on infection with HCV using infectious HCV particles as follows or described in the Examples below.

At the outset, infectious HCV particles (the method for producing the same was described above) are mixed with the antibody sample, and the reaction is allowed to proceed at 37°C for 1 hour. The sample (50 µl) is added to the Huh7 cells, which had been cultured on the previous day at 5 x 10³ cells/well on a 96-well plate, and culture is conducted at 37°C for 2.5 hours. After culture, the sample is removed, cells are washed with PBS, a fresh medium is added, and culture is continued. The culture supernatant is removed 48 hours later, cells are washed once with PBS, 100 µl of ISOGEN (Nippon Gene) is added, RNA is prepared from the cells, RNA is quantified, and the amount of HCV genomic RNA is then measured. HCV RNA is detected via quantitative RT-PCR by detecting RNA in the 5' end untranslated region of HCV RNA according to the method of Takeuchi et al. (Takeuchi T. et al., Gastroenterology, 116: 636-642, 1999).

Alternatively, inhibitory activity on infection with HCV can be evaluated by the following method. At the outset, the antibody sample is mixed with infectious HCV particles, and the resulting mixture is subjected to a reaction at 37°C for 1 hour. Subsequently, the sample (50 µl) is added to the Huh7 cells, which had been cultured on the previous day at 1x10⁴ cells/well on a 96-well plate, and culture is conducted at 37°C for 2.5 hours. After culture, the sample is removed, cells are washed with PBS, a fresh medium is added, and culture is continued. The culture supernatant is removed 72 hours later, the plate is introduced into ice-cooled methanol, and cells are fixed. Thereafter, methanol is removed via air drying, and cells are permeabilized with the use of Block Ace^{®} (Dainippon Pharmaceutical Co., Ltd.) containing 0.3% Triton^{®}-X 100 (GE Healthcare). The number of HCV-infected cells is counted under a fluorescent microscope (IX-70; Olympus) using a clone 2H9 anti-HCV-core antibody (Nat Med., 2005, 11: pp. 791-6) and goat anti-mouse IgG-Alexa488 (Molecular Probes), and the samples in the wells in which HCV infection is inhibited are designated as positive clones. Thus, target hybridomas can be selected. A monoclonal antibody produced by the hybridoma thus selected is an antibody according to a preferable embodiment of the present invention.

Hybridomas that produce the antibodies of the present invention are not particularly limited, provided that such hybridomas are selected in the above manner. Hybridoma cell lines of Accession Number FERM BP-10980, Accession Number FERM BP-10981, and Accession Number FERM BP-10982 are preferable. Hybridoma cell lines J6/1G11-25 (Accession Number: FERM BP-10980; transferred from Accession Number: FERM P-21318 deposited on July 19, 2007 (Receipt Number: FERM AP-21318) to international deposit), J6/4D4-2 (Accession Number: FERM BP-10981; transferred from Accession Number: FERM P-21319 deposited on July 19, 2007 (Receipt Number: FERM AP-21319) to international deposit), and JF/M1-4 (Accession Number: FERM BP-10982; transferred from Accession Number: FERM P-21320 deposited on July 19, 2007 (Receipt Number: FERM AP-21320) to international deposit) were deposited at the International Patent Organism Depositary of the National Institute of Advanced Industrial Science and Technology (Central 6, 1-1-1 Higashi, Tsukuba, Ibaraki, 305-8566, Japan) as of July 19, 2007. These hybridoma cell lines can be preferably cultured in a medium prepared by adding 1 mM sodium pyruvate, 55 µM 2-mercaptoethanol, and 10% fetal calf serum to Dulbecco's modified Eagle medium (high glucose) at 37°C.

In order to analyze an epitope of the antibody of the present invention produced by the hybridoma cell line selected in the above-described manner, HCV protein-based enzyme immunoassay (EIA), Western blotting, dot blotting, or other means can be employed. By performing such epitope analysis for the primary screening of the anti-HCV monoclonal antibody, the anti-HCV monoclonal antibody that targets a given HCV protein can be effectively screened.

A more preferable antibody having inhibitory activity on infection with HCV according to the present invention recognizes the amino acid sequence WLTPKCLVHYPYRLWHYPC (SEQ ID NO: 24) or an amino acid sequence comprising at least 10 continuous amino acids of WLTPKCLVHYPYRLWHYPC (SEQ ID NO: 24) of the E2 protein of HCV (and in particular, the JFH-1 strain of HCV) as an epitope. An amino acid sequence comprising at least 10 continuous amino acids in WLTPKCLVHYPYRLWHYPC is preferably LVHYPYRLWH (SEQ ID NO: 18), WLTPKCLVHY (SEQ ID NO: 19), PKCLVHYPYR (SEQ ID NO: 20), or YPYRLWHYPC (SEQ ID NO: 21), with LVHYPYRLWH (SEQ ID NO: 18) being more preferable. An antibody that recognizes NFTIFKIRMY (SEQ ID NO: 22) or IFKIRMYVCG (SEQ ID NO: 23), which is an amino acid sequence of the HCV E2 protein, is a more preferable antibody having inhibitory activity on infection with HCV according to the present invention. In the present description, for example, the term "amino acids 1 to 162 of the E1 protein" refers to a region from amino acids 1 to 162 in the amino acid sequence of the E1 protein. Also, a similar expression representing part of a protein or nucleic acid is to be similarly understood.

An example of the antibody according to a preferable embodiment of the present invention is an antibody having a heavy chain variable region containing the amino acid sequence as shown in SEQ ID NO: 52. Further, another example of the antibody according to a preferable embodiment of the present invention is an antibody having a light chain variable region containing the amino acid sequence as shown in SEQ ID NO: 54. An antibody having a heavy chain variable region containing the amino acid sequence as shown in SEQ ID NO: 52 and a light chain variable region containing the amino acid sequence as shown in SEQ ID NO: 54 (preferably a monoclonal antibody) is more preferable. An example of such monoclonal antibody is described as the JF/M1-4 monoclonal antibody in the Examples below. An antibody having a heavy chain variable region containing the amino acid sequence as shown in SEQ ID NO: 52 and/or a light chain variable region containing the amino acid sequence as shown in SEQ ID NO: 54 may be an antibody derived from any organism, preferably an antibody derived from a mammalian animal, and more preferably a human antibody or a chimeric antibody. A chimeric antibody obtained by transplanting a heavy chain and/or light chain variable region derived from an arbitrary organism (e.g., a mouse that can be easily genetically engineered) or a complementary determining region (a hypervariable region) thereof into an equivalent site of an antibody (preferably a monoclonal antibody) derived from an organism (e.g., a human) to which the antibody of interest is to be administered can be used. A particularly preferable example of a chimeric antibody is a humanized antibody obtained by transplanting a sequence of a hypervariable region (also referred to as the "complementary determining region (CDR)") of a mouse antibody into the relevant site of a human antibody.

The antibody of the present invention can comprise, as the complementary determining regions (CDRs) of the H chain V region (i.e., the heavy chain variable region), CDR1, CDR2, and CDR3 having the amino acid sequences as shown in SEQ ID NOs: 56, 58, and 60 derived from the JF/M1-4 monoclonal antibody, respectively. Also, the antibody of the present invention can comprise, as the complementary determining regions (CDRs) of the L chain V region (i.e., the light chain variable region), CDRs, CDR2, and CDR3 having the amino acid sequences as shown in SEQ ID NOs: 63, 65, and 67 derived from the JF/M1-4 monoclonal antibody, respectively. According to a further preferable embodiment, the antibody of the present invention can comprise, as the complementary determining regions (CDRs) of the H chain V region (i.e., the heavy chain variable region), CDR1, CDR2, and CDR3 having the amino acid sequences as shown in SEQ ID NOs: 56, 58, and 60, respectively, and, as the complementary determining regions (CDRs) of the L chain V region (i.e., the light chain variable region), CDR1, CDR2, and CDR3 having the amino acid sequences as shown in SEQ ID NOs: 63, 65, and 67, respectively. Such antibody can comprise any framework 1 to 4 regions in the heavy chain variable region and the light chain variable region. Such antibody can also comprise framework 1 to 4 regions of an any antibody class derived from an organism other than a mouse (e.g., human-derived framework 1 to 4 regions). Such antibodies having CDR1 to CDR3 of the H chain V region and/or CDR1 to CDR3 of the L chain V region derived from the JF/M1-4 monoclonal antibody show activities of inhibiting infection with HCV particles of genotype 2a.

According to another embodiment of the present invention, a more preferable antibody having inhibitory activity on infection with HCV recognizes an amino acid sequence comprising at least 10 continuous amino acid residues in the amino acid sequence NVTNPEDMPRPYCW (SEQ ID NO: 44) or NYTIFKIRMYVGG (SEQ ID NO: 45) of the E2 protein of HCV (and in particular, HCV of the J6CF strain) as an epitope. Examples of amino acid sequences comprising at least 10 continuous amino acids in the amino acid sequence NVTNPEDMPRPYCW (SEQ ID NO: 44) or NYTIFKIRMYVGG (SEQ ID NO: 45) include NVTNPEDMRP (SEQ ID NO: 29), NPEDMRPYCW (SEQ ID NO: 30), NYTIFKIRMY (SEQ ID NO: 31), and IFKIRMYVGG (SEQ ID NO: 32).

According to a further embodiment of the present invention, a more preferable antibody having inhibitory activity on infection with HCV recognizes an amino acid sequence comprising at least 10 continuous amino acids in the amino acid sequence FIVSPQHHWFVQDCNC (SEQ ID NO: 46) or MAWDMMMNWS (SEQ ID NO: 36) of the E1 protein of HCV (and in particular, HCV of the J6CF strain) or the amino acid sequence LIDYPYRLWHYPC (SEQ ID NO: 47), NPEDMRPYCWHYPPRQ (SEQ ID NO: 48), or NYTIFKIRMYVGG (SEQ ID NO: 45) of the E2 protein as the epitope. Examples of amino acid sequences each comprising at least 10 continuous amino acids of the amino acid sequences in FIVSPQHHWFVQDCNC (SEQ ID NO: 46), MAWDMMMNWS (SEQ ID NO: 36), LIDYPYRLWHYPC (SEQ ID NO: 47), NPEDMRPYCWHYPPRQ (SEQ ID NO: 48), or NYTIFKIRMYVGG (SEQ ID NO: 45) of the E2 protein include FIVSPQHHWF (SEQ ID NO: 33), SPQHHWFVQD (SEQ ID NO: 34), HHWFVQDCNC (SEQ ID NO: 35), and MAWDMMMNWS (SEQ ID NO: 36) of E1-derived epitopes. Examples of E2-derived epitopes include LIDYPYRLWH (SEQ ID NO: 37), YPYRLWHYPC (SEQ ID NO: 38), DMRPYCWHYP (SEQ ID NO: 39), NPEDMRPYCW (SEQ ID NO: 40), and PYCWHYPPRQ (SEQ ID NO: 41).

In order to select the antibody of the present invention, more specifically, HCV proteins are immobilized on a support (i.e., solid-phased), the antibody sample is then added thereto, and the reaction is then allowed to proceed for a certain period of time under conditions that are sufficient for forming an antibody/antigen complex. Subsequently, the thus-formed complex is brought into contact with an antibody (i.e., a secondary antibody) that recognizes the antibody sample to which a signal enzyme, dye, or radioisotope has been bound to form the second mixture. The second mixture is subjected to the reaction for a certain period of time under the conditions that are sufficient for forming an antibody/antigen complex. The presence of an antibody that recognizes the HCV protein is detected with the aid of a signal of an enzyme, dye, or radioisotope.

As HCV proteins to be immobilized on a support, HCV particles may be used. Alternatively, proteins expressed in E. Coli, yeast, mammalian animal cells, insect cells, or the like with the use of cDNA comprising the core protein-encoding sequence, the E1 protein-encoding sequence, the E2 protein-encoding sequence, the p7 protein-encoding sequence, the NS2 protein-encoding sequence, the NS3 protein-encoding sequence, the NS4A protein-encoding sequence, the NS4B protein-encoding sequence, the NS5A protein-encoding sequence, or the NS5B protein-encoding sequence of the HCV genome may be used. Further, such proteins may be chemically synthesized and used. The length of an amino acid sequence of a protein to be used for immobilization is not limited, and such length is 3 or amino acids and more preferably 8 amino acids.

Examples in which the E1 protein and the E2 protein, which are envelope proteins of the JFH-1 strain or the J6CF strain, are expressed in mammalian animal cells are described below. The E1 protein of the JFH-1 strain or the J6CF strain starts from amino acid 192 and ends at amino acid 383, when amino acid 1 starts at the initiator methionine of the amino acid sequence of the full-length JFH-1 protein (NCBI Protein Accession Number BAB32872). A region from amino acid 353 to amino acid 383 of the E1 protein is considered to be a transmembrane domain (also referred to as a "C-terminal hydrophobic domain") (Cocquerel, L. et al., J. Virol. 74 :3623-3633, 2000).

The E2 protein of the JFH-1 strain or the J6CF strain starts from amino acid 384 and ends at amino acid 750 of the above amino acid sequence. A region from amino acid 722 to amino acid 750 of the E2 protein is considered to be a transmembrane domain (Cocquerel, L. et al., J. Virol. 74: 3623-3633, 2000).

When proteins are secreted and expressed in the culture supernatant in mammalian animal cells, it is necessary for such proteins to have signal peptides, but it is not necessary for them to have transmembrane domains.

Accordingly, a protein that does not have the transmembrane domain of the E1 or E2 protein comprises a region from amino acid 192 to amino acid 352 or a region from amino acid 384 to amino acid 721. Shift of an amino acid location is not problematic, provided that the amino acid is qualitatively equivalent. In the present invention, a sequence from amino acid 192 to amino acid 352 of the E1 protein, a sequence from amino acid 384 to amino acid 720 (and preferably a sequence from amino acid 384 to amino acid 714) of the E2 protein of the JFH-1 strain, and a sequence from amino acid 192 to amino acid 352 of the E1 protein, and a sequence from amino acid 384 to amino acid 720 of the E2 protein of the J6CF strain can be used as proteins that do not comprise a transmembrane domain. When such amino acid numbers are applied to other sequences, sequences may be designated with the amino acid numbers corresponding in the alignment with the amino acid sequences of the full-length JFH-1 proteins.

A nucleic acid that encodes a protein not containing any transmembrane domain of the E1 or E2 proteins can be synthesized via PCR using cDNA of JFH-1 as a template based on the nucleic acid sequence of JFH-1 given in GenBank, or such nucleic acid can be fully synthesized.

The corresponding E1 and E2 protein regions of HCV strains other than JFH-1 can be easily determined by aligning the sequences (making alignment), so that the length of aligned sequences become the longest in comparison with the JFH-1 sequence, while taking substitution or deletion of the sequence of the HCV strain into consideration. Such analysis can be carried out using genetic information processing software (e.g., GENETYX, Software Development Co., Ltd.).

When a protein that does not contain a transmembrane domain of the E1 or E2 protein is secreted and expressed in a mammalian animal cell, a nucleic acid encoding such protein is ligated to a site downstream of a nucleic acid that encodes a signal peptide in such a manner that frames of codons are correctly aligned (i.e., in-frame), a stop codon is added to the 3' terminus, and the resultant is then inserted into an expression vector. A signal peptide is mainly composed of hydrophobic amino acids comprising 15 to 30 amino acid residues located at the N terminus of the secretory protein and a signal peptide is associated with the mechanisms of protein transport through the cell membrane.

A signal peptide that can be used for protein secretion and expression in a mammalian animal cell may be a signal peptide of a secretory protein. Examples of vectors having signal peptides include a vector having a signal peptide sequence of mouse GM-CSF (JP Patent Publication (kokai) No. S63-276490 A (1988)), a pSecTag/FRT/V5-His vector having a signal peptide sequence of the IgG κ strand (Invitrogen), a p3xFLAG-CMV 13 vector having a signal peptide sequence of preprotrypsin (Sigma), a pFUSE-Fc2 vector having a signal peptide sequence of IL-2 (InvivoGen), and a pTriEx-7 vector having a signal peptide sequence of IgM (Novagen).

When proteins are expressed, such proteins are expressed as fusion proteins of target proteins and label proteins, and fusion proteins can be detected or purified with the use of an antibody reacting with the label protein or a molecule that specifically binds thereto. Such label proteins are also referred to as "tags." Label proteins are not limited, and examples thereof include FLAG peptide (also referred to as flag peptide or Flag peptide), 3x FLAG peptide (also referred to as 3x FLAG peptide, 3x Flag peptide, or 3x flag peptide), HA peptide, 3x HA peptide, myc peptide, 6x His peptide, GST polypeptide, MBP polypeptide, PDZ domain polypeptide, alkaline phosphatase, immunoglobulin, and avidin. Such peptides or polypeptides are generally fused to the N- or C-terminus of the target proteins, but such peptides or polypeptides can be inserted into the target proteins according to need. A vector having a fusion polypeptide of preprotrypsin signal peptide and 3x FLAG peptide is available as the p3xFLAG-CMV-9 vector from Sigma.

### (7) Preparation of monoclonal antibody

The hybridomas selected in (6) above are conditioned to serum-free medium, such as Hybridoma-SFM (Invitrogen), and the supernatant cultured in serum-free medium can be designated as a monoclonal antibody sample. Culture can be conducted with the use of a flask, petri dish, spinner culture bottle, roller bottle, or high-density culture flask (CELLine, Becton, Dickinson and Company).

When monoclonal antibodies are prepared from animals, the anti-HCV monoclonal antibody-producing hybridoma cells obtained above are intraperitoneally injected into pristane-treated 8- to 10-week-old mice, nude mice, or SCID mice (0.5 ml of 2,6,10,14-tetramethylpentadecane (pristane) was administered intraperitoneally and grown for 2 weeks) at 2x10⁷ to 5x10⁶ cells/mouse. Hybridomas experience ascites tumor formation within 10 to 21 days. Ascites fluid is sampled from the mice or nude mice to prepare monoclonal antibody samples. The obtained antibody samples are centrifuged to remove cells or fructured cells, the samples are subjected to salting-out with 40% to 50% saturated ammonium sulfate, caprylic acid precipitation, DEAE-Sepharose column, Protein-A column, Protein-G column, HiTrap IgM Purification HP-column (GE Healthcare), mannan binding protein-column (Pierce), or gel filtration column, and such techniques are carried out alone or in adequate combination to recover IgG or IgM fractions. Thus, purified monoclonal antibodies are obtained. The purified monoclonal antibody subclasses are determined with the use of, for example a mouse monoclonal antibody typing kit (Pierce). The classes of the antibodies having inhibitory activity on infection with HCV of the present invention obtained in (4) to (7) above are not particularly limited. In the present invention, IgG or IgM is preferable, with IgM antibodies being more preferable.

### (8) Preparation of humanized anti-HCV monoclonal antibody

Humanized antibodies are also referred to as reshaped human antibodies, such reshaped antibodies are obtained by transplanting complementary determining regions (CDRs) of mammalian animals other than humans (e.g., mouse antibodies) into the complementary determining regions of human antibodies, and general gene recombination techniques are known (EP Patent Publication No. EP 125023). Specifically, a DNA sequence that is designed to ligate CDR of a mouse antibody to a framework region (FR) of a human antibody is synthesized via PCR using several oligonucleotide primers prepared so as to have regions overlapping at the terminal regions of CDR and FR.

Framework regions of human antibodies ligated via complementary determining regions (CDRs) in which CDRs form good antigen-binding sites are selected. According to need, amino acids in the framework regions in the antibody variable region may be substituted, so that the CDRs of the reshaped human antibodies form adequate antigen-binding sites (Sato, K., et al., Cancer Res. 53:851-856, 1993).

When humanized anti-HCV monoclonal antibodies having inhibitory activity on infection with HCV of the present invention are prepared, for example, cDNA encoding the H chain V region (VH) and the L chain V region (VL) of the anti-HCV monoclonal antibody is first obtained in the following manner. mRNA is extracted from hybridomas that produce anti-HCV monoclonal antibodies to synthesize cDNA. The synthesized cDNA is inserted into a phage or plasmid vector to prepare a cDNA library. Recombinant phages or plasmids having cDNA encoding VH and recombinant phages or plasmids having cDNA encoding VL are isolated from the resulting library with the use of the C or V region of the mouse antibody as a probe. The whole nucleotide sequences of VH and VL of the target antibody on the recombinant phage or plasmid are determined, and the whole amino acid sequences of VH and VL are deduced based on the nucleotide sequences.

Alternatively, cDNA encoding VH and VL can be cloned via PCR. cDNAs of hybridomas prepared in the above-described manner are used as templates, the templates are amplified using a plurality of primers designed based on the amino acid sequences conserved in the relevant genes, and the cDNA fragments are cloned into cloning vectors. Thus, cDNA encoding VH and VL can be obtained.

cDNA encoding VH and VL of the anti-HCV antibody may be inserted into a region upstream of the gene encoding the H chain C region (CH) and the L chain C region (CL) of the human antibody to construct cDNA encoding a humanized anti-HCV monoclonal antibody. In CH, Cγ1, Cγ2, Cγ3, and Cγ4 can be used, and in CL, Cκ and Cλ can be used. In order to improve stability of the antibody or production thereof, the C region may be modified.

In the case of mammalian animals, a promoter that can express the gene of interest in mammalian animals, the antibody gene to be expressed, and poly A signal is operably linked to a site downstream of the 3' terminus. The gene of interest can be expressed using it. Examples of promoters that can be used include virus promoters/enhancers, such as human cytomegalovirus, retrovirus, polyoma virus, adenovirus, and simian virus 40 (SV40) and promoters/enhancers derived from mammalian cells, such as the elongation factor 1α (EF1α).

In the case of E. coli, a promoter that can express the gene of interest in E. coli cells, a signal sequence for antibody secretion, and the antibody gene to be expressed may be operably linked to express the gene of interest. Examples of promoters include lacz promoter, araB promoter, Trp promoter, and T7 promoter.

In the case of insect cells, a promoter that can express the gene of interest in insect cells, the antibody gene to be expressed, and poly A signal may be operably linked to a site downstream of the 3' terminus to express the gene of interest. Examples of promoters that can be used include polyhedrin promoter and baculovirus OpNMPV-derived immediate-early OpIE2 promoter.

The antibody having a heavy chain variable region containing the amino acid sequence as shown in SEQ ID NO: 52 and/or a light chain variable region containing the amino acid sequence as shown in SEQ ID NO: 54 according to the present invention can be prepared in the similar manner to the above-mentioned method for preparing the humanized monoclonal antibody.

### (9) Use as inhibiting agent of HCV infection

When the antibody having inhibitory activity on infection with HCV according to the present invention is used as an inhibiting agent of HCV infection, the antibody is used as a research tool for elucidating the HCV infection mechanism. Further, it is preferably used as a medicament (e.g., a pharmaceutical composition) and it is more preferably used as a therapeutic or preventive agent against hepatitis C. When the antibody according to the present invention is used as a therapeutic or preventive agent against hepatitis C, it is used for preventing HCV infection caused by organ transplantation from a donor organ. Further, it can be used in combination with existing antiviral agents, such as interferon and ribavirin.

The antibody having inhibitory activity on infection with HCV according to the present invention can be effective on arbitrary forms of hepatitis C. For example, it is effective on chronic hepatitis or fulminant hepatitis, and it is particularly effective on hepatitis C induced by HCV of genotype 2a or 1b.

When the therapeutic or preventive agent against hepatitis C of the present invention is administered to a patient, an effective dose of the antibody of the present invention as an active ingredient is between 0.001 mg and 1,000 mg per kg of the body weight. Alternatively, an dose can be between 0.01 to 100,000 mg/body of a patient, although the dose is not limited thereto. Also, the therapeutic or preventive agent can be administered before or after the patient develops clinical symptoms of the disease.

The therapeutic or preventive agent against hepatitis C of the present invention is prepared in accordance with a conventional technique (Remington's Pharmaceutical Science, the latest edition, Mark Publishing Company, Easton, U.S.A.), and such agent may comprise pharmaceutically acceptable carriers or additives.

Examples of such pharmaceutically acceptable carriers and additives include water, a pharmaceutically acceptable organic solvent, collagen, polyvinyl alcohol, polyvinyl pyrrolidone, carboxyvinyl polymer, carboxymethylcellulose sodium, sodium polyacrylate, sodium alginate, water-soluble dextran, carboxymethyl starch sodium, pectin, methylcellulose, ethylcellulose, xanthan gum, gum Arabic, casein, agar, polyethylene glycol, diglycerine, glycerine, propylene glycol, vaseline, paraffin, stearyl alcohol, stearic acid, human serum albumin (HSA), mannitol, sorbitol, lactose, and a surfactant, which is acceptable as a pharmaceutical additive.

Actual additives are selected from among the above-mentioned additives in accordance with a dosage form of the therapeutic or preventive agent against hepatitis C according to the present invention, and such additive can be used alone or in adequate combinations, although additives are not limited to the above-mentioned additives. When an additive is used for an injection preparation, for example, the purified anti-HCV antibody is dissolved in a solvent, such as physiological saline, buffer, or a glucose solution, an adsorption inhibitor, such as Tween 80, Tween 20, gelatin, human serum albumin, or the like is added thereto, and the resulting mixture can be used. Alternatively, an additive may be lyophilized so as to be in a dosage form that allows it to be dissolved and reconstructed before use. Examples of excipients that can be used for lyophilization include sugar alcohols, such as mannitol or dextrose, and sugars.

### (10) Use of antibody for HCV detection

The antibody of the present invention can be applied to a diagnostic composition for HCV detection.

For example, a carrier or plate upon which the antibody of the present invention has been immobilized is brought into contact with a test sample, which may contain the HCV antigen, to produce a mixture. The resulting mixture is subjected to a reaction for a period of time under conditions that are sufficient for forming an antibody/antigen complex. Subsequently, the thus-generated complex is brought into contact with an antibody that recognizes the HCV antigen to which a signal enzyme, dye, or radioisotope has been bound to generate a second mixture. The second mixture is subjected to the reaction for a period of time under conditions that are sufficient for forming an antibody/antigen complex. The presence of the HCV antigen is detected with the aid of a signal of an enzyme, dye, or radioisotope.

Alternatively, a test sample, which may contain the HCV antigen, is spotted on a membrane on which proteins can be immobilized (e.g., a nitrocellulose membrane or PVDF membrane) to immobilize proteins contained in a test sample. Subsequently, this membrane is soaked in a 5% skimmed milk or 1% BSA solution to block the membrane. After the membrane has been washed, the membrane is soaked in a solution of the antibody of the present invention to which an enzyme, dye, or radioisotope has been bound, and the reaction is allowed to proceed for a period of time under the conditions that are sufficient for the antigen immobilized on the membrane and the antibody of the present invention to form a complex. After the membrane has been washed, the presence of the HCV antigen immobilized on the membrane is then detected with the aid of a signal of an enzyme, dye, or radioisotope.

### (11) Use for anti-HCV antibody detection

As another use of the present invention, an anti-HCV antibody that recognizes the same epitope as the antibody of the present invention can be detected. The present invention can be used as a competitive probe against the antibody of the present invention reacting with the HCV antigen and a test antibody. For example, the HCV antigen is immobilized upon a plate or membrane, the antibody of the present invention to which an enzyme, dye, or radioisotope has been bound and the test antibody are added, and the reaction is allowed to proceed for a certain period of time under conditions that are sufficient for the HCV antigen and the antibody to form a complex. By assaying a decrease in binding of the enzyme, dye, or radioisotope signal to a solid phase, whether or not the test antibody detects the same epitope that is recognized by the antibody of the present invention can be detected.

### Examples

Hereafter, the present invention is described in greater detail with reference to the examples. It should be noted that these examples are provided for illustrative purposes and the technical scope of the present invention is not limited to these examples.

### [Example 1] Preparation of JFH-1-HCV particles, J6/JFH-1-HCV particles, and TH/JFH-1-HCV particles

pJFH-1, which is plasmid DNA obtained by cloning cDNA (genome-length cDNA) obtained via reverse trascription of the entire genome RNA region of the JFH-1 strain (genotype 2a) of the hepatitis C virus (HCV) isolated from a fulminant hepatitis patient downstream of the T7 RNA promoter sequence of the pUC19 plasmid, was prepared as described in Wakita, T. et al., Nat. Med., 11, 2005, pp. 791-796 and International Publication WO 2004/104198. The nucleotide sequence of the genome-length cDNA derived from the JFH-1 strain inserted into pJFH-1 is as shown in SEQ ID NO: 1. pJFH-1 was digested with EcoRI and then partially digested with BclI to prepare a plasmid DNA fragment, which lacks a fragment of approximately 2,840 bp from the EcoRI site to the first BclI site, and the resulting fragment was purified.

pJ6CF, which is obtained by cloning genome-length cDNA derived from the HCV J6CF strain (GenBank Accession Number AF177036, Yanagi, M., et al., Virology 262: 250-263, 1999) downstream of the T7 RNA promoter sequence of the pUC19 plasmid, was prepared as described in International Publication WO 2006/022422. pJ6CF was partially digested with EcoRI and BclI, the resulting fragment of approximately 2,840 bp was purified, and the resultant was ligated to the pJFH-1 fragment lacking EcoRI-BclI as described above to prepare plasmid DNA pJ6/JFH-1. DNA (SEQ ID NO: 2) cloned into pJ6/JFH-1 is a chimeric viral genome-length cDNA comprising a 5 ' untranslated region, the sequence encoding core, E1, E2, and p7 proteins, and the sequence encoding a region from the N terminus to amino acid residue 16 of the NS2 protein of the genome-length cDNA of the J6CF strain; and the sequence encoding a region from amino acid residue 17 to the C terminus of the NS2 protein, the sequence sequentially encoding the NS3, NS4A, NS4B, NS5A, and NS5B proteins, and the 3' untranslated region of the genome-length cDNA of the JFH-1 strain ligated thereto.

Subsequently, plasmid DNA pTH/JFH-1 was prepared in the following manner. At the outset, 10 µl of 10x buffer supplied with the Phusion High-Fidelity DNA Polymerase kit (FINNZYMES), 4 µl of 2 mM dNTP mix, 1 µl each of 10 µM primers JFH-1-A (SEQ ID NO: 4: TGTAAAACGACGGCCAGT) and JFH-1-B (SEQ ID NO: 5: GGTTTAGGATTCGTGCTCATGGTGCACGGTCTACGAGACC) were added to the genome-length cDNA in pJFH-1 as a template, and deionized water was added to bring the total amount to 49.5 µl in the end. Then, 0.5 µl of Phusion DNA Polymerase (FINNZYMES) was added thereto, and PCR was carried out to amplify a DNA fragment comprising a part of the core protein-encoding sequence of the HCV TH strain contained in the JFH-1-B primer sequence, in addition to the 5' untranslated region of the JFH-1 strain. PCR was carried out with 30 cycles of 98°C for 10 seconds, 55°C for 15 seconds, and 72°C for 30 seconds. The resulting PCR product was designated as PCR product No. 1. Subsequently, 10 µl of 10x buffer supplied with the Phusion High-Fidelity DNA Polymerase kit (FINNZYMES), 4 µl of 2 mM dNTP mix, and 1 µl each of 10 µM primers TH-C (SEQ ID NO: 6: GGTCTCGTAGACCGTGCACCATGAGCACGAATCCTAAACC) and TH-D (SEQ ID NO: 7: AGATAGCACAACCACCACAGGAGCTTGGCGAGGAATGCCT) were added to the pTH plasmid containing genome-length cDNA derived from the HCV TH strain (Wakita et al., J.Biol.Chem., 269: 14205-14210, 1994; and Moradpour et al., Biochem. Biophys. Res. Commun., 246: 920-924, 1998) as a template, and then deionized water was added to bring the total amount to 49.5 µl in the end. 0.5 µl of Phusion DNA Polymerase (FINNZYMES) was added thereto, and PCR was carried out to amplify a DNA fragment comprising a part of the 5' untranslated region of the JFH-1 strain contained in the TH-C primer sequence and a part of the NS2 protein-encoding sequence of the JFH-1 strain contained in the YH-D primer sequence, in addition to a sequence comprising a region from a major part of the core protein-encoding sequence to a part of the NS2 protein-encoding sequence of the TH strain. PCR was carried out with 30 cycles of 98°C for 10 seconds, 55°C for 15 seconds, and 72°C for 30 seconds. The resulting PCR product was designated as PCR product No. 2. Further, 10 µl of 10x buffer supplied with the Phusion High-Fidelity DNA Polymerase kit (FINNZYMES), 4 µl of 2 mM dNTP mix, and 1 µl each of 10 µM primers JFH-1-E (SEQ ID NO: 8: AGGCATTCCTCGCCAAGCTCCTGTGGTGGTTGTGCTATCT) and JFH-1-F (SEQ ID NO: 9: CAGCTACCGAGGGGTTAAGC) were added to the above-mentioned pJFH-1 as a template, and then deionized water was added to bring the total amount to 49.5 µl in the end. 0.5 µl of Phusion DNA Polymerase (FINNZYMES) was added thereto, and PCR was carried out to amplify a DNA fragment comprising a part of the NS2 protein-encoding sequence of the TH strain contained in the JFH-1-E primer sequence, in addition to a part of the NS2 and NS3 protein-encoding sequence of the JFH-1 strain. PCR was carried out with 30 cycles of 98°C for 10 seconds, 55°C for 15 seconds, and 72°C for 30 seconds. The resulting PCR product was designated as PCR product No. 3.

Then, PCR was carried out using the PCR products No. 1, No. 2, and No. 3 prepared as described above, with the JFH-1-A primer (SEQ ID NO: 4) and the JFH-1-E primer (SEQ ID NO: 8). Thus, a fragment comprising the 5' untranslated region of the JFH-1 strain, a region from the core protein-encoding sequence to a part of the NS2 protein-encoding sequence of the TH strain, and a region from a part of the NS2 protein-encoding sequence to a part of the NS3 protein-encoding sequence of the JFH-1 strain ligated to each other was obtained (PCR product No. 4).

Subsequently, pJFH-1 and PCR product No.4 were treated with the EcoRI and SpeI restriction enzymes, and the resultants were ligated to obtain plasmid DNA pTH/JFH-1. The nucleotide sequence of the chimeric viral genome-length cDNA cloned into the resultant pTH/JFH-1 is shown in SEQ ID NO: 3.

pJFH-1, pJ6/JFH-1, and pTH/JFH-1 were cleaved with XbaI, and then subjected to phenol/chloroform extraction and ethanol precipitation. The cleaved plasmids each were used as a template and RNAs were synthesized using the MEGAscript T7 kit (Ambion) (see WO 2006/022422). The thus-synthesized HCV RNAs each were used for introduction into cells as described below.

The Huh-7 cells (3 x 10⁶ cells) and 5 µg of the HCV RNA were suspended in 400 µl of the Cytomix solution (120 mM KCl, 0.15 mM CaCl₂, 10 mM K₂HPO₄/KH₂PO₄, 25 mM Hepes, 2 mM EGTA, 5 mM MgCl₂, 20 mM ATP, and 50 mM glutathione), the suspension was transferred to a 4-mm cuvette, and subjected to electroporation of HCV RNA into Huh-7 cells using the Gene Pulser (BioRad) at 260 V and 950 µF. Thereafter, the cells into which HCV RNA had been introduced were seeded on a 10 cm² dish and then subcultured. At the time of subculture, the HCV core proteins contained in the culture supernatant were quantified using the HCV antigen ELISA test kit (ORTHO) to confirm the production of HCV particles. The culture supernatant comprising large quantities of core proteins and having high HCV particle production activity was selected and stored as a virus stock solution.

To the Huh-7 cells that had been cultured in 10% FCS-DMEM medium (1% MEM Non-essential Amino Acid Solution (Invitrogen), 10 mM HEPES-Tris (pH 7.3), and 1 mM sodium pyruvate) in a 10-cm dish, approximately 100 µl of the JFH-1 or J6/JFH-1 virus stock solution obtained above (4 x 10⁴ ffu/ml) was added to infect the Huh-7 cells with the HCV viruses.

The cells were adequately subcultured while avoiding cell culture becoming confluent, and culture expansion was carried out in 225 cm² flasks from one flask to 4 flasks and then 12 flasks. Subsequently, cells were detached from 8 of such 225-cm² flasks, and seeded in two 5-layer Cellstacks^{™} (Corning), and a medium was added thereto in an amount of 650 ml/cellstack. The cells obtained from the other 4 flasks were seeded in 12 flasks and virus production was effectively continued.

On the day following subculture, the media were discarded and 650 ml of 2% FCS-DMEM medium (1% MEM Non-essential amino acid solution (Invitrogen), 10 mM HEPES-Tris (pH 7.3), 1 mM sodium pyruvate) was added. The media were recovered 3 days after medium exchange, passed through a 0.45-µm filter, and the resultants were stored in a deep freezer. Also, 650 ml of 2% FCS-DMEM medium (1% MEM Non-essential amino acid solution, 10 mM HEPES-Tris (pH 7.3), and 1 mM sodium pyruvate) was added to the Cellstacks after the culture supernatants were recovered, and culture was continued. A similar procedure was repeated 2 days after the medium exchange and the culture supernatants were recovered. A similar procedure was then repeated one more time. The culture supernatants recovered herein were used in the Examples below.

Thus, it was demonstrated that infectious HCV particles were produced in the culture supernatants of cells into which RNAs synthesized from pJFH-1, pJ6/JFH-1, and pTH/JFH-1 each had been introduced.

### [Example 2] Purification of JFH-1, J6/JFH-1, and TH/JFH-1 HCV particles

The virus particles produced in Example 1 were purified via the following 4 steps.

### 1) Concentration

With the use of Pellicon 2 Mini Ultrafiltration Module PLCMK V 0.1 m² (300 kDa cut-off, Catalog No. P2C300V01, Millipore, hereafter referred to as "pellicon 2 mini"), the culture supernatant containing the HCV particles obtained in the Example above was concentrated to approximately 30- to 50-fold. The concentrate was filtered through a 0.45-µm filter and then stored at -80°C.

### 2) Density-gradient ultracentrifugation

To the Ultra-clear 25 x 89 mm centrifuge tube (Catalog No. 344058, Beckman Coulter), 3 ml of TNE buffer containing cold 60% sucrose (10 mM Tris-HCl (pH 7.4), 150 mM NaCl, 1 mM EDTA) was added, and 7 ml of TNE buffer containing 20% sucrose was overlaid thereon. Further, 25 ml of the sample was overlaid onto the TNE buffer containing 20% sucrose. Ultracentrifugation was carried out using a SW-28 (Beckman Coulter) at 28,000 rpm for 4 hours at 4°C.

The bottom of the tube was perforated using the 25G injection needle (Terumo) and 2.5 ml, 3 ml, 4.5 ml, and 25 ml fractions were successively obtained.

### 3) Heparin column purification

A 5-ml HiTrap Heparin HP column was equilibrated with 20 mM phosphate buffer (pH 7). The diluted fraction (not more than 6 columns) was applied to HiTrap Heparin at a flow rate of 5 ml/min or slower. The column was washed by passing 50 ml of 20 mM phosphate buffer (pH 7). The elution fraction was obtained by applying 25 ml of 20 mM phosphate buffer (pH 7) containing 500 mM NaCl thereto. Further, the column was washed by passing 50 ml of 20 mM phosphate buffer (pH 7) containing 3.8 M NaCl, the column was then equilibrated with 20 mM phosphate buffer (pH 7), and the column was stored.

### 4) Concentration and buffer exchange

The elution fraction was subjected to buffer exchange and concentration using Amicon. Ultra-15 Centrifugal Filter Units (Millipore) and TNE buffer. The thus-obtained concentrate was used as a virus solution containing infectious virus particles in the immunization step described below.

### [Example 3] Virus inactivation

The concentrated hepatitis C viruses obtained by the steps 1) to 4) in Example 2 were inactivated via ultraviolet irradiation. As a source of ultraviolet rays, GL-15 (Toshiba) was used. The solution containing purified hepatitis C virus particles (the JFH-1 strain) having an infectious titer of 1 x 10⁶ ffu/ml was introduced into a silicon-coated polyethylene Eppendorf tube (Assist Co., Ltd,), the tube was placed at a distance from the source of ultraviolet rays, so that the ultraviolet rays would be applied at the intensity of 20 mW/cm², and UV-C was applied for 5 minutes.

After ultraviolet irradiation, hepatitis C virus particles were serially diluted 50-fold, 250-fold, 1,250-fold, 6,250-fold, 31,250-fold, 156,250-fold, and 781,250-fold in Dulbecco's modified Eagle medium (DMEM).

On the previous day, the Huh-7 cells were seeded on a 96-well poly-L-lysine-coated plate (Corning 96 Well Clear Flat Bottom Poly-L-Lysine Coated Microplate, Corning) at 1 x 10⁴ cells/well, the serially-diluted virus particles were seeded thereonto, and culture was conducted at 37°C for 72 hours.

After the culture supernatant was removed, the plate was soaked in ice-cold methanol to fix the cells. Thereafter, methanol was removed via air drying, and cells were permeabilized with the use of Block Ace^{®} (Dainippon Pharmaceutical Co., Ltd.) containing 0.3% Triton^{®}-X 100 (GE Healthcare). HCV-infected cells were detected using the clone 2H9 anti-HCV-core antibody (Wakita, T. et al., Nat. Med. 11:791-796, 2005) and goat anti-mouse IgG-Alexa488 (Molecular Probes), and the number of HCV-infected cells was counted under a fluorescent microscope (IX-70; Olympus). The infectious titer of the ultraviolet-irradiated hepatitis C viruses was found to be below detection limit. HCV particles, which were demonstrated to have completely lost infectivity, were used for administration to mice in the Examples below.

### [Example 4] Immunization of mouse using inactivated HCV particles

Adjuvant was first prepared. To 100 µl of a solution containing J6/JFH-1-HCV particles (equivalent to 1.4 µg of HCV core proteins; equivalent to 10 µg of total HCV proteins), which had been inactivated as described in Example 3, the equivalent amount of Freund's complete adjuvant (Difco) was added to generate an emulsion. Generation of an emulsion was confirmed by preparing an adequate amount of water in a beaker, dropping a drop of the relevant mixture on the liquid surface, and observing that the liquid would not disperse. Balb/c mice (7-week-old, females) were etherized, and the emulsion containing inactivated J6/JFH-1-HCV particles thus prepared was intraperitoneally administered thereto to immunize the mice.

Two weeks later, to 100 µl of a solution containing J6/JFH-1-HCV particles (equivalent to 1.4 µg of HCV core proteins; equivalent to 10 µg of total HCV proteins), the equivalent amount of Freund's incomplete adjuvant (Difco) was added to generate an emulsion, and then mice were further immunized with the emulsion via intraperitoneal administration as described above. The emulsion was further administered intraperitoneally to mice 3 weeks later for further immunization.

Also, mice were immunized with the use of the JFH-1-HCV particles inactivated in Example 3 according to the above immunization schedule in the same manner.

### [Example 5] Measurement of inhibitory activity on infection with HCV in blood serum derived from mouse immunized with inactivated HCV particles

The blood serum samples that were obtained via partial blood sampling from mice immunized with inactivated HCV particles (J6/JFH-1-HCV particles or JFH-1-HCV particles) having structural proteins of genotype 2a among the mice that had been immunized in Example 4 were subjected to measurement of inhibitory activity on infection with HCV.

Huh7 cells were seeded on a 96-well plate at 5 x 10³ cells/well, and the cells were cultured overnight. The serum and heparin (10 units/ml) were added to the concentrated J6/JFH-1-HCV virus solution obtained in step 1) of Example 2 and the resultant was incubated at 37°C for 1 hour. The serum was diluted 20-fold and 100-fold using medium (DMEM containing 10% FCS (Invitrogen), 1% MEM Non-essential amino acid solution (Invitrogen), 10 mM HEPES-Tris (pH 7.3), and 1 mM sodium pyruvate) before using. After the medium was discarded, the virus solution was added at 50 µl/well and incubation was then carried out at 37°C for 3 hours. After the medium was discarded, the plate was washed once with PBS, medium was added at 100 µl/well, and incubation was then carried out at 37°C for 48 hours. After the medium was discarded, the plate was washed once with PBS, Isogen (Wako Pure Chemical Industries, Ltd.) was added at 100 µl/well, and total RNA was extracted according to accompanying instruction. Total RNA was quantified via quantitative RT-PCR, and the RNA copy number per 1 µg of total RNA was determined.

Further, whether or not the serum derived from mice immunized with J6/JFH-1-HCV particles having structural proteins of genotype 2a has activity of inhibiting infection with HCV having structural proteins of genotype 1b was examined in the following manner. To this end, chimeric HCV particles (TH/JFH-1-HCV) having structural proteins of the TH strain of genotype 1b and chimeric J6/JFH-1-HCV particles having structural proteins of genotype 2a prepared in Example 1 and in Example 2 were used as infectious agents to measure activity of the serum for inhibiting the infection in the same manner as described above. A control experiment was carried out in the same manner, except for the use of normal mouse serum sampled before administration of HCV particles.

As a result, the 20-fold diluted serum obtained from mice 62 days after immunization thereof with J6/JFH-1-HCV particles was found to have an infection level of J6/JFH-1-HCV lowered to 15% compared with the control experiment (100%), and thus the blood serum was found to have activity of inhibiting infection with HCV particles having structural proteins of genotype 2a (Fig. 1A). Further, the 20-fold diluted blood serum lowered infection level of TH/JFH-1-HCV to 34% compared with the control experiment (100%), and thus the blood serum was found to have activity of inhibiting infection with HCV particles having structural proteins of genotype 1b (Fig. 1B). These results demonstrate that antibodies induced via immunization with the HCV of genotype 2a include antibodies inhibiting infection with HCV of genotype 1b as well as antibodies inhibiting infection with HCV of genotype 2a.

Even when the sera from two mice obtained 42 days after immunization with JFH-1-HCV particles had been diluted 100-fold for use, the infection levels of JFH-1-HCV of genotype 2a were decreased, compared with the blood serum collected before HCV particle administration (100%) in the control experiment, and thus it was confirmed that the sera have activity of for inhibiting the infection (Fig. 2).

Subsequently, 200 µl of the blood sera from a mouse to which J6/JFH-1-HCV particles had been administered and 200 µl of the serum collected from a normal mouse before HCV particle administration each were applied to 1-ml Protein G Sepharose-columns (GE Healthcare), and the samples were eluted with 0.1M glycine buffer (pH 3.0) in amounts of 1 ml/fraction, following column washing. The eluted protein peak fraction was designated as the IgG fraction, and the IgG fraction and the non-adsorbed fraction (the IgM fraction) were separately prepared as purified fractions. Their inhibitory activities on infection with HCV thereof were measured in a similar manner as described above, as follows.

Instead of the blood serum samples used in the above-mentioned measurement, purified IgG obtained from the blood serum of mice to which HCV particles had been administered was used at 100 µg/ml or 10 µg/ml, or purified IgG obtained from normal mouse serum was used at 100 µg/ml. HCV particles (in the virus solution) were mixed with IgG and the like so as to achieve 10⁷ copies/ml, and allowed to stand for 1 hour at room temperature. Subsequently, the medium used for the Huh-7 cells that had been seeded at 4 x 10⁴ cells/well on a 24-well plate on the previous day and cultured therein was discarded, and the mixture of virus particles and IgG antibody as described above was added to the cells at 200 µl/well. The cells were incubated for 3 hours at 37°C, the solution containing virus particles was discarded, and the wells were washed once with PBS. A fresh medium was added thereto, culture was conducted for 2 days, the medium was discarded, the wells were washed once with PBS, and then RNA was extracted from the cells using the Trizol reagent (Invitrogen). The copy number of HCV RNA contained in the extracted RNA was determined. The determination of the RNA copy number was done according to the method of Takeuchi et al. (Gastroenterology, 1999, 116, pp. 636-42.).

Subsequently, the activity of inhibiting infection of the fraction that did not adsorb to Protein G Sepharose (i.e., the IgM fraction) was measured in the same manner. As a positive control, a blood serum (unpurified) sample obtained from a mouse to which J6/JFH-1-HCV particles had been administered was used. Protein content in the blood serum sample obtained from a mouse to which J6/JFH-1-HCV particles had been administered and protein content in the IgM fraction were measured, and then the experiment for measurement of the activity of inhibiting infection was carried out while adding the same amounts of proteins to the samples. Specifically, the blood serum obtained from a mouse to which J6/JFH-1-HCV particles had been administered was mixed so as to become 5% and HCV particles were mixed so as to become 10⁷ copies/ml in the mixture. The IgM fraction was mixed in an amount so as to contain the same amount of proteins as in the aforementioned 5% mouse serum, and HCV particles were mixed so as to become 10⁷ copies/ml. The mixtures were allowed to stand for 1 hour at room temperature. The medium used for the Huh-7 cells that had been seeded at 4 x 10⁴ cells/well on a 24-well plate on the previous day and cultured therein was discarded, and the mixture of virus particles and serum or IgM was added thereto at 200 µl/well. The cells were incubated for 3 hours at 37°C, a solution containing virus particles was discarded, and the wells were washed once with PBS. A fresh medium was added, culture was conducted for 2 days, the medium was discarded, the wells were washed once with PBS, and RNA was extracted from the cells using the Trizol reagent (Invitrogen). The copy number of HCV RNA contained in the extracted RNA was determined. The determination of the RNA copy number was done according to the method of Takeuchi et al. (Gastroenterology, 1999, 116, pp. 636-42.).

As a result, the IgM fraction derived from the blood serum obtained from a mouse to which J6/JFH-1-HCV particles had been administered was found to have inhibitory activity on infection with HCV, as shown in Fig. 3.

### [Example 6] Preparation of hybridomas

The mouse myeloma cell line SP2/0 cells (obtained from ECACC) were cultured in Dulbecco's modified MEM medium (Invitrogen) containing 5 x 10⁻⁵ M 2-mercaptoethanol, 100 units/ml penicillin, 100 µg/ml streptomycin, and 10% fetal calf serum (FCS, Invitrogen), and SP2/0 cells were obtained in the logarithmic growth phase. The cells were washed three times with serum-free Dulbecco's modified MEM.

Subsequently, spleen cells were prepared from mice to which HCV particles of Example 4 (JFH-1 particles or J6/JFH-1 particles) had been administered, and washed three times with serum-free Dulbecco's modified MEM. The SP2/0 cells and the mouse spleen cells were introduced at a ratio of 1:5 into a 50-ml centrifuge tube and the cells were centrifuged at 1,000 rpm for 10 minutes. The supernatant was completely aspirated off, and the bottom of the tube was tapped with fingers to loosen the cell pellet. 1 ml of 50% polyethylene glycol-1500 solution heated at 37°C (Roche) was added to the cells for 1 minute, and the reaction was allowed to continue at 37°C for 1 minute. Subsequently, 1 ml of serum-free Dulbecco's modified MEM was gradually added for 1 minute, and 1 ml of serum-free Dulbecco's modified MEM was gradually added again for 1 minute. In the end, 7 ml of serum-free Dulbecco's modified MEM was added for 3 minutes to dilute the ethylene glycol solution. The cells in the diluent were centrifuged at 1,000 rpm for 10 minutes, 50 ml of HT medium (Dulbecco's modified MEM medium containing 5 x 10⁻⁵M 2-mercaptoethanol, 100 units/ml penicillin, 100 µg/ml streptomycin, 10% FCS, 10⁻⁴M hypoxanthine, and 1.5 x 10⁻⁵M thymidine) was added thereto, and the cell pellet was loosened via pipetting. The cells were transferred to two 75-cm² flasks and cultured at 37°C in a 5% CO₂ incubator overnight.

The cells were centrifuged at 1,000 rpm for 10 minutes and recovered. The cell pellet was loosened via tapping and suspended in 10 ml of Dulbecco's modified MEM. The cell suspension was added to and thoroughly mixed in 90 ml of methylcellulose HAT selection medium (Stem Cell Technology), the resultant was added to 10-cm dishes in amounts of 10 ml per dish, and culture was conducted at 37°C in a 5% CO₂ incubator.

After the culture for 10 to 14 days, hybridoma colonies, each of which was considered to have grown from a single cell, were suctioned with a pipette chip, and each introduced into wells of a 96-well plate to which 200 µl each HT medium containing 10% hybridoma growth factors (Bio Veris) had been added, and culture was then conducted.

### [Example 7] Screening of hybridomas producing an antibody inhibiting HCV infection

When the hybridomas prepared in Example 6 had sufficiently proliferated, the culture supernatants were recovered, and screening thereto was carried out as follows.

Screening was carried out by immobilizing E1 proteins and E2 proteins on a plate, evaluating whether or not the antibodies in the hybridoma supernatant would bind to the proteins immobilized on the plate via EIA, and evaluating whether or not the antibodies in the hybridoma supernatant would be able to inhibit HCV infection.

### 1) Preparation of E1 and E2 proteins derived from J6CF strain

E1 and E2 proteins of the J6CF strain were prepared as follows. With the use of genome-length cDNA derived from the J6CF strain of genotype 2a (GenBank Accession Number AF177036) as a template, a gene encoding an E1 protein lacking a transmembrane region, which is equivalent to a region from amino acids 192 to 352 when amino acid 1 starts at the initiator methionine at the N terminus of the full-length protein sequence of J6CF (i.e., the continuous protein sequence encoded by the genome sequence of the J6CF strain: GenBank Accession Number AF177036), was amplified via PCR using the Advantage GC2 PCR kit (Takara Bio) with J6E1dTM-s (SEQ ID NO: 10: CACAAGCTTGCCGAAGTGAAGAACATCAGT) and J6E1dTM-as (SEQ ID NO: 11: GCTCTAGATTAATGAGCCCCGCTAATGATGTC). The amplified DNA fragment was cloned into pCR-TOPO (Invitrogen), and 3 clones were subjected to nucleotide sequence analysis. A clone containing an insert having a correct nucleotide sequence was designated as pTOPO-J6E1dTM.

pTOPO-J6E1dTM was digested with HindIII and XbaI and a gel containing the resultant DNA fragment of approximately 500 bp (the E1 fragment) was excised. The DNA fragment was purified from the gel using GeneElute (SIGMA). Similarly, p3xFLAG-CMV-9 (SIGMA) was digested with HindIII and XbaI, the resultant was electrophoresed on 1% agarose gel, a gel containing a fragment of approximately 6,400 bp was excised, and the DNA fragment was purified from the gel using GeneElute (SIGMA). The purified DNA fragments were ligated to each other using T4 ligase (Takara Bio), thereby obtaining an animal cell expression vector CMV-3xFLAGJ6E1dTM into which the J6CF E1 fragment had been incorporated.

Subsequently, a gene encoding the E2 protein lacking a transmembrane region, which is equivalent to a region from amino acids 384 to 720 when amino acid 1 starts at the initiator methionine at the N terminus of the full-length protein sequence of the J6CF strain, was amplified via PCR using the Advantage GC2 PCR kit (Takara Bio) with J6E2dTM-s (SEQ ID NO: 12: CACAAGCTTCGCACCCATACTGTTGGGG) and J6E2dTM-as (SEQ ID NO: 13: GCTCTAGATTACCATCGGACGATGTATTTTGT). The amplified DNA fragment was cloned into pCR-TOPO (Invitrogen), and 3 clones were subjected to nucleotide sequence analysis. A clone containing an insert having a correct nucleotide sequence was designated as pTOPO-J6E2dTM.

Subsequently, DNA obtained by digesting p3xFLAG-CMV-9 (SIGMA) with HindIII and XbaI was ligated with the aid of T4 DNA ligase to the DNA fragment of approximately 1,000 bp excised from pTOPO-J6E2dTM with HindIII and XbaI for cyclization. The resulting vector was designated as CMV-3xFLAGJ6E2dTM.

CMV-3xFLAGJ6E1dTM and CMV-3xFLAGJ6E2dTM were introduced into the COS1 cells derived from monkey kidney cells (Accession Number RCB0143, obtained from Riken Cell Bank) to express proteins therein as described below.

The COS1 cells were cultured in Dulbecco's MEM (D-MEM: Invitrogen) containing 10% fetal calf serum (Invitrogen), 100 U/ml penicillin, and 100 µg/ml streptomycin sulfate. The COS1 cells were seeded in a 150-cm² culture flask (Corning Coaster) at a split ratio of 1:2 on the previous day of gene introduction, and the cells were cultured at 37°C in a 5% CO₂ incubator overnight.

Separately, DEAE dextran (Pharmacia) and chloroquine (SIGMA) were added to D-MEM medium (Invitrogen) to result in final concentrations of 400 µg/ml and 100 µM, respectively, 50 µg of the expression vector (CMV-3xFLAGJ6E1dTM or CMV-3xFLAGJ6E2dTM) was added at 0.1 µg/µl per 13 ml of the solution, and culture was then conducted. Subsequently, the supernatant of the cultured COS1 cells was aspirated off, and 10 ml of PBS(-) (Nissui) was added thereto and the cells were washed once. After PBS(-)was aspirated off, 13 ml of the DEAE dextran-DNA mixture was added thereto per 150-cm² flask, and incubation was then carried out at 37°C in the presence of 5% CO₂ for 4 hours.

4 hours later, the DEAE dextran-DNA mixture was aspirated off, and the cells were washed once with 10 ml of PBS. CHO-SFM medium (Invitrogen) was added thereto in amounts of 50 ml per flask, and culture was conducted at 37°C in the presence of 5% CO₂. The culture supernatant was collected in a 50-ml centrifuge tube (Corning Coaster) 4 days later. The collected supernatant was centrifuged at 6,000 rpm (with the use of a HITACHI RPR9-2 rotor) for 30 minutes at 4°C and filtered through a 0.2-µm filter (Whatman).

The culture supernatant was purified with the use of anti-FLAG M2 agarose (SIGMA) as follows. To 500 ml of the culture supernatant, 1 ml of anti-FLAG M2 agarose was added, and the reaction was allowed to proceed at 4°C (in a low-temperature chamber) for 2 hours while undergoing agitation in a spinner bottle. A mixture of the supernatant and anti-FLAG M2 agarose was transferred to the Econo-Column (BIO-RAD) 2 hours later, and flow-through fractions were collected. Subsequently, the column was washed twice with 10 ml of TBS (50 mM Tris-HC1, 150 mM NaCl, pH 7.4). Six fractions (1 ml of each fraction) were eluted with the use of 0.1M glycine-HCl (pH 3.5). Immediately after elution, 1M Tris-HCl (pH 9.5) was added for neutralization. The fractions (20 µl each) were subjected to SDS-polyacrylamide gel electrophoresis under reducing conditions and stained with Coomassie brilliant blue. As a result, E1 proteins or E2 proteins derived from the J6CF strain were found to be purified.

### 2) Preparation of E1 and E2 proteins derived from JFH-1 strain

E1 and E2 proteins of the JFH-1 strain were prepared as follows. With the use of genome-length cDNA derived from the JFH-1 strain of genotype 2a as a template, a gene encoding the E1 protein, which is equivalent to a region from amino acids 192 to 352 when amino acid 1 starts at the initiator methionine at the N terminus of the full-length protein sequence (i.e., the continuous protein sequence encoded by the genome sequence of the JFH-1 strain: the amino acid sequence disclosed in GenBank Accession Number AB047639; Kato, T. et al., Gastroenterology, 125, 2003, pp. 1808-1817), was amplified via PCR using the Advantage GC2 PCR kit (Takara Bio) with JFHE1dTM-s (SEQ ID NO: 14: CACAAGCTTGCCCAGGTGAAGAATACCAGT), and JFHE1dTM-as (SEQ ID NO: 15: GCTCTAGATTAGTGAGCCCCGCTAACGATGTC). The amplified DNA fragment was cloned into pCR-TOPO. Three clones were subjected to nucleotide sequence analysis, and a clone containing an insert having a correct nucleotide sequence was designated as pTOPO-JFHE1dTM.

pTOPO-JFHE1dTM was digested with HindIII and XbaI and a gel containing the resultant DNA fragment of approximately 500 bp (the E1 fragment) was excised. The DNA fragment was purified from the gel using GeneElute (SIGMA). Similarly, p3xFLAG-CMV-9 (SIGMA) was digested with HindIII and XbaI, the resultant was electrophoresed on 1% agarose gel, a gel containing a fragment of approximately 6,400 bp was excised, and the DNA fragment was purified from the gel using GeneElute (SIGMA). The purified DNA fragments were ligated to each other using T4 ligase (Takara Bio), thereby obtaining an animal cell expression vector CMV-3xFLAGJFHE1dTM into which the JFH-1 E1 fragment had been incorporated.

Subsequently, a gene encoding the E2 protein lacking a transmembrane region, which is equivalent to a region from amino acids 384 to 714 when amino acid 1 starts at the initiator methionine at the N terminus of the full-length protein sequence of the JFH-1 strain, was amplified via PCR using the Advantage GC2 PCR kit (Takara Bio) with JFE2dTM-s (SEQ ID NO: 16: CACAAGCTTGGCACCACCACCGTTGGAG) and JFE2dTM-as (SEQ ID NO: 17: GCTCTAGATTATGTGATAGCAGGTGAGAGGCC). The amplified DNA fragment was cloned into pCR-TOPO (Invitrogen), and 3 clones were subjected to nucleotide sequence analysis. A clone containing an insert having a correct nucleotide sequence was designated as pTOPO-JFHE2dTM.

pTOPO-JFHE2dTM was digested with HindIII and XbaI and a gel containing the resultant E2 fragment of approximately 1,000 bp was excised. The DNA fragment was purified from the gel using GeneElute (SIGMA). Similarly, the p3xFLAG-CMV-9 vector (SIGMA) was digested with HindIII and XbaI, the resultant was electrophoresed on 1% agarose gel, a gel containing a fragment of approximately 6,400 bp was excised. The DNA fragment was purified from the gel using GeneElute (SIGMA). The purified DNA fragments were ligated to each other using T4 ligase (Takara Bio), thereby obtaining an animal cell expression vector CMV-3xFLAGJFHE2dTM into which the JFH-1 E2 fragment had been incorporated.

CMV-3xFLAGJFHE1dTM and CMV-3xFLAGJFHE2dTM were introduced into the COS1 cells derived from monkey kidney cells (Accession Number RCB0143, obtained from Riken Cell Bank) to express proteins therein as described below.

The COS1 cells were cultured in Dulbecco's MEM (D-MEM: Ivitrogen) containing 10% fetal calf serum (Invitrogen), 100 U/ml penicillin, and 100 µg/ml streptomycin sulfate. The COS1 cells were seeded in a 150-cm² culture flask (Corning Coaster) at a split ratio of 1:2 on the previous day of gene introduction, and the cells were cultured at 37°C in a 5% CO₂ incubator overnight.

Separately, DEAE dextran (Pharmacia) and chloroquine (SIGMA) were added to D-MEM medium (Invitrogen) to result in final concentrations of 400 µg/ml and 100 µM, respectively, 50 µg of the expression vector (CMV-3xFLAGJFHE1dTM or CMV-3xFLAGJFHE2dTM) was added at 0.1 µg/µl per 13 ml of the solution, and culture was then conducted. Subsequently, the supernatant of the cultured COS1 cells was aspirated off, 10 ml of PBS(-) (Nissui) was added thereto and the cells were washed once. After PBS(-)was aspirated off, 13 ml of a DEAE dextran-DNA mixture was added thereto per 150-cm² flask, and incubation was then carried out at 37°C in the presence of 5% CO₂ for 4 hours.

4 hours later, the DEAE dextran-DNA mixture was aspirated off, and the cells were washed once with 10 ml of PBS. CHO-SFM medium (Invitrogen) was added thereto in amounts of 50 ml per flask, and culture was conducted at 37°C in the presence of 5% CO₂. The culture supernatant was collected in a 50-ml centrifuge tube (Corning Coaster) 4 days later. The collected supernatant was centrifuged at 6,000 rpm (with the use of a HITACHI RPR9-2 rotor) for 30 minutes at 4°C and filtered through a 0.2-µm filter (Whatman).

The culture supernatant was purified with the use of anti-FLAG M2 agarose (SIGMA) as follows. To 500 ml of the culture supernatant, anti-FLAG M2 agarose was added, and the reaction was allowed to proceed at 4°C (in a low-temperature chamber) for 2 hours while undergoing agitation in a spinner bottle. A mixture of the supernatant and anti-FLAG M2 agarose was transferred to the Econo-Column (BIO-RAD) 2 hours later, and flow-through fractions were collected. Subsequently, the column was washed twice with 10 ml of TBS (50 mM Tris-HCl, 150 mM NaCl, pH 7.4). Six fractions (1 ml of each fraction) were eluted with the use of 0.1M glycine-HCl (pH 3.5). Immediately after elution, 1M Tris-HCl (pH 9.5) was added for neutralization. The fractions (20 µl each) were subjected to SDS-polyacrylamide gel electrophoresis under reducing conditions and stained with Coomassie brilliant blue. As a result, E1 proteins or E2 proteins derived from the JFH-1 strain were found to be purified.

### 3) Preparation of E1 and E2 proteins-immobilized plate

The E1 and E2 proteins derived from the J6CF strain were diluted with PBS to become 1 µg of each of the two proteins/ml. The solution of E1 and E2 protein mixture (50 µl) was added to wells of the immunoplate (NUNC), the plate was allowed to stand at 4°C overnight, and the proteins were immobilized on the plate. The protein solution was removed, 150 µl of Block Ace (Dainippon Pharmaceutical Co., Ltd.) was added to each well, and the plate was subjected to blocking at room temperature for 4 hours.

E1 and E2 proteins derived from the JFH-1 were also diluted with PBS to become 1 µg of each of the two proteins/ml. The solution of E1 and E2 protein mixture (50 µl) was added to wells of the immunoplate (NUNC), the plate was allowed to stand at 4°C overnight, and the proteins were immobilized on the plate. The protein solution was removed, 150 µl of Block Ace (Dainippon Pharmaceutical Co., Ltd.) was added to each well, and the plate was subjected to blocking at room temperature for 4 hours.

These plates were used for screening for the anti-HCV antibody in the culture supernatant of hybridomas as described below.

### 4) Screening of culture supernatant of hybridoma prepared using J6/JFH-1-HCV particle antigen

The plate prepared in 3) above upon which E1 and E2 proteins derived from the J6CF strain have been immobilized was washed 4 times with PBS containing 0.1% Tween 20 (SIGMA), 50 µl of the hybridoma supernatant samples obtained in Example 6 each was added to the wells, and the reaction was allowed to proceed at room temperature for 1 hour while the plate was shaken with a plate mixer. After the reaction, the plate was washed 4 times with PBS containing 0.1% Tween 20 (SIGMA), 50 µl of the HRP-labeled anti-mouse IgG antibody (Amersham Biosciences), which had been diluted 5,000-fold with PBS containing 0.1% Tween 20, was added to each well, and the reaction was allowed to proceed at room temperature for 1 hour while the plate was shaken. After the reaction, the plate was washed 4 times with PBS containing 0.1% Tween 20 (SIGMA), color was developed using a color development kit for peroxidase (Sumitomo Bakelite Co., Ltd.), the absorbance at 450 nm was measured using Multi-Scan (Titer-Tech), and positive clones were selected.

Subsequently, inhibitory activity on infection with HCV of the culture supernatants of the positive clones was evaluated using J6/JFH-1-HCV particles as infectious agents. Specifically, a solution containing J6/JFH1-HCV particles having the infectious titer of 1 x 10⁶ ffu/ml was mixed with the equivalent amount of the hybridoma supernatant, and the mixture was cultured at 37°C for 1 hour. Thereafter, the mixture sample of J6/JFH-1-HCV particles and hybridoma culture supernatant was added to a 96-well poly-L-lysine-coated plate (Corning 96 Well Clear Flat Bottom Poly-L-Lysine Coated Microplate, Corning) to which the Huh-7 cells had been seeded at 1 x 10⁴ cells/well on the previous day, and then culture was conducted at 37°C for 72 hours.

After the sample was removed, the plate was soaked in ice-cold methanol to fix the cells. Thereafter, methanol was removed via air drying, and cells were permeabilized with the use of Block Ace^{®} (Dainippon Pharmaceutical Co., Ltd.) containing 0.3% Triton^{®}-X 100 (GE Healthcare). HCV-infected cells were detected using the clone 2H9 anti-HCV-core antibody (Wakita, T. et al., Nat. Med. 11:791-796, 2005) and goat anti-mouse IgG-Alexa488 (Molecular Probes), and the number of HCV-infected cells was counted under a fluorescent microscope (IX-70; Olympus). Two samples each exhibiting a smaller number of infected cells were selected as hybridoma cell lines that produce monoclonal antibodies having inhibitory activity on infection with HCV. These cell lines were cloned via limiting dilution and clones J6/1G11-25 (Accession Number: FERM BP-10980) and J6/4D4-2 (Accession Number: FERM BP-10981) were obtained.

### 5) Screening of culture supernatant of hybridoma prepared using JFH-1-HCV particle antigen

The plate prepared in 3) above upon which E1 and E2 proteins derived from the JFH-1 strain had been immobilized was washed 4 times with PBS containing 0.1% Tween 20 (SIGMA), 50 µl of the hybridoma supernatant samples obtained in Example 6 each was added to the wells, and the reaction was allowed to proceed at room temperature for 1 hour while shaking the plate with a plate mixer. After the reaction, the plate was washed 4 times with PBS containing 0.1% Tween 20 (SIGMA), 50 µl of the HRP-labeled anti-mouse IgG antibody (Amersham Biosciences), which had been diluted 5,000-fold with PBS containing 0.1% Tween 20, was added to each well, and the reaction was allowed to proceed at room temperature for 1 hour while the plate was shaken. After the reaction, the plate was washed 4 times with PBS containing 0.1% Tween 20 (SIGMA), color was developed using a color development kit for peroxidase (Sumitomo Bakelite Co., Ltd.), and the absorbance at 450 nm was measured using Multi-Scan (Titer-Tech). Four clones were selected from among the positive clones as hybridoma cell lines that produce monoclonal antibodies recognizing the E1 and/or E2 proteins of the JFH-1 strain. Subsequently, inhibitory activity on infection with HCV of the culture supernatant of positive clones was evaluated in the same manner as in 4) using the J6/JFH-1-HCV particles as infectious agents. As a result, a positive clone JF/M1-4 (Accession Number: FERM BP-10982) was obtained. The positive clone JF/M1-4 is a hybridoma cell line that produces a monoclonal antibody recognizing the E1 and/or E2 protein of the JFH-1 strain and produces an antibody inhibiting infection with J6/JFH-1-HCV.

### [Example 8] Analysis of monoclonal antibody inhibiting infection with HCV

The three hybridoma cell lines selected in example 7: J6/1G11-25 (Accession Number: FERM BP-10980); J6/4D4-2 (Accession Number: FERM BP-10981); and JF/M1-4 (Accession Number: FERM BP-10982) were deposited at the International Patent Organism Depositary of the National Institute of Advanced Industrial Science and Technology (Central 6, 1-1-1 Higashi, Tsukuba, Ibaraki, 305-8566, Japan) as of July 19, 2007.

Properties of monoclonal antibodies inhibiting infection with HCV produced from these hybridoma cell lines were analyzed as follows.

### 1) Antibody subclass

Mouse antibody subclass was determined by analyzing the culture supernatant of the positive clones using the ImmunoPure Monoclonal Antibody Isotyping Kit (Pierce). As a result of isotype analysis of the clones, all clones were found to have µ-type H chains and κ-type L chains. Thus, these clones were found to be of the IgM subclass.

### 2) Molecular weight

The hybridoma was cultured in serum-free medium, and the culture supernatant was subjected to salting-out with 50% ammonium sulfate, followed by dialysis against PBS. Thereafter, the resultant was subjected to SDS-polyacrylamide gel electrophoresis under reducing conditions and stained with Coomassie brilliant blue, and the molecular weights of the H chains and the L chains of the antibodies were estimated. As a result, antibodies from all clones were found to have H chains with a molecular weight of 70 kDa and L chains with a molecular weight of 23 kDa.

### 3) Evaluation of inhibitory activity on infection with HCV of monoclonal antibodies

Monoclonal antibodies were purified from each of culture supernatants of the hybridoma cell lines. The purification of the antibodies was done by subjecting the supernatant of hybridomas conditioned to and cultured in serum-free medium Hybridoma-SFM to 50% ammonium sulfate precipitation and dialysis against PBS. The concentration of the purified antibody sample was determined by performing SDS-PAGE using purified mouse IgMκ (Sigma) as the standard and Coomassie staining.

Inhibitory activity on infection with HCV of the thus-prepared purified monoclonal antibodies derived from the hybridoma cell lines was evaluated in the same manner as described above. That is, the antibodies were added with infectious HCV particles to cells, incubation was carried out, the HCV RNA concentration in the infected cells was measured, and the HCV RNA amount in the cells was analyzed. Since HCV RNA in the cells are replicated from infectious HCV particles, the amount of HCV RNA in the cell should decrease if the infection is inhibited by the monoclonal antibodies.

Specifically, purified monoclonal antibodies each was mixed with infectious HCV particles (J6/JFH-1-HCV particles) such that the monoclonal antibodies became 10 µg/ml and the infectious HCV particles achieved 10⁷ copies/ml in the mixture, and the resultant was allowed to stand for 1 hour at room temperature. The medium used for the Huh-7 cells that had been seeded at 4 x 10⁴ cells/well on a 24-well plate on the previous day and cultured therein was discarded, and the mixture of J6/JFH-1-HCV particles and purified monoclonal antibodies was added thereto at 200 µl/well. The cells were incubated for 3 hours at 37°C, the solution containing virus particles was discarded, and the wells were washed once with PBS. A fresh medium was added, culture was conducted for 2 days, the medium was discarded, the wells were washed once with PBS, and RNA was extracted using the Trizol reagent (Invitrogen). The copy number of HCV RNA contained in the extracted RNA was determined. The determination of the RNA copy number was done according to the method of Takeuchi et al. (Gastroenterology, 1999, 116, pp. 636-42.).

Fig. 4 shows the results of evaluation of inhibitory activity on infection with J6/JFH-1-HCV for the purified monoclonal antibodies derived from hybridoma cell lines J6/1G11-25, J6/4D4-2, and JF/M1-4 (referred to as J6/1G11-25 monoclonal antibody, J6/4D4-2 monoclonal antibody, and JF/M1-4 monoclonal antibody, respectively) and the control antibody (human IgG). Also, Fig. 5 shows the results of evaluation of inhibitory activity on infection with J6/JFH-1-HCV for the purified monoclonal antibody derived from hybridoma cell line JF/M1-4 and the control antibody. As shown in Fig. 4 and in Fig. 5, all antibodies other than control antibody showed activity of inhibiting infection with HCV.

### [Example 9] Analysis of epitope of JF/M1-4 monoclonal antibody

Regarding amino acids 1 to 162 of the E1 protein of the JFH-1 strain (SEQ ID NO: 25) and amino acids 1 to 337 of the E2 protein of the JFH-1 strain (SEQ ID NO: 26), peptides comprising amino acid sequences having 10 continuous amino acids which were designed to each be shifted by 3 amino acids from the N terminuses were synthesized. The N terminuses of the peptides were biotinylated, and the C terminuses were glycine amide (the peptides were synthesized by JPT on consignment).

The synthesized peptides were dissolved in DMSO and then dissolved in PBS at a concentration of 0.01 nmol/µl. The peptide solution (50 µl) was added to wells of the streptavidin-coated plate (Nunc) and the reaction was allowed to proceed at room temperature for 1 hour. Subsequently, the peptide solution was discarded, Blocking One (Nacalai Tesque) was added at 200 µl/well, and the plate was allowed to stand at room temperature for 5 hours. The Blocking One solution was discarded, the plate was washed 4 times with 150 µl/well of PBS (pH 7.2) containing 0.05% Tween 20, the JF/M1-4 monoclonal antibody diluted 1 µg/ml with PBS (pH 7.2) containing 0.05% Tween 20 was added at 50 µl/well, and the reaction was allowed to proceed at room temperature for 1 hour. Subsequently, the antibody solution was discarded, the plate was washed 5 times with 150 µl/well of PBS (pH 7.2) containing 0.05% Tween 20, 50 µl/well of the HRP-labeled anti-mouse IgG goat antibody (GE Healthcare) diluted 5,000-fold with PBS containing 0.05% Tween 20 was added, and the reaction was allowed to proceed at room temperature for 1 hour. After the reaction, the antibody solution was discarded, and the plate was washed 5 times with 150 µl/well of PBS (pH 7.2) containing 0.05% Tween 20. After washing, antibodies bound to peptides were detected using a color development kit for HPR (Sumitomo Bakelite Co., Ltd.).

Fig. 6A shows binding intensity of the JF/M1-4 monoclonal antibody to peptides (peptide nos. 1 to 52) derived from the E1 protein of the JFH-1 strain, which is indicated by the antibody level as assayed at OD 450 nm. Fig. 6B shows binding intensity of the JF/M1-4 monoclonal antibody to peptides (peptide nos. 1 to 110) derived from the E2 protein of the JFH-1 strain, which is indicated by the antibody level as assayed at OD 450 nm. When a measured value at OD 450 nm (i.e., a value along the vertical axis in Fig. 6A or 6B) is high, the binding intensity of the JF/M1-4 monoclonal antibody to the peptide is high, which indicates that the antibody recognizes the peptide.

As a result, the JF/M1-4 monoclonal antibody did not recognize any peptides derived from the E1 protein of the JFH-1 strain (Fig. 6A) but recognized some peptides derived from the E2 protein of the JFH-1 strain (Fig. 6B). A particularly strong epitope is LVHYPYRLWH (SEQ ID NO: 18; peptide no. 77 in Fig. 6B), and the peptides WLTPKCLVHY (SEQ ID NO: 19; peptide no. 75 in Fig. 6B), PKCLVHYPYR (SEQ ID NO: 20; peptide no. 76 in Fig. 6B) and YPYRLWHYPC (SEQ ID NO: 21; peptide no. 78 in Fig. 6B) which overlap with the strong peptide were weak epitopes. Also, the peptides NFTIFKIRMY (SEQ ID NO: 22; peptide no. 82 in Fig. 6B) and IFKIRMYVCG (SEQ ID NO: 23; peptide no. 83 in Fig. 6B) were identified as weak epitopes.

Thus, the JF/M1-4 monoclonal antibody was found to be capable of recognizing, as the epitope, WLTPKCLVHYPYRLWHYPC (SEQ ID NO: 24) within the E2 protein of the JFH-1 strain, which contains a strong epitope.

### [Example 10] Analysis of epitope of J6/1G11-25 monoclonal antibody and J6/4D4-2 monoclonal antibody to J6/JFH-1-HCV particles

Regarding amino acids 1 to 162 of the E1 protein of the J6CF strain (SEQ ID NO: 27) and amino acids 1 to 337 of the E2 protein of the J6CF strain (SEQ ID NO: 28), peptides comprising amino acid sequences having 10 continuous amino acids which were designed to each be shifted by 3 amino acids from the N terminuses were synthesized. The N terminuses of the peptides were biotinylated, and the C terminuses were glycine amide (the peptides were synthesized by JPT on consignment).

The synthesized peptides were dissolved in DMSO and then dissolved in PBS at a concentration of 0.01 nmol/µl. The peptide solution (50 µl) was added to wells of the streptavidin-coated plate (Nunc) and the reaction was allowed to proceed at room temperature for 1 hour. Subsequently, the peptide solution was discarded, Blocking One (Nacalai Tesque) was added at 200 µl/well, and the plate was allowed to stand at room temperature for 5 hours. The Blocking One solution was discarded, each well of the plate was washed 4 times with 150 µl of PBS (pH 7.2) containing 0.05% Tween 20, the J6/1G11-25 or J6/4D4-2 monoclonal antibody diluted 1 µg/ml with PBS (pH 7.2) containing 0.05% Tween 20 was added at 50 µl/well, and the reaction was allowed to proceed at room temperature for 1 hour. Subsequently, the antibody solution was discarded, the plate was washed 5 times with 150 µl/well of PBS (pH 7.2) containing 0.05% Tween 20, 50 µl/well of the HRP-labeled anti-mouse IgG goat antibody (GE Healthcare) diluted 5,000-fold with PBS containing 0.05% Tween 20 was added, and the reaction was allowed to proceed at room temperature for 1 hour. After the reaction, the antibody solution was discarded, and the plate was washed 5 times with 150 µl/well of PBS (pH 7.2) containing 0.05% Tween 20. Subsequently, antibodies bound to peptides were detected using a spectrophotometer (at OD 450 nm) with the aid of a color development kit for HPR (Sumitomo Bakelite Co., Ltd.).

Fig. 7A shows binding intensity of the J6/1G11-25 monoclonal antibody to peptides (peptide nos. 1 to 52) derived from the E1 protein of the J6CF strain. Fig. 7B shows binding intensity of the J6/1G11-25 monoclonal antibody to peptides (peptide nos. 1 to 110) derived from the E2 protein of the J6CF strain. When a measured value at OD 450 nm (i.e., a value along the vertical axis in Fig. 7A or 7B) is high, the binding intensity of the J6/1G11-25 monoclonal antibody to the peptide is high, which means that the antibody specifically recognizes the peptide.

As a result, the J6/1G11-25 monoclonal antibody did not recognize any peptides derived from the E1 protein of the J6CF strain (Fig. 7A) but recognized some peptides derived from the E2 protein of the J6CF strain (Fig. 7B). Peptides recognized by the J6/1G11-25 monoclonal antibody were NVTNPEDMRP (SEQ ID NO: 29; peptide no. 32 in Fig. 7B) and NPEDMRPYCW (SEQ ID NO: 30; peptide no. 33 in Fig. 7B) overlapping therewith, and NYTIFKIRMY (SEQ ID NO: 31; peptide no. 82 in Fig. 7B) and IFKIRMYVGG (SEQ ID NO: 32; peptide no. 83 in Fig. 7B) overlapping therewith.

Fig. 8A shows binding intensity of the J6/4D4-2 monoclonal antibody to peptides (peptide nos. 1 to 52) derived from the E1 protein of the J6CF strain. Fig. 8B shows binding intensity of the J6/4D4-2 monoclonal antibody to peptides (peptide nos. 1 to 110) derived from the E2 protein of the J6CF strain. When a measured value at OD 450 nm (i.e., a value along the vertical axis in Fig. 8A and 8B) are high, the binding intensity of the J6/4D4-2 monoclonal antibody to the peptide is high, which indicates that the antibody specifically recognizes the peptide.

As a result, the J6/4D4-2 monoclonal antibody recognized some peptides derived from the E1 protein of the J6CF strain and some peptides derived from the E2 protein of the J6CF strain, as shown in Fig. 8. An E1-derived strong epitope was FIVSPQHHWF (SEQ ID NO: 33; peptide no. 34 in Fig. 8A), and SPQHHWFVQD (SEQ ID NO: 34; peptide no. 35 in Fig. 8A) and HHWFVQDCNC (SEQ ID NO: 35; peptide no. 36 in Fig. 8A) which overlap with the strong peptide were weak epitopes. Another strong epitope was MAWDMMMNWS (SEQ ID NO: 36; peptide no. 43 in Fig. 8A).

E2-derived strong epitopes were LIDYPYRLWH (SEQ ID NO: 37; peptide no. 77 in Fig. 8B) and YPYRLWHYPC (SEQ ID NO: 38; peptide no. 78 in Fig. 8B) overlapping with the peptide, and DMRPYCWHYP (SEQ ID NO: 39; peptide no. 34 in Fig. 8B). NPEDMRPYCW (SEQ ID NO: 40; peptide no. 33 in Fig. 8B) and PYCWHYPPRQ (SEQ ID NO: 41; peptide no. 35 in Fig. 8B) which overlap with the last strong peptide were weak epitopes.

A relatively strong epitope was NYTIFKIRMY (SEQ ID NO: 31; peptide no. 82 in Fig. 8B), and IFKIRMYVGG (SEQ ID NO: 32; peptide no. 83 in Fig. 8B) overlapping therewith was a weak epitope.

Further, STRPPLGSWF (SEQ ID NO: 42; peptide no. 54 in Fig. 8B) and GSWFGCTWMN (SEQ ID NO: 43; peptide no. 56 in Fig. 8B) were identified as weak epitopes.

Thus, the J6/1G11-25 monoclonal antibody was found to recognize the amino acid sequences within the E2 protein of the J6CF strain, NVTNPEDMPRPYCW (SEQ ID NO: 44) and NYTIFKIRMYVGG (SEQ ID NO: 45), as epitopes.

Also, the J6/4D4-2 monoclonal antibody was found to recognize the amino acid sequences within the E1 protein of the J6CF strain, FIVSPQHHWFVQDCNC (SEQ ID NO: 46) and MAWDMMMNWS (SEQ ID NO: 36), as epitopes as well as the amino acid sequences within the E2 protein of the J6CF strain, LIDYPYRLWHYPC (SEQ ID NO: 47), NPEDMRPYCWHYPPRQ (SEQ ID NO: 48), and NYTIFKIRMYVGG (SEQ ID NO: 45), as epitopes.

### [Example 11] Cloning of DNA encoding V region of JF/M1-4 monoclonal antibody

DNA encoding a variable region of the JF/M1-4 mouse monoclonal antibody against HCV particles was cloned as follows.

### 1) Preparation of total RNA

Total RNA was prepared from the JF/M1-4 hybridoma cell line using PureLink Micro-to-Midi (Invitrogen) in accordance with the method described in the accompanying manual. Specifically, 2 x 10⁸ hybridoma line JF/M1-4 cells were suspended in 2.4 ml of RNA Lysis solution, and the cells were passed through a syringe with a 18 gauge needle several times for homogenization. The resulting homogenate was centrifuged, 70% ethanol was added to the same amount of the resulting supernatant, and the mixture was applied on an RNA spin cartridge. The cartridge was thoroughly washed with the addition of a washing solution and RNA was eluted with RNase-free water.

### 2) Synthesis of single-stranded cDNA

With the use of µ-type H chain- and κ-type L chain-specific primers, single-stranded cDNA was synthesized from the total RNA prepared in 1) above as follows.

### i) Synthesis of cDNA encoding µ-type H chain of JF/M 1-4 monoclonal antibody

To 5 µg of RNA, 50 pmol of the µ-type H chain-specific primer mCHm-as (SEQ ID NO: 49; 5'-AGGCTTACTAGTAGCTGGAATGGGCACATGCAGATC-3') was added for annealing, and 5 µl of 0.1M DTT, 1.25 µl of 20 mM dNTPs solution, and 10 µl of 5x RT Buffer supplied with SuperScriptII were added thereto, and distilled water (DW) was added thereto to bring the final volume to 49 µl.

Subsequently, 1 µl of a reverse transcriptase SuperScriptII (Invitrogen) was added, the reaction was carried out at 42°C for 30 minutes and at 50°C for 30 minutes, and the reaction solution was directly used for the next step, i.e., polymerase chain reaction (PCR).

### ii) Synthesis of cDNA encoding κ-type L chain of JF/M1-4 monoclonal antibody

To 5 µg of RNA, 50 pmol of the κ-type L chain-specific primer mCK1-as (SEQ ID NO: 50; 5'-CATGTCTAGAACACTCATTCCTGTTGAAGCTCTTG-3') was added for annealing, and 5 µl of 0.1M DTT, 1.25µl of 20 mM dNTPs solution, and 10 µl of 5x RT Buffer supplied with SuperScriptII were added thereto, and distilled water (DW) was added thereto to bring the final volume to 49 µl. Subsequently, 1 µl of a reverse transcriptase SuperScriptII (Invitrogen) was added, the reaction was carried out at 42°C for 30 minutes and at 50°C for 30 minutes, and the reaction product was directly used for the next step, i.e., polymerase chain reaction (PCR).

### 3) Amplification via PCR method of gene encoding variable region of JF/M1-4 monoclonal antibody

PCR was carried out with the GeneAmp^{(R)} PCR System 9700 (Applied Biosystems) using the Advantage GC2 DNA polymerase kit (TAKARA).

### i) Amplification of cDNA encoding mouse H chain V region

PCR was carried out using the Mouse Ig-Primer Set (69831-3, Novagen) in accordance with the conditions described in the accompanying manual.

Specifically, PCR was carried out using the MuIgVH5'-A to MuIgVH5'-F primers (that hybridize to the leader sequence of the mouse H chain) and the MuIgMVH3'-1 primer (that hybridizes to the mouse µ-type H chain C region) supplied with the kit.

To 5 µl of the reaction mixture resulting from synthesis of single-stranded cDNA encoding the µ-type H chain prepared above, 10 µl of 5x PCR buffer supplied with the kit, 10 µl of GC Melt, 4 µl of 2.5 mM dNTPs solution, 1.25 units of Advantage GC2 DNA polymerase mix (TAKARA), 1 µl of 10 pmol/µl MuIgVH5' primer, and 1 µl of 5 pmol/µl MuIgMVH3'-1 primer were added. Distilled water (DW) was further added to bring the final volume to 50 µl. The PCR solution (50 µl) was incubated at 94°C for 4 minutes and then heated at 94°C for 1 minute, 50°C (in the case of MuIgVH5'-A and MuIgVH5'-B) or 60°C (in the case of MuIgVH5'-C to MuIgVH5'-F) for 1 minute, and 72°C for 2 minutes, in that order. This thermal cycle was repeated 40 times and the reaction mixture was subsequently further incubated at 72°C for 6 minutes.

### ii) Amplification of gene encoding mouse L chain V region

PCR was carried out using the Mouse Ig-Primer Set (69831-3, Novagen) in accordance with the conditions described in the accompanying manual.

Specifically, PCR was carried out using the MuIgκVL5'-A to MuIgκVL5'-F primers (that hybridize to the leader sequence of the mouse κ-type L chain) and the MuIgκVL3'-1 primer (that hybridizes to the mouse κ-type L chain C region) supplied with the kit.

To 5 µl of the reaction mixture resulting from synthesis of single-stranded cDNA encoding the κ-type L chain prepared above, 10 µl of 5x PCR buffer supplied with the kit, 10 µl of GC Melt, 4 µl of 2.5 mM dNTPs solution, 1.25 units of Advantage GC2 DNA polymerase mix (TAKARA), 1 µl of 10 pmol/µl MuIgκVL 5' primer, and 1 µl of 5 pmol/µl MuIgκVL3'-1 primer were added. Distilled water (DW) was further added to bring the final volume to 50 µl. The PCR solution (50 µl) was incubated at 94°C for 4 minutes and then heated at 94°C for 1 minute, 50°C (in the case of MuIgVL5'-A and MuIgVL5'-B) or 60°C (in the case of MuIgVL5'-C to MuIgVL5'-F) for 1 minute, and 72°C for 2 minutes, in that order. This thermal cycle was repeated 40 times and the reaction mixture was subsequently further incubated at 72°C for 6 minutes.

### 4) Purification of PCR product and cloning thereof into plasmid vector

The DNA fragmets PCR amplified as described above were separated via agarose gel electrophoresis. A DNA fragment of approximately 450 bp in length was purified using GeneElute (Sigma). A DNA fragment was cloned using the TOPO TA Cloning kit (Invitrogen). The pCR-TOPO vector supplied with the kit, the DNA fragment, and a salt solution supplied with the kit were added, the resultant was allowed to stand at room temperature for 5 minutes, and a part of the reaction solution was added to competent cells of E. Coli Mach1 (Invitrogen). The E. Coli competent cells were placed on ice for 30 minutes, subsequently heated at 42°C for 30 seconds, and placed again on ice for 1 minute. SOC medium (room temperature) was added thereto, the cells was cultured at 37°C for 1 hour and seeded on an agar medium, and then cultured at 37°C overnight. Plasmid DNA was prepared from the resulting transformant, and the nucleotide sequence of the cloned DNA was determined in accordance with a conventional technique.

### 5) Analysis of nucleic acid sequence of cDNA encoding V region of JF/M1-4 mouse monoclonal antibody

DNAs cloned in the plasmids, the nucleotide sequences of which were determined in 4) above (which are cDNAs encoding the H chain V region and the L chain V region of the JF/M1-4 mouse monoclonal antibody), were analyzed as follows.

### i) Analysis of nucleic acid sequence of cDNA encoding mouse H chain V region

Nucleic acid sequences of the H chain V regions cloned in many resultant clones were found to be identical except for the regions of the mouse H chain leader sequences. This indicates that a plurality of clones tested are derived from independent isolates obtained via PCR utilizing a mixture of various primers encoding the mouse H chain leader sequences. Further, by searching a database for encoded amino acid sequences deduced from their nucleic acid sequences, sequences of regions from the framework 1 to the J segment (framework 4) of the H chain V region of the JF/M1-4 mouse monoclonal antibody were deduced (SEQ ID NOs: 55 to 61). The nucleic acid sequence and the amino acid sequence thereby determined are shown in SEQ ID NOs: 51 and 52 of the Sequence Listing, respectively. Further, CDR1 to CDR3 deduced from the amino acid sequences are shown in Fig. 9. It was revealed that the H chain V region of the JF/M1-4 mouse monoclonal antibody (nucleic acid sequence: SEQ ID NO: 51; amino acid sequence: SEQ ID NO: 52) plays a role in recognition of an epitope (i.e., WLTPKCLVHYPYRLWHYPC (SEQ ID NO: 24) or parts thereof containing 10 or more continuous amino acid residues) by the antibody.

### ii) Analysis of nucleic acid sequence of cDNA encoding mouse L chain V region

Nucleic acid sequences of the L chain V regions cloned in many resultant clones were found to be identical except for the regions of the mouse L chain leader sequences. This indicates that a plurality of clones tested are derived from independent isolates obtained via PCR utilizing a mixture of various primers encoding the mouse L chain leader sequences. Further, by searching a database for encoded amino acid sequences deduced from their nucleic acid sequences, sequences of regions from the framework 1 to the J segment (framework 4) of the L chain V region of the JF/M1-4 mouse monoclonal antibody were deduced (SEQ ID NOs: 62 to 68). The nucleic acid sequence and the amino acid sequence thereby determined are shown in SEQ ID NOs: 53 and 54. Further, CDR1 to CDR3 deduced from the amino acid sequences are shown in Fig. 10. It was revealed that the L chain V region of the JF/M1-4 mouse monoclonal antibody (nucleic acid sequence: SEQ ID NO: 53; amino acid sequence: SEQ ID NO: 54) played a role in recognition of an epitope (i.e., WLTPKCLVHYPYRLWHYPC (SEQ ID NO: 24) or parts thereof containing 10 or more continuous amino acid residues) by the antibody.

### Industrial Applicability

The antibody according to the present invention which has inhibitory activity on infection with HCV can be used as a therapeutic or preventive medicament for HCV infection.

### Sequence Listing Free Text

SEQ ID NO: 1 discloses the HCV genome-length cDNA cloned into pJFH-1.
SEQ ID NO: 2 discloses the chimeric HCV genome-length cDNA sequence cloned into pJ6/JFH-1.
SEQ ID NO: 3 discloses the HCV genome-length cDNA sequence cloned into pTH/JFH-1.
SEQ ID NO: 4 discloses the sequence of the primer (JFH-1-A).
SEQ ID NO: 5 discloses the sequence of the primer (JFH-1-B).
SEQ ID NO: 6 discloses the sequence of the primer (TH-C).
SEQ ID NO: 7 discloses the sequence of the primer (TH-D).
SEQ ID NO: 8 discloses the sequence of the primer (JFH-1-E).
SEQ ID NO: 9 discloses the sequence of the primer (JFH-1-F).
SEQ ID NO: 10 discloses the sequence of the primer (J6E1dTM-s).
SEQ ID NO: 11 discloses the sequence of the primer (J6E1dTM-as).
SEQ ID NO: 12 discloses the sequence of the primer (J6E2dTM-s).
SEQ ID NO: 13 discloses the sequence of the primer (J6E2dTM-as).
SEQ ID NO: 14 discloses the sequence of the primer (JFHE1dTM-s).
SEQ ID NO: 15 discloses the sequence of the primer (JFHE1dTM-as).
SEQ ID NO: 16 discloses the sequence of the primer (JFHE2dTM-s).
SEQ ID NO: 17 discloses the sequence of the primer (JFHE2dTM-as).
SEQ ID NOs: 18 to 24 disclose the sequences of synthetic peptides.
SEQ ID NO: 25 discloses the amino acid sequence comprising amino acids 1 to 162 of E1 protein of the JFH-1 strain.
SEQ ID NO: 26 discloses the amino acid sequence comprising amino acids 1 to 337 of the E2 protein of the JFH-1 strain.
SEQ ID NO: 27 discloses the amino acid sequence comprising amino acids 1 to 162 of the E1 protein of the J6CF strain.
SEQ ID NO: 28 discloses the amino acid sequence comprising amino acids 1 to 337 of the E2 protein of the J6CF strain.
SEQ ID NOs: 29 to 48 disclose the sequences of synthetic peptides.
SEQ ID NO: 49 discloses the sequence of the primer (mCHm-as).
SEQ ID NO: 50 discloses the sequence of the primer (mCK1-as).
SEQ ID NO: 51 discloses the nucleotide sequence of the gene encoding the H chain V region (i.e., the heavy chain variable region) of the JF/M1-4 monoclonal antibody.
SEQ ID NO: 52 discloses the amino acid sequence of the H chain V region (i.e., the heavy chain variable region) of the JF/M1-4 monoclonal antibody.
SEQ ID NO: 53 discloses the nucleotide sequence of the gene encoding the L chain V region (i.e., the light chain variable region) of the JF/M1-4 monoclonal antibody.
SEQ ID NO: 54 discloses the amino acid sequence of the L chain V region (i.e., the light chain variable region) of the JF/M1-4 monoclonal antibody.
SEQ ID NO: 55 discloses the amino acid sequence of the framework 1 of the H chain V region of the JF/M1-4 monoclonal antibody.
SEQ ID NO: 56 discloses the amino acid sequence of CDR1 of the H chain V region of the JF/M1-4 monoclonal antibody.
SEQ ID NO: 57 discloses the amino acid sequence of the framework 2 of the H chain V region of the JF/M1-4 monoclonal antibody.
SEQ ID NO: 58 discloses the amino acid sequence of CDR2 of the H chain V region of the JF/M1-4 monoclonal antibody.
SEQ ID NO: 59 discloses the amino acid sequence of the framework 3 of the H chain V region of the JF/M1-4 monoclonal antibody.
SEQ ID NO: 60 discloses the amino acid sequence of CDR3 of the H chain V region of the JF/M1-4 monoclonal antibody.
SEQ ID NO: 61 discloses the amino acid sequence of the framework 4 (i.e., the J segment) of the H chain V region of the JF/M1-4 monoclonal antibody.
SEQ ID NO: 62 discloses the amino acid sequence of the framework 1 of the L chain V region of the JF/M1-4 monoclonal antibody.
SEQ ID NO: 63 discloses the amino acid sequence of CDR1 of the L chain V region of the JF/M1-4 monoclonal antibody.
SEQ ID NO: 64 discloses the amino acid sequence of the framework 2 of the L chain V region of the JF/M1-4 monoclonal antibody.
SEQ ID NO: 65 discloses the amino acid sequence of CDR2 of the L chain V region of the JF/M1-4 monoclonal antibody.
SEQ ID NO: 66 discloses the amino acid sequence of the framework 3 of the L chain V region of the JF/M1-4 monoclonal antibody.
SEQ ID NO: 67 discloses the amino acid sequence of CDR3 of the L chain V region of the JF/M1-4 monoclonal antibody.
SEQ ID NO: 68 discloses the amino acid sequence of the framework 4 (i.e., the J segment) of the L chain V region of the JF/M1-4 monoclonal antibody.

## Claims

1. An antibody that recognizes a hepatitis C virus (HCV) particle obtained from the genome of a hepatitis C virus (HCV) comprising the following (i) and (ii) ligated to each other as an antigen and has inhibitory activity on infection with hepatitis C virus (HCV):
(i)
(a) the 5' untranslated region, the core protein-encoding sequence, the E1 protein-encoding sequence, the E2 protein-encoding sequence, and the p7 protein-encoding sequence of the JFH-1 strain of the hepatitis C virus (HCV), or
(b) the 5' untranslated region, the core protein-encoding sequence, the E1 protein-encoding sequence, the E2 protein-encoding sequence, and the p7 protein-encoding sequence of the J6CF strain of the hepatitis C virus (HCV); and
(ii) the NS2 protein-encoding sequence, the NS3 protein-encoding sequence, the NS4A protein-encoding sequence, the NS4B protein-encoding sequence, the NS5A protein-encoding sequence, the NS5B protein-encoding sequence, and the 3' untranslated region of the JFH-1 strain.

2. The antibody according to claim 1, wherein the hepatitis C virus (HCV) genome is a nucleic acid comprising the nucleotide sequence as shown in SEQ ID NO: 1 or 2 of the Sequence Listing (provided that nucleotide "T" in the nucleotide sequence is read as "U" when the nucleic acid is RNA).

3. The antibody according to claim 1 or 2, wherein the antibody class is IgM.

4. The antibody according to any one of claims 1 to 3, which recognizes at least 10 continuous amino acids in the amino acid sequence as shown in SEQ ID NO: 24 of the Sequence Listing.

5. The antibody according to claim 4, which has a heavy chain variable region comprising the amino acid sequence as shown in SEQ ID NO: 52 of the Sequence Listing.

6. The antibody according to claim 4 or 5, which has a light chain variable region comprising the amino acid sequence as shown in SEQ ID NO: 54 of the Sequence Listing.

7. The antibody according to any one of claims 4 to 6, which is produced by the hybridoma cell line of Accession Number FERM BP-10982.

8. The antibody according to any one of claims 1 to 3, which recognizes at least 10 continuous amino acids in the amino acid sequence as shown in SEQ ID NO: 44 or 45 of the Sequence Listing.

9. The antibody according to claim 8, which is produced by the hybridoma cell line of Accession Number FERM BP-10980.

10. The antibody according to any one of claims 1 to 3, which recognizes at least 10 continuous amino acids in the amino acid sequence as shown in SEQ ID NO: 36, 45, 46, 47, or 48 of the Sequence Listing.

11. The antibody according to claim 10, which is produced by the hybridoma cell line of Accession Number FERM BP-10981.

12. An inhibitory agent for infection with hepatitis C virus (HCV) comprising, as an active ingredient, the antibody according to any one of claims 1 to 11.

13. A medicament comprising, as an active ingredient, the antibody according to any one of claims 1 to 11.

14. A therapeutic or preventive agent for hepatitis C comprising, as an active ingredient, the antibody according to any of claims 1 to 11.

15. A method for detecting hepatitis C virus (HCV) comprising detecting hepatitis C virus (HCV) particles using the antibody according to any one of claims 1 to 11.
